# EUROPEAN PATENT SPECIFICATION

(11) **EP 4 168 582 B1**
(45) Date of publication and mention of the grant of the patent: **19.11.2025**
(21) Application number: 21736674.9
(22) Date of filing: 18.06.2021
(51) Int. Cl.: C12Q 1/6869

(54) **A METHOD OF SELECTIVELY CHARACTERISING A POLYNUCLEOTIDE USING A DETECTOR**
VERFAHREN ZUR SELEKTIVEN CHARAKTERISIERUNG EINES POLYNUKLEOTIDS UNTER VERWENDUNG EINES DETEKTORS
PROCÉDÉ DE CARACTÉRISATION SÉLECTIVE D'UN POLYNUCLÉOTIDE À L'AIDE D'UN DÉTECTEUR

(30) Priority: 18.06.2020 GB 202009334; 19.05.2021 GB 202107193
(43) Date of publication of application: 26.04.2023
(73) Proprietor: Oxford Nanopore Technologies plc, Oxford Science Park Oxford OX4 4DQ (GB)
(72) Inventor: BOWEN, Rebecca Victoria, Oxford Oxfordshire OX4 4DQ (GB); BROWN, Clive Gavin, Oxford Oxfordshire OX4 4DQ (GB); BRUCE, Mark John, Oxford Oxfordshire OX4 4DQ (GB); GARALDE, Daniel Ryan, Oxford Oxfordshire OX4 4DQ (GB); GRAHAM, James Edward, Oxford Oxfordshire OX4 4DQ (GB); HERON, Andrew John, Oxford Oxfordshire OX4 4DQ (GB); RAIMONDEAU, Etienne, Oxford Oxfordshire OX4 4DQ (GB); WHITE, James, Oxford Oxfordshire OX4 4DQ (GB); YOUD, Christopher Peter, Oxford Oxfordshire OX4 4DQ (GB)
(74) Representative: J A Kemp LLP
(86) International application number: PCT/GB2021/051555
(87) International publication number: WO 2021/255475

(56) References cited:
- WO-A1-2015/110813
- WO-A1-2016/059427
- WO-A2-2013/057495

## Description

### Field

The present invention relates to methods of selectively characterising polynucleotides of a desired property, such as length, as they move with respect to a nanopore and to methods of sequencing polynucleotides using the novel methods.

### Background

Nanopore sensing is an approach to analyte detection and characterization that relies on the observation of individual binding or interaction events between the analyte molecules and an ion conducting channel. Nanopore sensors can be created by placing a pore of nanometre dimensions in an electrically insulating membrane and measuring signals, such as voltage-driven ion currents, through or across the pore in the presence of analyte molecules. The presence of an analyte alters the measured signal and the properties of the signal are characteristic of the analyte. For example, the signal measured may be a voltage driven ion current measured through the pore, and in the presence of an analyte such as a polynucleotide the current signal can be measured as the analyte interacts with the pore. For example, when the analyte is a polynucleotide, information about the properties of the polynucleotide can be determined from the duration and extent of current blocks and the variance of current levels during the interaction time of the polynucleotide with the pore.

Polynucleotides are important analytes for sensing in this manner. Nanopore sensing of polynucleotide analytes can reveal the identity and perform single molecule counting of the sensed analytes, but can also provide information on their composition such as their nucleotide sequence, as well as the presence of characteristics such as base modifications, oxidation, reduction, decarboxylation, deamination and more. Nanopore sensing has the potential to allow rapid and cheap polynucleotide sequencing, providing single molecule sequence reads of polynucleotides of tens to hundreds of thousands (or even more) bases in length.

It is important to maximise the efficiency of nanopore sensing, especially when characterising analytes such as extremely long polynucleotides, e.g. polynucleotides of tens to hundreds of thousands (or more) bases in length. It is particularly desirable to avoid unproductive characterisation of unwanted analytes such as impurities. Such undesirable unproductive characterisation needlessly consumes reagents thus increasing costs, and increases the time required to obtain characterisation information for desired analytes as acquisition time incurred in unproductive characterisation is wasted. There is thus a need to selectively characterise desired polynucleotides in a sample so that these problems can be mitigated.

To address this problem, it is known to take measurements of a polymer such as a polynucleotide as it moves with respect to a nanopore. In known methods, the polymer to be characterised is allowed to partially translocate through the nanopore and data characteristic of its sequence (the "test sequence") is compared to reference data characteristic of a reference sequence. Responsive to the measure of similarity between the test sequence and the reference sequence, the polymer may be rejected from the nanopore. Thus, if the similarity between the test sequence and the reference sequence is too low, indicating that the analyte is not the intended analyte (e.g. is an impurity), the polymer may be rejected from the nanopore. This ensures that reagents are not consumed in needlessly characterising unwanted analytes and the time required to fully characterise the analyte is not wasted in unproductive characterisation. Such methods are described in WO 2016/059427.

Whilst such methods have led to some improvements in the efficiency of characterising polymers, technical challenges remain. One issue is that for a decision to be taken whether or not to characterise a given analyte, an appropriate reference signal needs to be defined. For some applications no such reference signal is readily available. A second issue is that in such methods the assessment of similarity and therefore the decision whether or not to reject the polymer is based on a partial translocation of the polymer through the nanopore. Differences between the test sequence and reference sequence outside the portion of the polymer which is allowed to translocate through the pore may not be identified until the full characterisation has been completed. This may mean that unwanted analytes such as truncated polymers which have the correct initial test sequence but which are significantly different to the intended analytes may be unproductively characterised, thus wasting reagents and time. A third issue is that to compare the test sequence of a given analyte to a reference sequence it is necessary to conduct a detailed analysis of the test sequence in order to identify its sequence. Accurately determining sequences of polynucleotides can have a significant technical (e.g. computational) burden especially when the portion of the polymer to be assessed is necessarily relatively long (e.g. when the reference sequence is long) and it would therefore be desirable if the decision whether or not to reject the polymer did not require determining the sequence of the polymer.

The methods provided herein are intended to address some or all of these issues.

### Summary

The invention is defined by the scope of the appended claims.

The disclosure relates to a method of characterising a polynucleotide in a sample. The method comprises contacting a nanopore with a polynucleotide. A first part of the polynucleotide is allowed to move freely with respect to the nanoporeunder an applied force such as a potential or under a force applied by an enzyme which permits the movement of the polynucleotide with respect to the nanopore(e.g. its translocation through a nanopore) e.g. at close to voltage-driven translocation speeds (discussed further herein). One or more properties of the first part of the polynucleotide are assessed. Various suitable properties are discussed herein including the approximate length of the first part of the polynucleotide. Where the polynucleotide has one or more desired properties, the method comprises controlling the movement of a second part of the polynucleotide with respect to the nanoporeand taking measurements as the polynucleotide moves with respect to the nanoporein order to determine one or more characteristics of the polynucleotide, thereby characterising the polynucleotide. Where the polynucleotide does not have one or more desired characteristics, the method comprises rejecting the polynucleotide. Where the first part of the polynucleotide does not have one or more desired characteristics, the method typically is repeated with further polynucleotides from the sample until a polynucleotide having a first part having one or more desired characteristics is identified. The polynucleotide which does have one or more desired characteristics can then be characterised as set out above.

Accordingly, provided herein is a method of characterising a polynucleotide in a sample, the method comprising:
(i) contacting a nanoporewith a polynucleotide;
(ii a) taking measurements as a first part of the polynucleotide moves freely with respect to the nanoporeunder an applied force;
(ii b) assessing one or more properties of the first part of the polynucleotide; and
(iii) (a) where the first part of the polynucleotide has one or more desired properties, controlling the movement of a second part of the polynucleotide with respect to the nanopore using a polynucleotide binding proteinand taking measurements as the second part of the polynucleotide moves with respect to the nanopore to determine one or more characteristics of the polynucleotide, thereby characterising the polynucleotide; or (b) where the first part of the polynucleotide does not have one or more desired properties, rejecting the polynucleotide.

In some embodiments, where the first part of the polynucleotide does not have one or more desired properties, step (iii)(b) comprises ejecting the polynucleotide from the nanopore.

In some embodiments, when the first part of the polynucleotide does not have one or more desired properties, step (iii) comprises repeating steps (i), (ii a) and (ii b) with further polynucleotides from the sample until a polynucleotide having a first part having one or more desired properties is identified.

In some embodiments, said one or more desired properties are selected from the approximate length of the first part of the polynucleotide, the structure of the first part of the polynucleotide, and the composition of the first part of the polynucleotide. In some embodiments, assessing said one or more properties of the polynucleotide comprises determining the approximate length of the first part of the polynucleotide. In some embodiments determining the approximate length of the first part of the polynucleotide comprises determining the time taken for the first part of the polynucleotide to move freely with respect to the nanopore.

The nanopore may be a transmembrane nanopore. In some embodiments step (ii a) comprises allowing the first part of the polynucleotide to translocate freely through or across the nanopore under an applied potential. In some embodiments determining the approximate length of the first part of the polynucleotide comprises determining the time taken for the first part of the polynucleotide to translocate through or across the nanopore. In some embodiments, step (iii)(a) comprises controlling the movement of a second part of the polynucleotide through or across the nanopore and taking measurements as the second part of the polynucleotide moves with respect to the nanopore to determine one or more characteristics of the polynucleotide.

In step (iii)(a), the movement of the second part of the polynucleotide is controlled using a polynucleotide binding protein.

In some embodiments, the polynucleotide is a double-stranded polynucleotide comprising a first strand connected to a second strand by a hairpin or hairpin adapter. In some embodiments, prior to step (i) a polynucleotide binding protein is bound to and/or stalled at the hairpin or hairpin adapter.

In some embodiments, determining one or more characteristics of the polynucleotide comprises determining the sequence of the polynucleotide.

In some embodiments, the provided method comprises:
(i) contacting a nanopore with a polynucleotide having a polynucleotide binding protein capable of controlling the movement of the polynucleotide stalled thereon;
(ii a) determining the time taken for a first part of the polynucleotide to move freely with respect to the nanopore under an applied force; and
(ii b) determining the approximate length of the first part of the polynucleotide.

In some embodiments, an adapter is attached to one or both ends of the polynucleotide prior to step (i). In some embodiments, prior to step (i) a polynucleotide binding protein capable of controlling the movement of the polynucleotide is bound to the polynucleotide or to an adapter attached to the polynucleotide.

In some embodiments, the polynucleotide comprises a single stranded leader sequence at one end and has a polynucleotide binding protein bound thereto at the other end on the same strand of the polynucleotide or to an adapter attached to the other end of the same strand of the polynucleotide. In some embodiments, the polynucleotide binding protein is bound to the adapter. In some embodiments, the polynucleotide binding protein is stalled on the polynucleotide or adapter.

In some embodiments, in step (ii a) the first part of the polynucleotide moves freely with respect to the nanopore in a first direction relative to the applied force, and in step (iii) the polynucleotide binding protein controls the movement of the second part of the polynucleotide with respect to the nanopore in a second direction relative to the applied force.

In some embodiments, step (i) comprises contacting the nanopore with a first end of the polynucleotide or an adapter attached to the first end of the polynucleotide and the polynucleotide binding protein is bound to a second end of the polynucleotide or to an adapter attached to the second end of the polynucleotide. In some embodiments, step (i) comprises contacting the nanopore with a leader sequence at the first end of the polynucleotide and the polynucleotide binding protein is stalled at a second end of the polynucleotide or on an adapter attached to the second end of the polynucleotide; and the first part of the polynucleotide is the part between the leader sequence and the polynucleotide binding protein and the second part of the polynucleotide is the same as the first part of the polynucleotide; and the polynucleotide binding protein is orientated on the polynucleotide such that the polynucleotide binding protein controls the movement of the second part of the polynucleotide with respect to the nanopore against the applied force.

In some embodiments,
- the polynucleotide is single-stranded;
- the polynucleotide comprises a leader sequence, wherein the leader sequence is located at the first end of the polynucleotide or is comprised in an adapter attached to the first end of the polynucleotide; and
- the polynucleotide binding protein is stalled at a second end of the polynucleotide or is stalled on an adapter at the second end of the polynucleotide.

In some embodiments, the polynucleotide is double stranded. In some embodiments, the polynucleotide is double stranded and comprises a single stranded leader sequence at one end of a first strand of the double stranded polynucleotide and has polynucleotide binding protein bound thereto at one end of the second strand of the double stranded polynucleotide. In some embodiments:
- the polynucleotide is double-stranded and comprises a first strand and a second strand;
- the polynucleotide comprises a leader sequence located at a first end of the polynucleotide, wherein the leader sequence is comprised in the first strand or is comprised in an adapter attached to the first strand; and
- the polynucleotide binding protein is stalled at a second end of the polynucleotide or is stalled on an adapter at the second end of the polynucleotide.

In some embodiments, the polynucleotide binding protein is stalled at the second end of the first strand of the double-stranded polynucleotide or is stalled on an adapter at the second end of the first strand of the double-stranded polynucleotide.

In some embodiments, the first strand and the second strand are attached together by a hairpin adapter at the second end of the first strand. In some embodiments, the polynucleotide binding protein is stalled at the hairpin adapter. In some embodiments, a hairpin adapter is attached to one end of the double stranded polynucleotides and an adapter comprising a single stranded leader sequence is attached to the other end of the double stranded polynucleotides, and wherein a polynucleotide binding protein capable of controlling the movement of a polynucleotide is bound to the hairpin adapter. In some embodiments, a) the first part of the double-stranded polynucleotide is the part of the first stand between the leader sequence and the polynucleotide binding protein and the second part of the polynucleotide is the same as the first part of the polynucleotide; and b) the polynucleotide binding protein is orientated on the polynucleotide such that the polynucleotide binding protein controls the movement of the second part of the polynucleotide with respect to the nanopore against the applied force.

In some embodiments, the polynucleotide is double stranded and comprises a first strand and a second strand; the polynucleotide comprises a leader sequence located at a first end of the first strand or comprised in an adapter attached to the first end of the first strand; the first strand and the second strand are attached together by a hairpin adapter attached to (i) the second end of the first strand and (ii) a first end of the second strand; and the polynucleotide binding protein is stalled at a second end of the second strand or is stalled on an adapter at the second end of the second strand of the polynucleotide.

In some embodiments, a) the first part of the polynucleotide comprises (i) the part of the first stand between the leader sequence and the hairpin adapter, (ii) the hairpin adapter, and (iii) the part of the second strand between the hairpin adapter and the polynucleotide binding protein; and the second part of the polynucleotide is the same as the first part of the polynucleotide; and b) the polynucleotide binding protein is orientated on the polynucleotide such that the polynucleotide binding protein controls the movement of the second part of the polynucleotide back through or across the nanopore against the applied force.

In some embodiments, the single stranded leader sequence is contacted with the nanopore, the first part of the polynucleotide is a first strand of the double stranded polynucleotide, the second part of the polynucleotide is the second strand of the double stranded polynucleotide and the polynucleotide binding protein controls the movement of the second part of the polynucleotide with respect to the nanopore.

In some embodiments, the polynucleotide comprises a portion which is complementary to a tag sequence, wherein preferably the tag sequence is attached to the nanopore. In some embodiments, the polynucleotide comprises a portion having an oligonucleotide hybridised thereto, and wherein the oligonucleotide comprises: (a) a hybridising portion for hybridising to the polynucleotide and (b) (i) a portion complementary to a tag sequence or (ii) an affinity molecule capable of binding to a tag.

In some embodiments, the polynucleotide is double stranded and the portion which is complementary to a tag sequence is a portion of the first strand of the polynucleotide and/or the portion having an oligonucleotide hybridised thereto is a portion of the first strand of the polynucleotide. In some embodiments, the second strand hybridises to the tag sequence as the first strand moves with respect to the nanopore. In some embodiments, movement of the first strand with respect to the nanopore is temporarily paused to allow hybridisation of the second strand to the tag sequence.

In some embodiments, the second strand comprises a leader sequence that is hybridised to the first strand such that it is captured by the nanopore only after the first strand has moved through the nanopore.

In some embodiments, prior to step (i) a polynucleotide binding protein is stalled on the polynucleotide or an adapter bound thereto; and step (iii) further comprises a step of destalling the polynucleotide binding protein. In some embodiments, destalling the polynucleotide binding protein comprises applying a destalling force to the polynucleotide, wherein said destalling force is lower in magnitude and/or of opposite direction to (a) the force applied in step (ii a) and/or (b) the read force, wherein the read force is the force applied whilst the polynucleotide binding protein controls the movement of a second part of the polynucleotide with respect to the nanopore and the measurements to determine one or more characteristics of the polynucleotide are taken. In some embodiments, destalling the polynucleotide binding protein comprises stepping the applied force one or more times between the destalling force and the read force.

In some embodiments, the polynucleotide comprises a blocking moiety to prevent a polynucleotide binding protein from disengaging from the polynucleotide. In some embodiments, a) step (i) comprises contacting a leader sequence at the first end of the polynucleotide with the nanopore and a polynucleotide binding protein is stalled at a second end of the polynucleotide or on an adapter attached to the second end of the polynucleotide; and b) the blocking moiety is positioned between the polynucleotide binding protein and the second end of the polynucleotide thereby preventing the polynucleotide binding protein from disengaging from the polynucleotide at the second end of the polynucleotide.

In some embodiments, the first part of the polynucleotide has a length of at least 1000 kB.

In some embodiments, in step (ii a) the free movement of the polynucleotide with respect to the nanopore is governed by an ultra-fast polynucleotide-handling enzyme.

In some embodiments of the methods described above and herein, the polynucleotide binding protein, preferably a helicase, is stalled at a stalling site comprising one or more stalling units independently selected from:
- a polypeptide secondary structure, preferably a G-quadruplex (TBA);
- a nucleic acid analog, preferably selected from peptide nucleic acid (PNA), glycerol nucleic acid (GNA), threose nucleic acid (TNA), locked nucleic acid (LNA), bridged nucleic acid (BNA) and abasic nucleotides;
- spacer units selected from nitroindoles, inosines, acridines, 2-aminopurines, 2-6-diaminopurines, 5-bromo-deoxyuridines, inverted thymidines (inverted dTs), inverted dideoxy-thymidines (ddTs), dideoxy-cytidines (ddCs), 5-methylcytidines, 5-hydroxymethylcytidines, 2'-O-Methyl RNA bases, Iso-deoxycytidines (Iso-dCs), Iso-deoxyguanosines (Iso-dGs), C3 (OC₃H₆OPO₃) groups, photo-cleavable (PC) [OC₃H₆-C(O)NHCH₂-C₆H₃NO₂-CH(CH₃)OPO₃] groups, hexandiol groups, spacer 9 (iSp9) [(OCH₂CH₂)₃OPO₃] groups, more spacer 18 (iSp18) [(OCH₂CH₂)₆OPO₃] groups; and thiol connections; and
- fluorophores, avidins such as traptavidin, streptavidin and neutravidin, and/or biotin, cholesterol, methylene blue, dinitrophenols (DNPs), digoxigenin and/or anti-digoxigenin and dibenzylcyclooctyne groups.

The disclosure also relates to a method of selectively characterising polynucleotides of a desired length. The methods comprise contacting a nanopore such as a transmembrane nanopore with a sample of the polynucleotides. A first part of a polynucleotide in the sample is allowed to translocate freely through or across the nanopore under an applied force such as a potential or under a force applied by an enzyme which permits the translocation of a polynucleotide through the pore e.g. at close to voltage-driven translocation speeds (discussed further herein). The time for the first part of a polynucleotide in the sample to translocate through or across the nanopore is determined in order to assess the approximate length of the polynucleotide. Where the polynucleotide is not of the desired length and is therefore incorrect, the polynucleotide is ejected from the nanopore. A second polynucleotide in the sample can then be assessed without wasting time and reagents in unproductive characterisation of the incorrect polynucleotide. This process can be repeated as necessary until a polynucleotide in the sample is assessed and is found to be of the desired length. Once a polynucleotide of the desired length is identified, the movement of a second part of the polynucleotide through or across the nanopore is controlled and measurements of the second part of the polynucleotide are taken as it moves with respect to the pore in order to determine one or more characteristics of the polynucleotide. By taking measurements of characteristics of the polynucleotide only once a polynucleotide of the desired length has been identified, the method provides the selective characterisation of polynucleotides of a desired length.

The disclosed methods are particularly amenable to methods in which a polynucleotide is moved through a nanopore or through a structure containing a nanopore, e.g. a well in a detector chip.

Also disclosed is a method of selectively sequencing polynucleotides of a desired length, the method comprising:
(i) contacting a transmembrane nanopore with a sample of polynucleotides;
(ii) determining the time taken for a first part of a polynucleotide in the sample to translocate freely through the nanopore under an applied potential, in order to assess the approximate length of the polynucleotide;
(iii) where the polynucleotide is not of the desired length, ejecting the polynucleotide from the nanopore and repeating steps (ii) and (iii); or where the polynucleotide is of the desired length, controlling the movement of a second part of the polynucleotide through the nanopore using a polynucleotide binding protein and taking measurements as the second part of the polynucleotide moves through the nanopore to determine the sequence of the polynucleotide, thereby selectively sequencing polynucleotides of a desired length.

In some embodiments, an adapter is attached to one or both ends of the polynucleotides in the sample prior to step (i).

In some embodiments, the polynucleotide binding protein capable of controlling the movement of a polynucleotide is bound to the adapter attached to at least one end of the polynucleotide.

In some embodiments, the polynucleotides in the sample are double stranded.

In some embodiments, the polynucleotide binding protein capable of controlling the movement of a polynucleotide is bound to one end of at least one strand of the double stranded polynucleotides.

In some embodiments, a hairpin adapter is attached to one end of the double stranded polynucleotides and an adapter comprising a single stranded leader sequence is attached to the other end of the double stranded polynucleotides, and wherein the polynucleotide binding protein capable of controlling the movement of a polynucleotide is bound to the hairpin adapter. In some embodiments, in step (ii) the single stranded leader sequence is captured by the transmembrane nanopore under the applied potential, the first part of the polynucleotide is a first strand of the double stranded polynucleotide, the second part of the polynucleotide is the second strand of the double stranded polynucleotide and the polynucleotide binding protein controls the movement of the second part of the polynucleotide through the transmembrane nanopore.

In some embodiments, the polynucleotide comprises a single stranded leader sequence at one end and has a polynucleotide binding protein bound thereto at the other end on the same strand of the polynucleotide. In some embodiments, the single stranded leader sequence is captured by the transmembrane nanopore under the applied potential, the first part of the polynucleotide is the part between the leader polynucleotide and the polynucleotide binding protein, the polynucleotide binding protein is orientated on the polynucleotide such that the polynucleotide binding protein controls the movement of the second part of the polynucleotide back through the nanopore against the applied potential, and the second part of the polynucleotide is the same as the first part of the polynucleotide.

In some embodiments, the polynucleotide is double stranded and comprises a single stranded leader sequence at one end of a first strand of the double stranded polynucleotide and has polynucleotide binding protein bound thereto at one end of the second strand of the double stranded polynucleotide.

In some embodiments, a portion of the second strand of the double stranded polynucleotide comprises a sequence complementary to a tag sequence, or wherein an oligonucleotide is hybridised to the second strand, wherein the oligonucleotide comprises: (a) a portion complementary to a portion of the second strand and (b) (i) a portion complementary to a tag sequence or (ii) an affinity molecule capable of binding to a tag.

In some embodiments, the first part of the polynucleotide is at least a portion of the first strand and the second part of the polynucleotide is at least a portion of the second strand. In some embodiments, the oligonucleotide or the second strand is bound to a tag sequence attached to the nanopore. In some embodiments, the second strand hybridises to the tag sequence as the first strand moves through the nanopore. In some embodiments, movement of the first strand through the transmembrane nanopore is temporarily paused to allow hybridisation of the second strand to the tag sequence. In some embodiments, the second strand comprises a leader sequence that is hybridised to the first strand such that it is captured by the nanopore only after the first strand has moved through the nanopore.

### Brief Description of the Figures

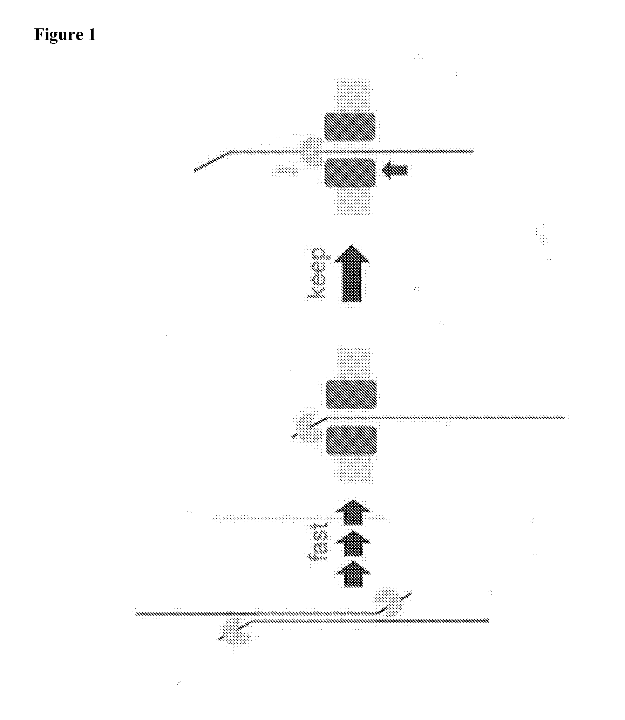

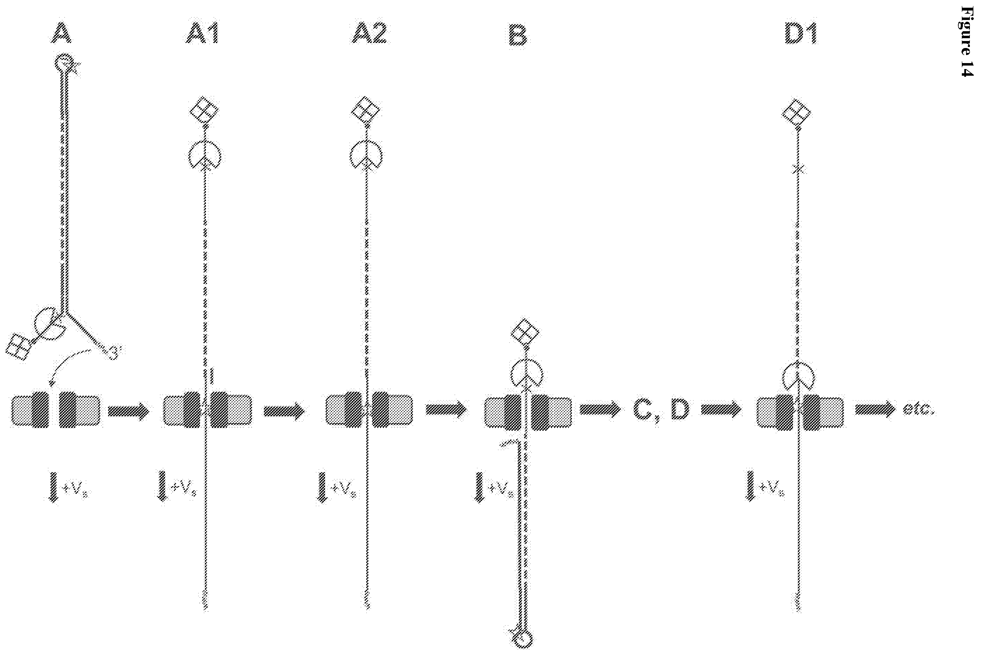

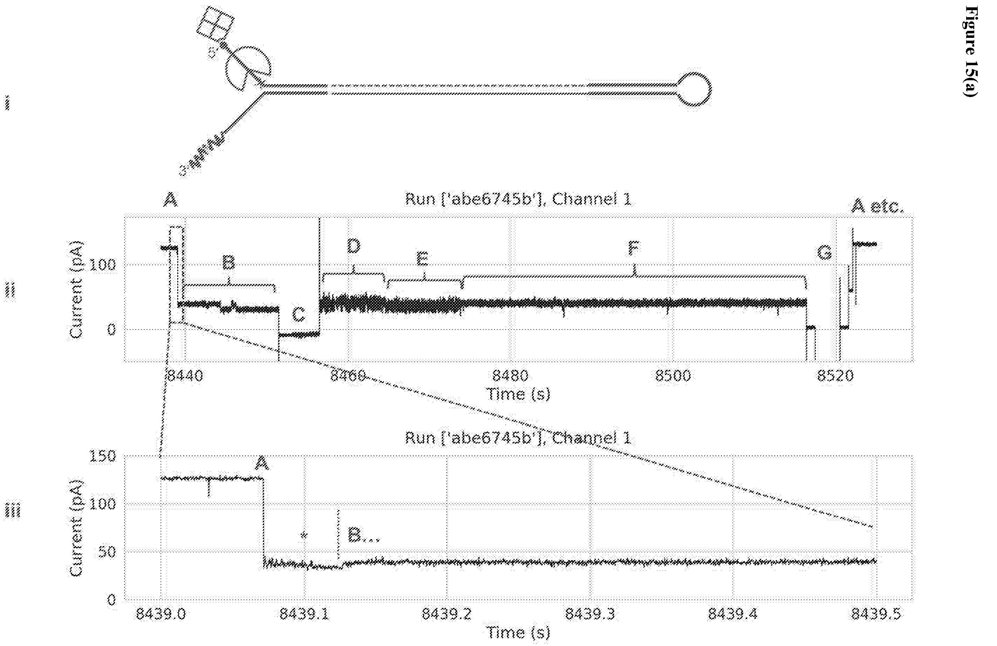

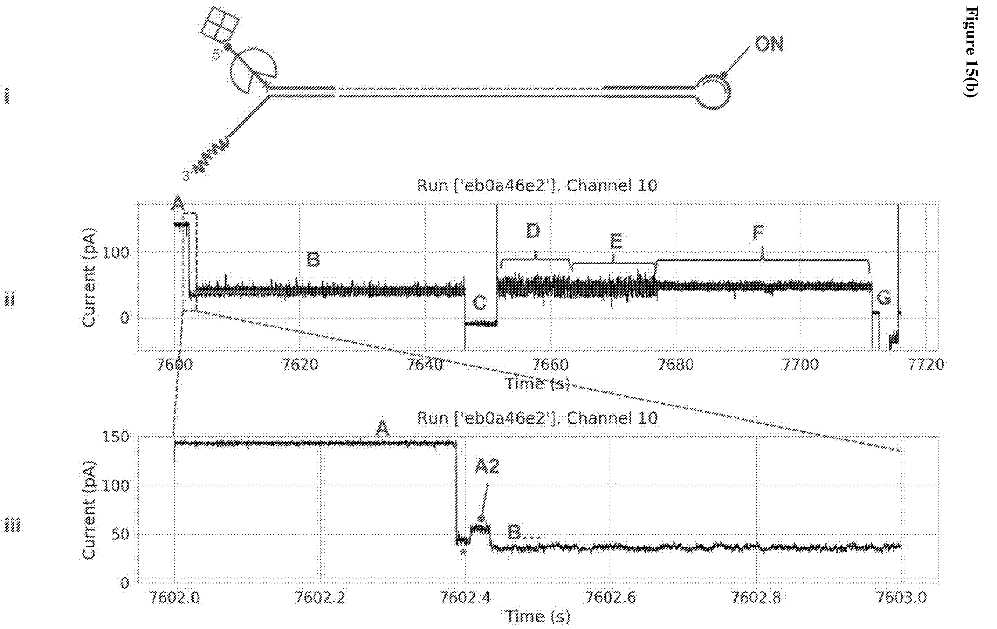

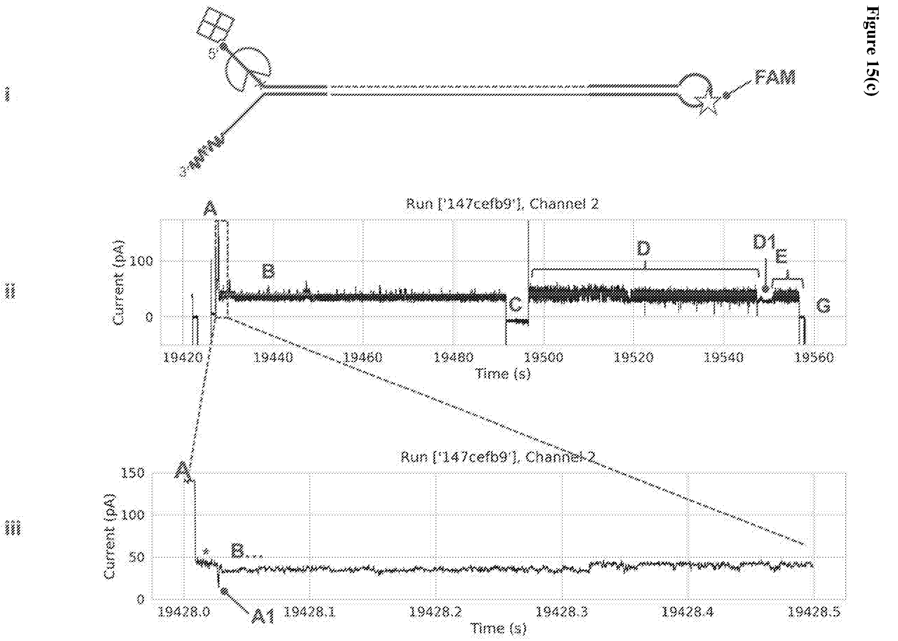

**Figure 1****.** Schematic of an embodiment of the methods provided herein. (i) An asymmetric double stranded polynucleotide is added to a nanopore system. (ii) A leader comprised in an adapter attached to the double-stranded polynucleotide is captured by a nanopore. A polynucleotide binding protein, stalled with a stalling chemistry, is located at the distal end of the freely translocating first strand of the polynucleotide. The first strand of the polynucleotide freely translocates through the nanopore until it reaches the polynucleotide binding protein. The time taken for the first strand to freely translocate is calculated. A decision is made whether to reject the polynucleotide based on the duration of the freely translocating polynucleotide. (iii) If the decision is made to accept the polynucleotide, the polynucleotide binding protein is permitted to pull the polynucleotide back through the nanopore, controlling the movement of the polynucleotide. If the decision is made to reject the polynucleotide, it is ejected from the nanopore, for example by reversing the voltage potential. An example of this embodiment is discussed in example 1.

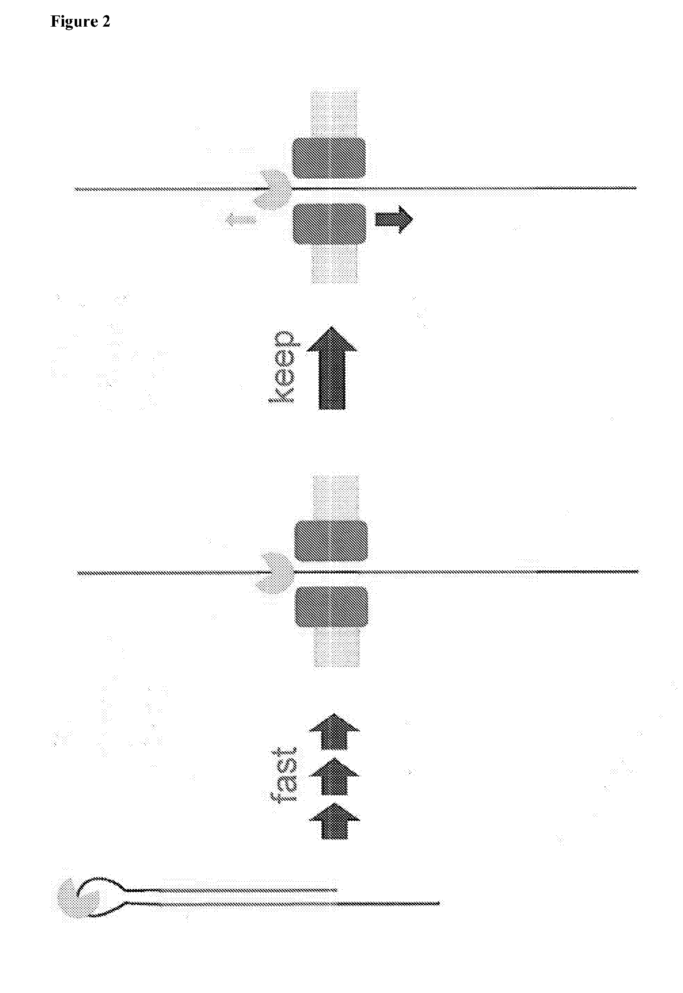

**Figure 2**. Schematic of an embodiment of the methods provided herein. (i) A double stranded polynucleotide with the two strands linked by a hairpin adapter comprising a polynucleotide binding protein is added to a nanopore system. (ii) A leader comprised in an adapter attached to a first strand of the double-stranded polynucleotide is captured by a nanopore. The first strand of the polynucleotide freely translocates through the nanopore until it reaches the polynucleotide binding protein. The nanopore separates the duplex. The time taken for the first strand to freely translocate is calculated. A decision is made whether to reject the polynucleotide based on the duration of the freely translocating polynucleotide. (iii) If the decision is made to accept the polynucleotide, the polynucleotide binding protein is permitted to control the movement of the second strand with respect to the nanopore. If the decision is made to reject the polynucleotide, it is ejected from the nanopore, for example by reversing the voltage potential.

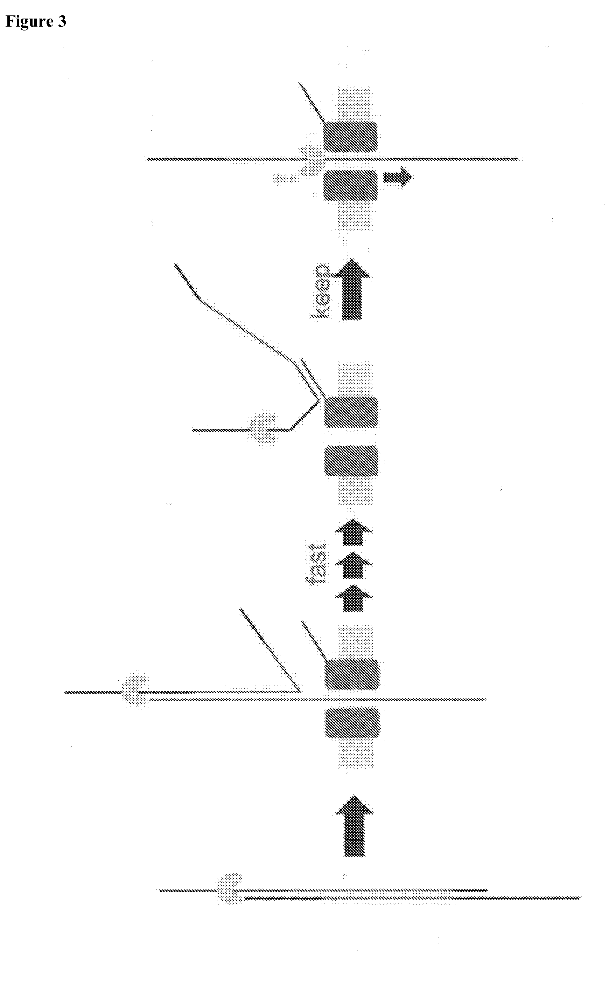

**Figure 3**. Schematic of an embodiment of the methods provided herein. (i) An asymmetric double stranded polynucleotide is added to a nanopore system. (ii) A leader sequence comprised in an adapter attached to a first strand of the double stranded polynucleotide is captured by the nanopore. The first strand of the polynucleotide freely translocates through the nanopore. The nanopore separates the duplex. (iii) As the nanopore separates the duplex, a binding site (e.g. a polynucleotide sequence) is revealed in the second strand of the double stranded polynucleotide. This binding site attaches to a tag (e.g. a complementary oligonucleotide) attached to the nanopore. The time taken for the first strand to fully translocate through the nanopore is determined, e.g. by measuring the duration from a start signal (such as caused by a blockage in the open-pore current level caused by the double-stranded polynucleotide) to a stop signal (such as a return to the open-pore current level, or the capture of an adapter attached to the second strand of the polynucleotide. (iv) An adapter attached to the second strand of the polynucleotide is captured by the nanopore. A decision is made whether to reject the polynucleotide based on the duration of the freely translocating first strand. If the decision is made to keep the polynucleotide, a polynucleotide binding protein controls the movement of the second strand. If a decision is made to reject the second strand, it is ejected from the nanopore. This embodiment is described further herein, including in Example 2.

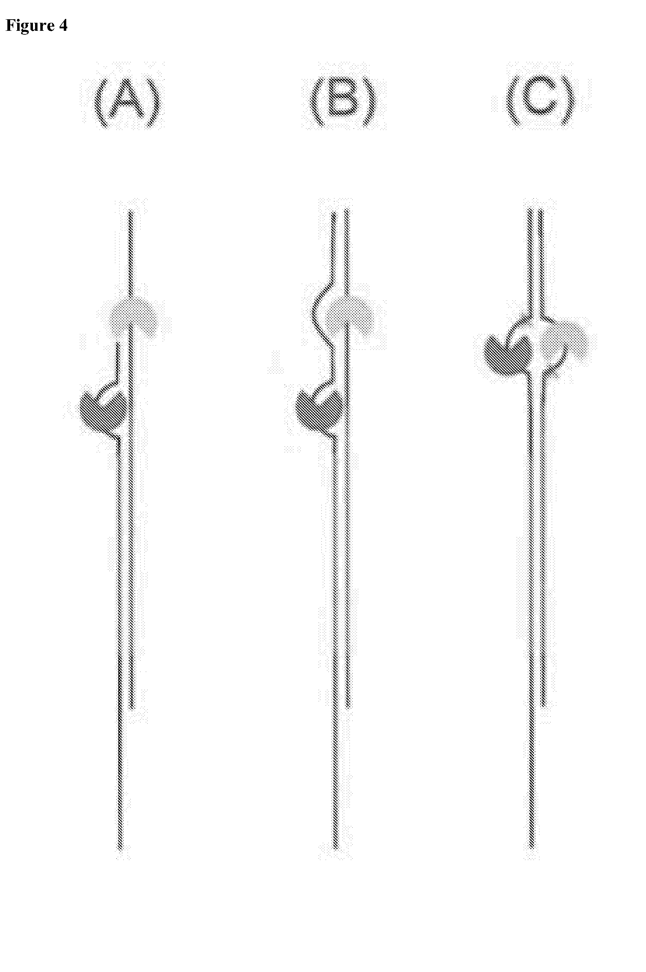

**Figure 4****.** Schematics of further embodiments. (A) shows a variant of the embodiment of Figure 3. A pausing moiety (such as a polynucleotide binding protein, G-quadruplex (TBA), BNA / LNA moiety, a spacer such as C3 or Sp18, or a blocking moiety such as biotin/desthiobiotin bound to streptavidin or monovalent streptavidin) is located at the distal end of the freely translocating first part of the polynucleotide, creating a pause and a detectable signal when the first part of the polynucleotide translocates through or across the nanopore. (B) A variant of (A). A sequencing adapter leader on the second strand of the double-stranded polynucleotide is only exposed after the first strand has fully translocated through the nanopore. This promotes capture of the Y adapter on the first strand. (C) A variant of (B), in which the sequencing adapter contains two binding sites for a polynucleotide binding protein.

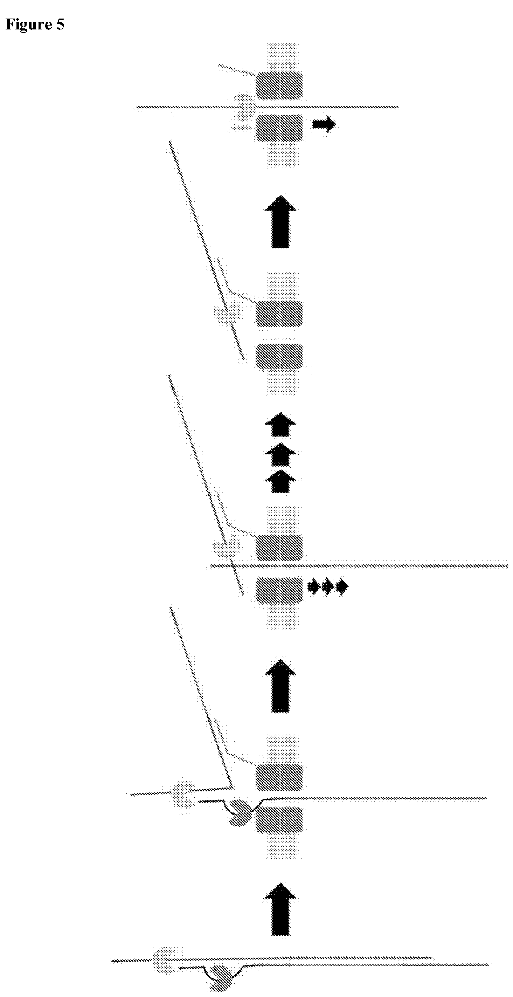

**Figure 5**. Schematic of an embodiment of the methods provided herein using the setup of Figure 4(A). (i) An asymmetric double stranded polynucleotide is added to a nanopore system. (ii) A leader sequence comprised in an adapter attached to a first strand of the asymmetric double stranded polynucleotide is captured by the nanopore. The first strand of the polynucleotide freely translocates through the nanopore. The nanopore separates the duplex. A pausing moiety such as a polynucleotide binding protein, G-quadruplex (TBA), BNA / LNA stall, a spacer such as C3 or Sp18, or a blocking moiety such as biotin/desthiobiotin bound to streptavidin, or monovalent streptavidin is located at the distal end of the freely translocating polynucleotide. (iii) As the nanopore separates the duplex, the pausing moiety creates a pause and a signal. A binding site (e.g. a polynucleotide sequence) is revealed on the second strand of the double stranded polynucleotide. This binding site attaches to a tag (e.g. a complementary oligonucleotide) attached to the nanopore, whilst the first strand is paused. The time taken for the first strand to fully translocate through the nanopore is determined, e.g. by measuring the duration from a start signal (such as caused by a blockage in the open-pore current level caused by the double-stranded polynucleotide) to a stop signal (such as a return to the open-pore current level, or the capture of an adapter attached to the second strand of the polynucleotide. (iv) An adapter attached to the second strand of the polynucleotide is captured by the nanopore. A decision is made whether to reject the polynucleotide based on the duration of the freely translocating first strand. If the decision is made to keep the polynucleotide, a polynucleotide binding protein controls the movement of the second strand. If a decision is made to reject the second strand, it is ejected from the nanopore. This embodiment is described further herein, including in Example 3.

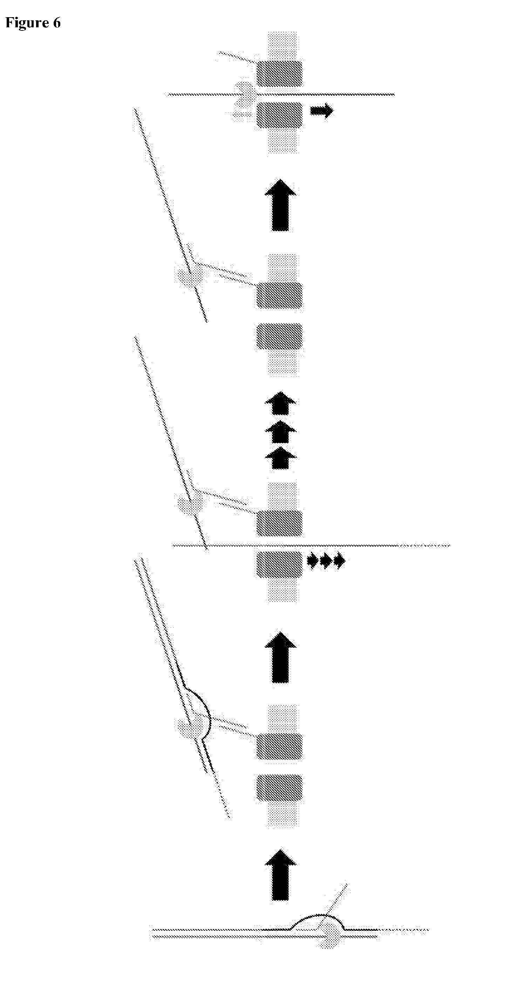

**Figure 6****.** Schematic of an embodiment of the methods provided herein. (i) An asymmetric double stranded polynucleotide is added to a nanopore system. (ii) A binding site (e.g. a sequence of an oligonucleotide hybridised to a strand of a Y-adapter) attached to the double-stranded polynucleotide strand attaches to a tag (e.g. by hybridising to a complementary oligonucleotide) attached to the nanopore, keeping the second strand localised to the nanopore. (iii) A leader sequence comprised in an adapter attached to the first strand of the polynucleotide is captured by the nanopore. The first strand of the polynucleotide freely translocates through the nanopore. The nanopore separates the duplex, revealing a leader for capture of the second strand. (iv) The time taken for the first strand to fully translocate through the nanopore is determined, e.g. by measuring the duration from a start signal (such as caused by a blockage in the open-pore current level caused by the double-stranded polynucleotide) to a stop signal (such as a return to the open-pore current level, or the capture of an adapter attached to the second strand of the polynucleotide. (v). An adapter attached to the second strand of the polynucleotide is captured by the nanopore. A decision is made whether to reject the polynucleotide based on the duration of the freely translocating first strand. If the decision is made to keep the polynucleotide, the polynucleotide binding protein controls the movement of the second strand. If a decision is made to reject the second strand, it is ejected from the nanopore. This embodiment is described further herein, including in Example 4.

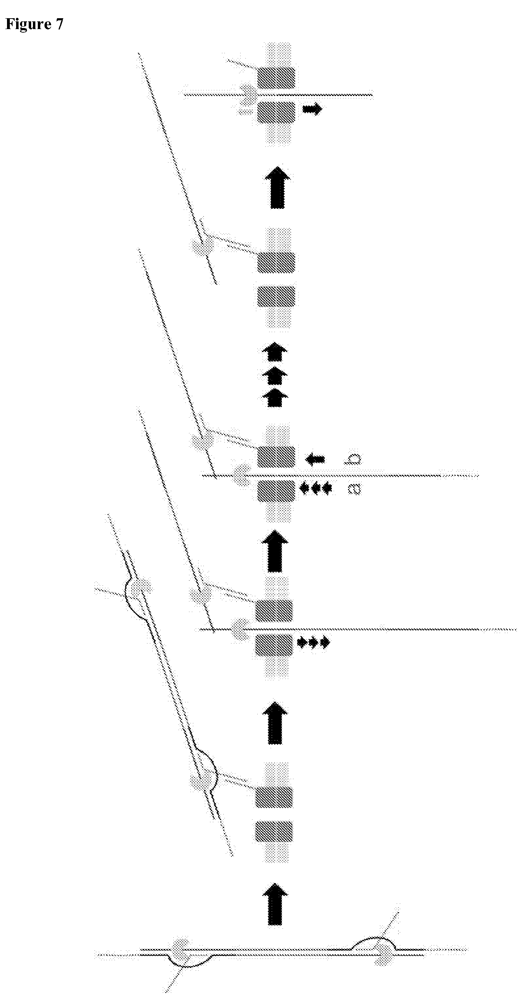

**Figure 7****.** Schematic of an embodiment of the methods provided herein. A symmetric double stranded polynucleotide, containing a binding site (e.g. a sequence in a Y-adapter) attached to the double-stranded polynucleotide strand that attaches to a tag (e.g. by hybridising to a complementary oligonucleotide) attached to a nanopore, is added to a nanopore system. The hybridisation keeps the second strand localised to the nanopore. A leader sequence comprised in an adapter attached to the first strand is captured by the nanopore. The first strand of the polynucleotide freely translocates through the nanopore. The nanopore separates the duplex. A polynucleotide binding protein is encountered at the distal end of the freely translocating polynucleotide, creating a pause and a signal. The time taken for the first strand to translocate through the nanopore is determined. A decision is made whether to reject the polynucleotide based on the duration of the freely translocating first strand. (**Step a**) The first strand may be ejected from the nanopore e.g. by a reversal of applied potential, allowing the adapter of the second strand to be captured and the polynucleotide binding protein to control the movement of the second strand. (**Step b**) The polynucleotide binding protein attached to the first strand is permitted to overcome stalling chemistry in the Y adapter. The first strand polynucleotide binding protein controls the movement of the polynucleotide as it pulls the DNA back out of the nanopore. The adapter of the second strand is captured and the polynucleotide binding protein attached to the second strand controls the movement of the second strand with respect to the nanopore.

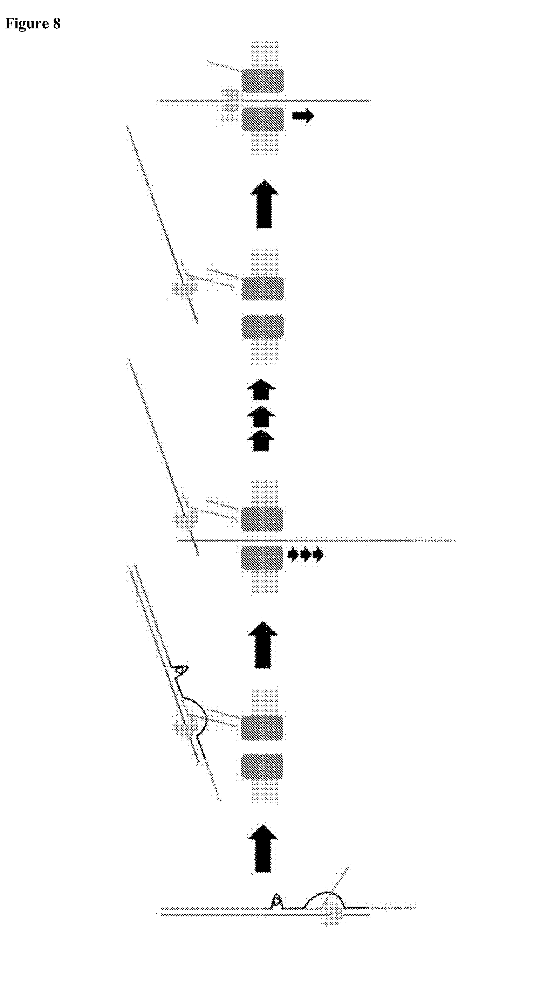

**Figure 8****.** Schematic of an embodiment of the methods provided herein. A variant in which an asymmetric double stranded polynucleotide containing a sequence in a Y-adapter attached to the polynucleotide strand that attaches to a tag (e.g. by hybridising to a complementary oligonucleotide) attached to a nanopore is provided. The hybridisation keeps the second strand localised to the nanopore. A leader comprised in an adapter attached to the first strand is captured by the nanopore. A G-quadruplex (TBA), BNA / LNA stall, a spacer such as C3 or Sp18, or a blocking moiety such as biotin/desthiobiotin bound to streptavidin, or monovalent streptavidin is comprised in the Y adapter of the first strand, and creates a pause when it encounters the nanopore. A binding site (e.g. a sequence in the Y-adapter on the second strand of the double stranded polynucleotide) hybridises to a tag (e.g. a complementary oligonucleotide) attached to the nanopore whilst the first strand is paused. The first strand of the polynucleotide freely translocates through the nanopore. The nanopore separates the duplex, revealing a leader for capture of the second strand. The time taken for the first strand to translocate through the nanopore is determined. A decision is made whether to reject the polynucleotide based on the duration of the freely translocating first strand. If retained, the adapter of the second strand is captured, and a polynucleotide binding protein on the second strand controls the movement of the second strand with respect to the nanopore.

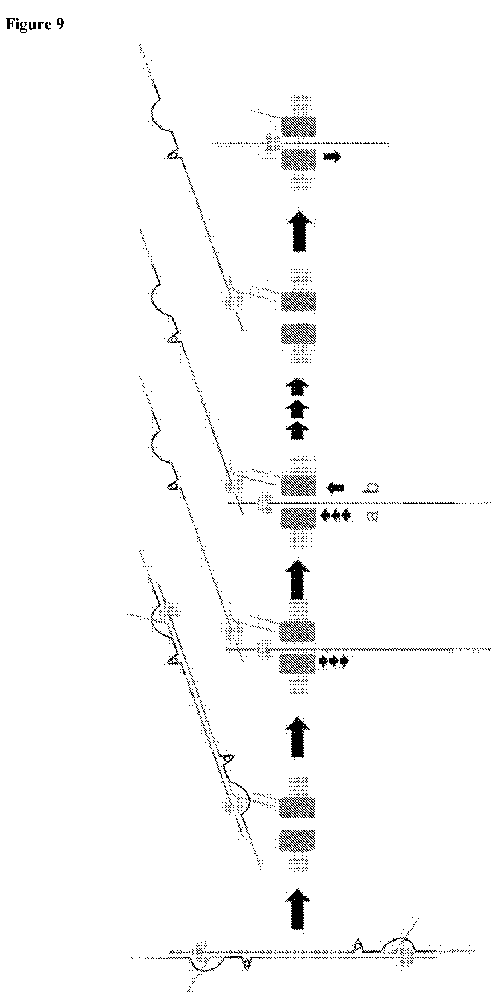

**Figure 9****.** Schematic of an embodiment of the methods provided herein. A variant in which a symmetric double stranded polynucleotide, containing a sequence in a Y-adapter attached to the polynucleotide strand that attaches to a tag (e.g. by hybridising to a complementary oligonucleotide) attached to a nanopore is provided. The hybridisation keeps the second strand localised to the nanopore. A leader comprised in an adapter attached to the first strand is captured by the nanopore. A G-quadruplex (TBA), BNA / LNA stall, a spacer such as C3 or Sp18, or a blocking moiety such as biotin/desthiobiotin bound to streptavidin, or monovalent streptavidin is comprised in the Y adapter of the first strand, and creates a pause when it encounters the nanopore. A binding site (e.g. a sequence in the Y-adapter on the second strand of the double stranded polynucleotide) hybridises to a tag (e.g. a complementary oligonucleotide) attached to the nanopore whilst the first strand is paused. The first strand of the polynucleotide freely translocates through the nanopore. The nanopore separates the duplex, revealing a leader for capture of the second strand. A polynucleotide binding protein is located at the distal end of the freely translocating polynucleotide, creating a pause and a signal when it encounters the nanopore. The time taken for the first strand to translocate through the nanopore is determined. A decision is made whether to reject the polynucleotide based on the duration of the freely translocating first strand. (**Step a**) The first strand may be ejected from the nanopore by a reversal of applied potential. The adapter of the second strand is captured, and a polynucleotide binding protein on the second strand used to control the movement of the second strand. (**Step b**) The first strand polynucleotide binding protein is permitted to overcome stalling chemistry in the Y-adapter. The first strand polynucleotide binding protein controls the movement of the polynucleotide as it pulls the DNA back out of the nanopore. The adapter of the second strand is captured and the polynucleotide binding protein controls the movement of the second strand with respect to the nanopore.

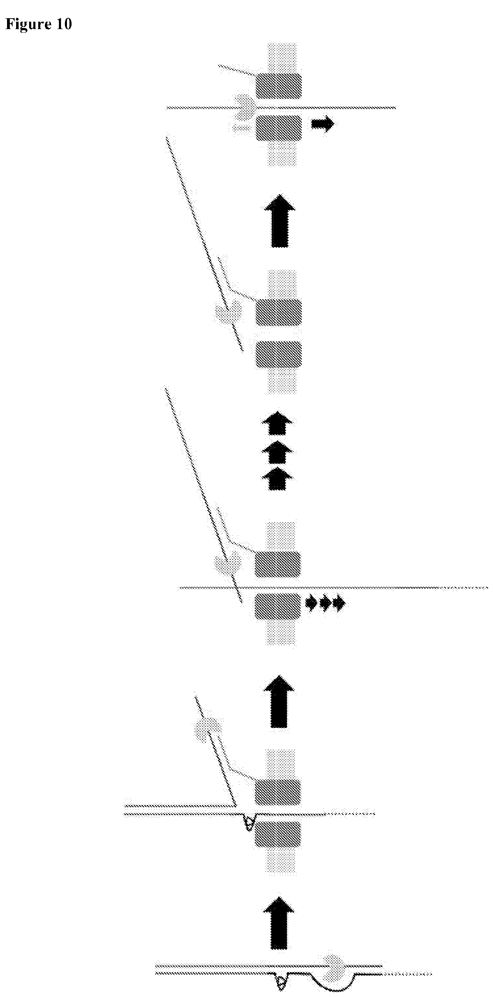

**Figure 10****.** Schematic of an embodiment of the methods provided herein. A variant in which an asymmetric double stranded polynucleotide is added to a nanopore system. A leader comprised in an adapter attached to the first strand of the asymmetric double stranded polynucleotide is captured by the nanopore. The first strand of the polynucleotide freely translocates through the nanopore. The nanopore separates the duplex, revealing a leader for capture of the second strand. A G-quadruplex (TBA), BNA / LNA stall, a spacer such as C3 or Sp18, or a blocking moiety such as biotin/desthiobiotin bound to streptavidin, or monovalent streptavidin is comprised in the Y adapter of the first strand, and creates a pause when it encounters the nanopore. A sequence is revealed on the second strand of the double stranded polynucleotide, this sequence hybridises to a complementary oligonucleotide chemically attached to the nanopore, whilst the first strand is paused. Optionally, a G-quadruplex (TBA), BNA / LNA stall, a spacer such as C3 or Sp18, or a blocking moiety such as biotin/desthiobiotin bound to streptavidin, or monovalent streptavidin is encountered at the distal end adapter of the freely translocating polynucleotide, creating a pause and a signal. The time taken for the first strand to translocate through the nanopore is determined. A decision is made whether to reject the polynucleotide based on the duration of the freely translocating first strand. If retained, the adapter of the second strand is captured, and a polynucleotide binding protein on the second strand controls the movement of the second strand with respect to the nanopore.

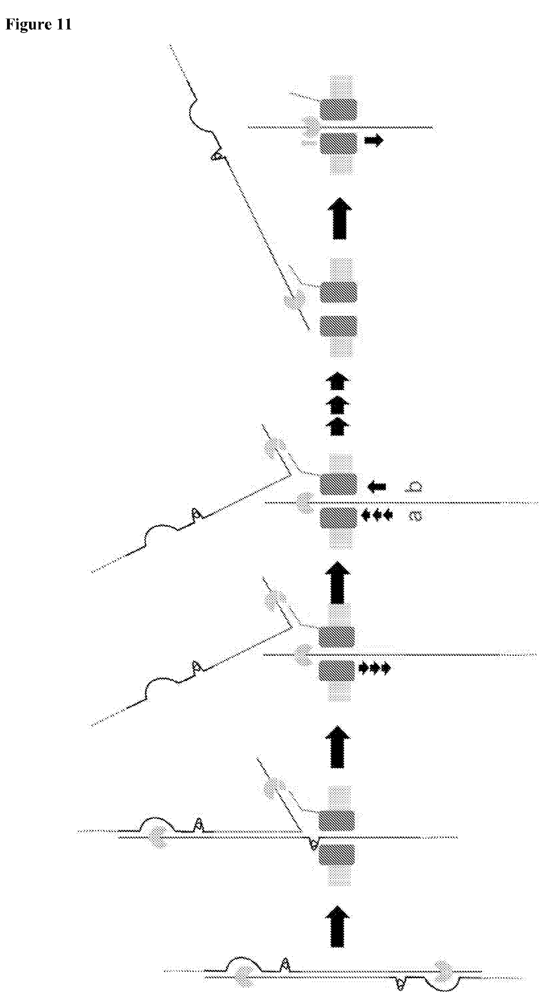

**Figure 11****.** Schematic of an embodiment of the methods provided herein. A variant in which a symmetric double stranded polynucleotide is added to a nanopore system. A leader of the first strand of an asymmetric double stranded polynucleotide is captured by the nanopore. The first strand of the polynucleotide freely translocates through the nanopore. The nanopore separates the duplex. A G-quadruplex (TBA), a BNA / LNA stall, a spacer such as C3 or Sp18, or a blocking moiety such as biotin/desthiobiotin bound to streptavidin, or monovalent streptavidin is comprised in in the Y-adapter of the first strand, creating a pause when it is encountered by the nanopore. A sequence is revealed on the second strand of the double stranded polynucleotide, this sequence hybridises to a complementary oligonucleotide chemically attached to the nanopore, whilst the first strand is paused. The first strand of the polynucleotide freely translocates through the nanopore. The nanopore separates the duplex. A polynucleotide binding protein is encountered at the distal end of the freely translocating polynucleotide, creating a pause and a signal. The time taken for the first strand to translocate through the nanopore is determined. A decision is made whether to reject the polynucleotide based on the duration of the freely translocating first strand. (**Step a**) The first strand may be is ejected from the nanopore by a reversal of applied potential. The adapter of the second strand is captured, and a polynucleotide binding protein on the second strand used to control the movement of the second strand. (**Step b**) The first strand polynucleotide binding protein is permitted to overcome stalling chemistry in the Y-adapter. The first strand polynucleotide binding protein controls the movement of the polynucleotide as it pulls the DNA back out of the nanopore. The adapter of the second strand is captured and the polynucleotide binding protein controls the movement of the second strand with respect to the nanopore.

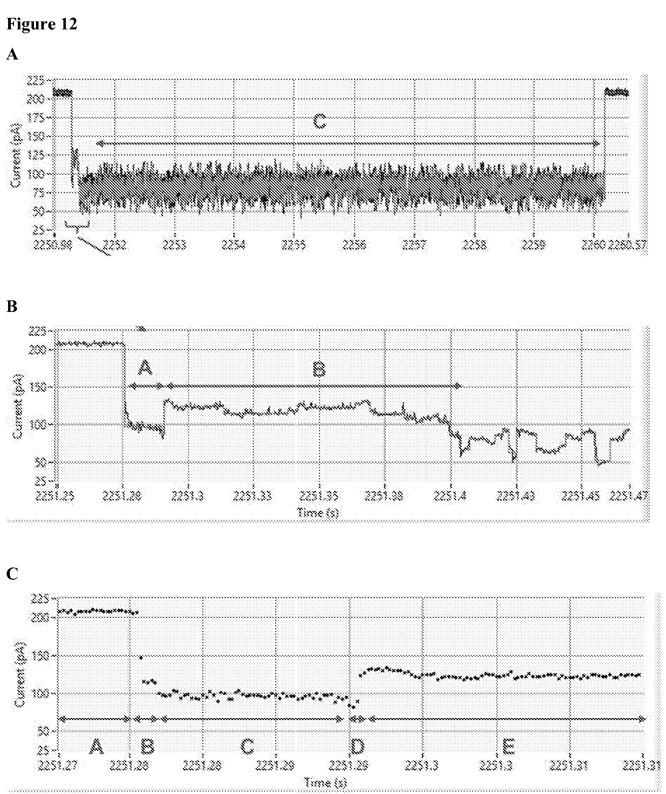

**Figure 12**. Data for example 5. **A**: The signal from the second part of the polynucleotide, movement controlled with a polynucleotide binding protein (labelled C). **B**: The first part of the polynucleotide, freely translocating (labelled A) and the signal from Sp18 spacers in the hairpin adapter (labelled B). **C**: A zoomed-in image of figure 12A, showing the signal from the open pore (labelled A); the signal from the drop from open pore to the freely translocating polynucleotide level (labelled B - this may also contain some signal from the Y-adapter; the signal from the freely translocating polynucleotide (labelled C); a drop to the pT level from the hairpin adapter (labelled D); and the signal from the Sp18 chemistry in the hairpin adapter (labelled E).

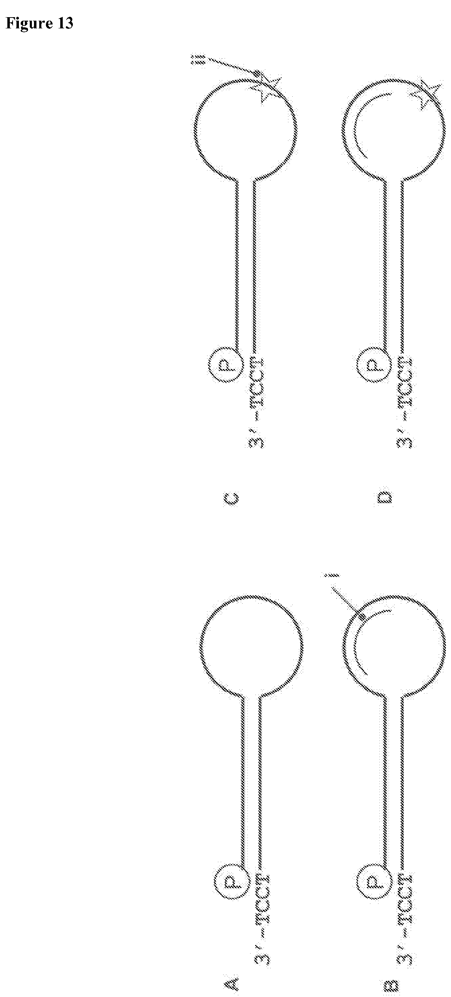

**Figure 13****.** Hairpin moities of the experiment described in Example 7 in which both strands of a polynucleotide analyte are first translocated without enzyme through a nanopore; then the enzyme is 'de-stalled'; then the enzyme controls the movement of both strands of the polynucleotide analyte out of the nanopore. Additional moieties in the hairpin introduce an additional signal during the initial enzyme-free capture phase. These moieties are depicted in the figure as follows:
   (A) No moiety in hairpin, as control.
   (B) Hairpin with oligonucleotide i hybridized to hairpin loop
   (C) Three consecutive fluorescein-dT bases ii in hairpin loop, denoted by star
   (D) per (C), but with oligonucleotide I hybridized to hairpin loop
**Figure 14****.** Schematic showing the capture and enzyme-free translocation of a double-stranded polynucleotide analyte bearing a hairpin moiety, in which the hairpin moiety optionally carries a bulky fluorophore and optionally an oligonucleotide hybridized to the hairpin loop. The schematic shows two additional detectable intermediates, A1 and A2, corresponding to the oligonucleotide hybridized to the hairpin loop atop the nanopore, with the fluorophore in the lumen of the nanopore, and to the fluorophore in the lumen of the nanopore alone. An additional state, D1, corresponds to the fluorophore in the lumen of the nanopore, and the enzyme moving over the fluorophore.
**Figure 15****.**
   **(a)** Data showing the identification of enzyme-free movement of a polynucleotide whose template and complement strands are joined through a hairpin moiety. The polynucleotide is guided through a nanopore via an applied potential prior to the enzyme-controlled movement step. The hairpin is that described in Figure 13, A. (i) Polynucleotide library ligated to sequencing adapter and hairpin adapter containing DNA only. (ii) Representative current-time trace for the molecule shown in (i). (iii) Expanded view of the boxed region shown in (ii), showing identification of open pore level A and stall level B. The asterisked region, which has a different shape and noise to B, and also by relation to the other representative molecules described in this Example, is presumed to arise from the enzyme-free translocation portion.
   **(b)** Data showing the identification of enzyme-free movement of a polynucleotide whose template and complement strands are joined through a hairpin moiety, where an oligonucleotide is hybridised to the hairpin. The polynucleotide is guided through a nanopore via an applied potential prior to the enzyme-controlled movement step. The hairpin is that described in Figure 13, B. (i) Polynucleotide library ligated to sequencing adapter and hairpin adapter containing DNA with oligonucleotide (ON) hybridised thereto. (ii) Representative current-time trace for the molecule shown in (i). (iii) Expanded view of the boxed region shown in (ii), showing identification of open pore level A and stall level B. An additional level A2 (described in Figure 14) arises from the hybridized oligonucleotide, when compared to the example shown in Figure 15a. Thus, the asterisked region corresponds to enzyme-free translocation.
   **(c)** Data showing the identification of enzyme-free movement of a polynucleotide whose template and complement strands are joined through a hairpin moiety, where a three bulky groups (three consecutive fluorescein-dT bases; FAM) are present in the hairpin. The polynucleotide is guided through a nanopore via an applied potential prior to the enzyme-controlled movement step. The hairpin is that described in Figure 13, C. (i) Polynucleotide library ligated to sequencing adapter and hairpin adapter containing fluorescein bases. (ii) Representative current-time trace for the molecule shown in (i). An additional level D1 is presumed to arise through slow movement of the enzyme over the bulky FAM region. (The complement region E is curtailed owing to the eject phase G, so state F is not seen in this example). (iii) Expanded view of the boxed region shown in (ii), showing identification of open pore level A and stall level B. An additional down-tick current level A1 of ~20 pA (described in Figure 14) arises from the FAM groups, when compared to the example shown in Figure 15a. Thus, the asterisked region corresponds to enzyme-free translocation.
   **(d)** Data showing the identification of enzyme-free movement of a polynucleotide whose template and complement strands are joined through a hairpin moiety, where a three bulky groups (three consecutive fluorescein-dT bases; FAM) are present in the hairpin and an oligonucleotide (ON) is hybridised thereto. The polynucleotide is guided through a nanopore via an applied potential prior to the enzyme-controlled movement step. The hairpin is that described in Figure 13, D. (i) Polynucleotide library ligated to sequencing adapter and hairpin adapter containing fluorescein bases (FAM), with oligonucleotide (ON) hybridised thereto. (ii) Representative current-time trace for the molecule shown in (i). An additional level D1 with current level down-ticks is presumed to arise through slow movement of the enzyme over the bulky FAM region. (iii) Expanded view of the boxed region shown in (ii), showing identification of open pore level A and stall level B. An additional down-tick current level A1 of ~20 pA (described in Figure 14) arises from the FAM groups, when compared to the example shown in Figure 15a and Figure 15c. The additional level A2 owing to the hybridized ON is also seen, through comparison to Figure 15b. Thus, the asterisked region corresponds to enzyme-free translocation.
   **(e)** Measurement of the duration of enzyme-free translocation of an *E. coli* test library. (i) Four representative examples from a random *E. coli* test library described in Example 7, in which the double-stranded polynucleotide is ligated to a sequencing adapter at one end and a hairpin moiety at the other. The hairpin moiety has an oligonucleotide hybridized thereto. The resultant polynucleotide therefore resembles that of Figure 15b, except the polynucleotide is of random length. The four examples shown are event-fitted current-time traces, which simplifies the raw data. Level A2 and the enzyme-free portion (denoted by an asterisk) are shown in each example. A threshold of 60 pA (dotted line) was used to demarcate the enzyme-free portion A2. The duration of the asterisked portion was therefore measured between the times the current crosses the 60 pA threshold between open pore level A and oligonucleotide level A2. (ii) Relationship between enzyme-controlled strand duration (measured as sum of periods D and E shown in Figure 15b, ii) and enzyme-free capture duration (measured as described in this Figure, part i), measured for 30 examples, and shown as a scatter plot. A linear regression line is shown with *R*² value 0.414, demonstrating positive correlation.

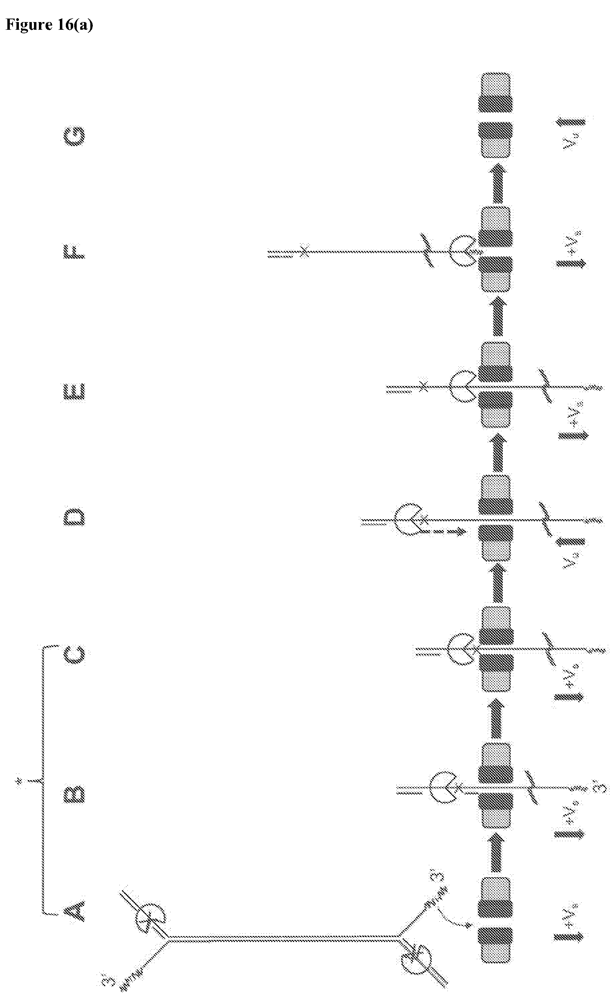

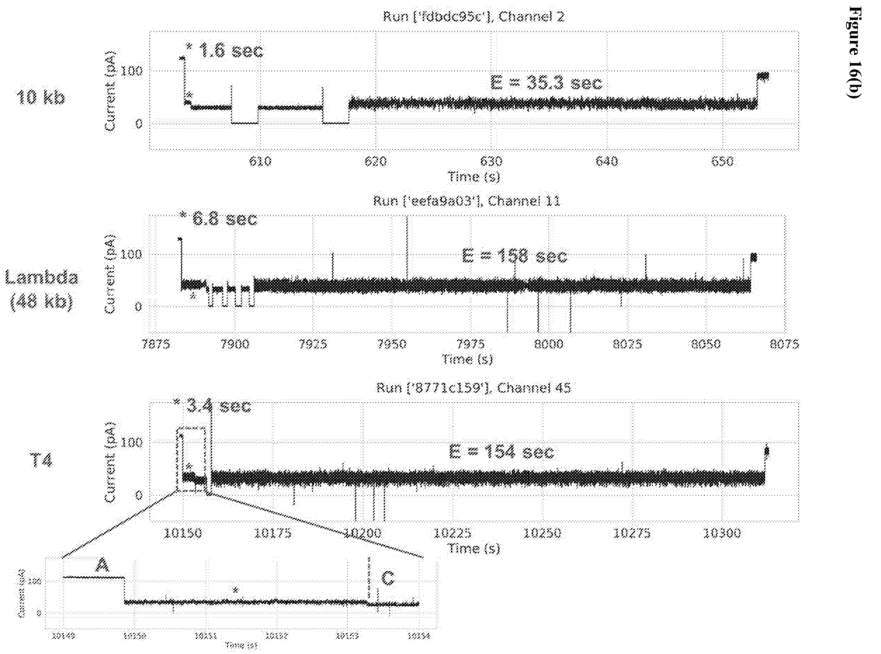

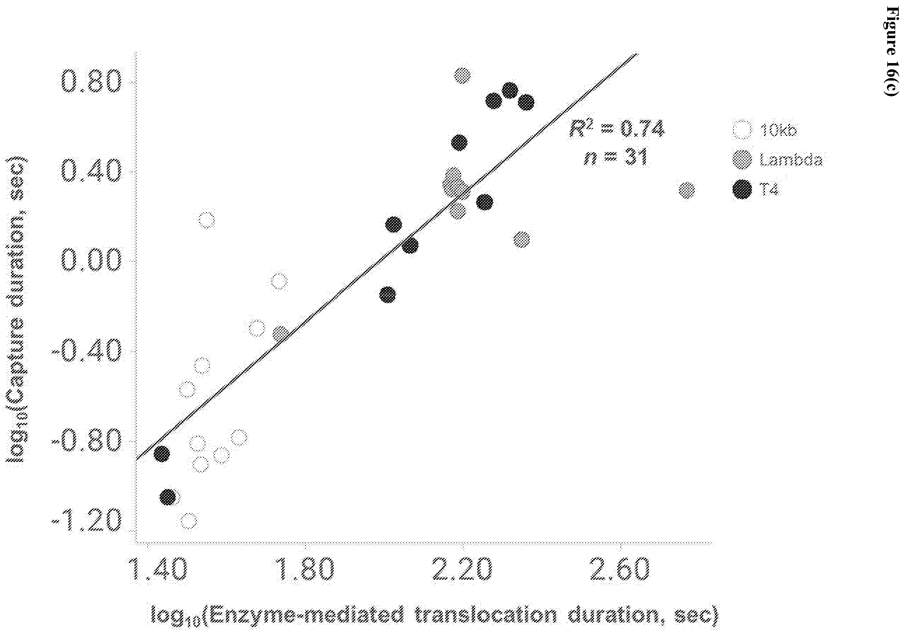

**Figure 16****.** **(a)** Experimental schematic. The 'entry' phase, used to measure enzyme-free translocation (between steps A and C) is marked with an asterisk. (b) Representative current-time traces for three library examples shown in Example 8: a 10 kb PCR fragment (top); bacteriophage lambda DNA (middle); and T4 DNA (bottom). Full-length reads of T4 DNA were not recorded, so an example part-fragment is shown. In each example, the 'entry' phase is marked with an asterisk and the enzyme-controlled phase marked **E**. Durations of each portion were measured by hand and are marked on the traces. An expanded view of the entry phase for the T4 example is shown. It is not possible to reliably detect the portion marked **B** per Figure 16a (blocker oligonucleotide atop pore). **(c)** Log-log scatter plot of the measured capture durations measured from 31 example traces described in Example 8. Markers are coloured in grayscale according to the library from which they were derived.

### Detailed Description

The present invention will be described with respect to particular embodiments and with reference to certain drawings but the invention is not limited thereto but only by the claims. Any reference signs in the claims shall not be construed as limiting the scope. Of course, it is to be understood that not necessarily all aspects or advantages may be achieved in accordance with any particular embodiment of the invention. Thus, for example those skilled in the art will recognize that the invention may be embodied or carried out in a manner that achieves or optimizes one advantage or group of advantages as taught herein without necessarily achieving other aspects or advantages as may be taught or suggested herein.

The invention, both as to organization and method of operation, together with features and advantages thereof, may best be understood by reference to the following detailed description when read in conjunction with the accompanying drawings. The aspects and advantages of the invention will be apparent from and elucidated with reference to the embodiment(s) described hereinafter. Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment.

It should be appreciated that "embodiments" of the disclosure can be specifically combined together unless the context indicates otherwise. The specific combinations of all disclosed embodiments (unless implied otherwise by the context) are further disclosed embodiments of the claimed invention.

In addition as used in this specification and the appended claims, the singular forms "a", "an", and "the" include plural referents unless the content clearly dictates otherwise. Thus, for example, reference to "a polynucleotide" includes two or more polynucleotides, reference to "a polynucleotide binding protein" includes two or more such proteins, reference to "a helicase" includes two or more helicases, reference to "a monomer" refers to two or more monomers, reference to "a pore" includes two or more pores and the like.

### Definitions

Where an indefinite or definite article is used when referring to a singular noun e.g. "a" or "an", "the", this includes a plural of that noun unless something else is specifically stated. Where the term "comprising" is used in the present description and claims, it does not exclude other elements or steps. Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 4th ed., Cold Spring Harbor Press, Plainsview, New York (2012); and Ausubel et al., Current Protocols in Molecular Biology (Supplement 114), John Wiley & Sons, New York (2016), for definitions and terms of the art. The definitions provided herein should not be construed to have a scope less than understood by a person of ordinary skill in the art.

"About" as used herein when referring to a measurable value such as an amount, a temporal duration, and the like, is meant to encompass variations of ± 20 % or ± 10%, more preferably ± 5 %, even more preferably ± 1 %, and still more preferably ± 0.1 % from the specified value, as such variations are appropriate to perform the disclosed methods.

"Nucleotide sequence", "DNA sequence" or "nucleic acid molecule(s)" as used herein refers to a polymeric form of nucleotides of any length, either ribonucleotides or deoxyribonucleotides. This term refers only to the primary structure of the molecule. Thus, this term includes double- and single-stranded DNA, and RNA. The term "nucleic acid" as used herein, is a single or double stranded covalently-linked sequence of nucleotides in which the 3' and 5' ends on each nucleotide are joined by phosphodiester bonds. The polynucleotide may be made up of deoxyribonucleotide bases or ribonucleotide bases. Nucleic acids may be manufactured synthetically in vitro or isolated from natural sources. Nucleic acids may further include modified DNA or RNA, for example DNA or RNA that has been methylated, or RNA that has been subject to post-translational modification, for example 5'-capping with 7-methylguanosine, 3'-processing such as cleavage and polyadenylation, and splicing. Nucleic acids may also include synthetic nucleic acids (XNA), such as hexitol nucleic acid (HNA), cyclohexene nucleic acid (CeNA), threose nucleic acid (TNA), glycerol nucleic acid (GNA), locked nucleic acid (LNA) and peptide nucleic acid (PNA). Sizes of nucleic acids, also referred to herein as "polynucleotides" are typically expressed as the number of base pairs (bp) for double stranded polynucleotides, or in the case of single stranded polynucleotides as the number of nucleotides (nt). One thousand bp or nt equal a kilobase (kb). Polynucleotides of less than around 40 nucleotides in length are typically called "oligonucleotides" and may comprise primers for use in manipulation of DNA such as via polymerase chain reaction (PCR).

The term "amino acid" in the context of the present disclosure is used in its broadest sense and is meant to include organic compounds containing amine (NH₂) and carboxyl (COOH) functional groups, along with a side chain (e.g., a R group) specific to each amino acid. In some embodiments, the amino acids refer to naturally occurring L α-amino acids or residues. The commonly used one and three letter abbreviations for naturally occurring amino acids are used herein: A=Ala; C=Cys; D=Asp; E=Glu; F=Phe; G=Gly; H=His; I=Ile; K=Lys; L=Leu; M=Met; N=Asn; P=Pro; Q=Gln; R=Arg; S=Ser; T=Thr; V=Val; W=Trp; and Y=Tyr (Lehninger, A. L., (1975) Biochemistry, 2d ed., pp. 71-92, Worth Publishers, New York). The general term "amino acid" further includes D-amino acids, retro-inverso amino acids as well as chemically modified amino acids such as amino acid analogues, naturally occurring amino acids that are not usually incorporated into proteins such as norleucine, and chemically synthesised compounds having properties known in the art to be characteristic of an amino acid, such as β-amino acids. For example, analogues or mimetics of phenylalanine or proline, which allow the same conformational restriction of the peptide compounds as do natural Phe or Pro, are included within the definition of amino acid. Such analogues and mimetics are referred to herein as "functional equivalents" of the respective amino acid. Other examples of amino acids are listed by Roberts and Vellaccio, The Peptides: Analysis, Synthesis, Biology, Gross and Meiehofer, eds., Vol. 5 p. 341, Academic Press, Inc., N.Y. 1983.

The terms "polypeptide", and "peptide" are interchangeably used herein to refer to a polymer of amino acid residues and to variants and synthetic analogues of the same. Thus, these terms apply to amino acid polymers in which one or more amino acid residues is a synthetic non-naturally occurring amino acid, such as a chemical analogue of a corresponding naturally occurring amino acid, as well as to naturally-occurring amino acid polymers. Polypeptides can also undergo maturation or post-translational modification processes that may include, but are not limited to: glycosylation, proteolytic cleavage, lipidization, signal peptide cleavage, propeptide cleavage, phosphorylation, and such like. A peptide can be made using recombinant techniques, e.g., through the expression of a recombinant or synthetic polynucleotide. A recombinantly produced peptide it typically substantially free of culture medium, e.g., culture medium represents less than about 20 %, more preferably less than about 10 %, and most preferably less than about 5 % of the volume of the protein preparation.

The term "protein" is used to describe a folded polypeptide having a secondary or tertiary structure. The protein may be composed of a single polypeptide, or may comprise multiple polypepties that are assembled to form a multimer. The multimer may be a homooligomer, or a heterooligmer. The protein may be a naturally occurring, or wild type protein, or a modified, or non-naturally, occurring protein. The protein may, for example, differ from a wild type protein by the addition, substitution or deletion of one or more amino acids.

A "variant" of a protein encompass peptides, oligopeptides, polypeptides, proteins and enzymes having amino acid substitutions, deletions and/or insertions relative to the unmodified or wild-type protein in question and having similar biological and functional activity as the unmodified protein from which they are derived. The term "amino acid identity" as used herein refers to the extent that sequences are identical on an amino acid-by-amino acid basis over a window of comparison. Thus, a "percentage of sequence identity" is calculated by comparing two optimally aligned sequences over the window of comparison, determining the number of positions at which the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity.

For all aspects and embodiments of the present invention, a "variant" has at least 50%, 60%, 70%, 80%, 90%, 95% or 99% complete sequence identity to the amino acid sequence of the corresponding wild-type protein. Sequence identity can also be to a fragment or portion of the full length polynucleotide or polypeptide. Hence, a sequence may have only 50 % overall sequence identity with a full length reference sequence, but a sequence of a particular region, domain or subunit could share 80 %, 90 %, or as much as 99 % sequence identity with the reference sequence.

The term "wild-type" refers to a gene or gene product isolated from a naturally occurring source. A wild-type gene is that which is most frequently observed in a population and is thus arbitrarily designed the "normal" or "wild-type" form of the gene. In contrast, the term "modified", "mutant" or "variant" refers to a gene or gene product that displays modifications in sequence (e.g., substitutions, truncations, or insertions), post-translational modifications and/or functional properties (e.g., altered characteristics) when compared to the wild-type gene or gene product. It is noted that naturally occurring mutants can be isolated; these are identified by the fact that they have altered characteristics when compared to the wild-type gene or gene product. Methods for introducing or substituting naturally-occurring amino acids are well known in the art. For instance, methionine (M) may be substituted with arginine (R) by replacing the codon for methionine (ATG) with a codon for arginine (CGT) at the relevant position in a polynucleotide encoding the mutant monomer. Methods for introducing or substituting non-naturally-occurring amino acids are also well known in the art. For instance, non-naturally-occurring amino acids may be introduced by including synthetic aminoacyl-tRNAs in the IVTT system used to express the mutant monomer. Alternatively, they may be introduced by expressing the mutant monomer in *E. coli* that are auxotrophic for specific amino acids in the presence of synthetic (i.e. non-naturally-occurring) analogues of those specific amino acids. They may also be produced by naked ligation if the mutant monomer is produced using partial peptide synthesis. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art and may be selected in accordance with the properties of the 20 main amino acids as defined in Table 1 below. Where amino acids have similar polarity, this can also be determined by reference to the hydropathy scale for amino acid side chains in Table 2.

**Table 1 - Chemical properties of amino acids**

| | | | |
|---|---|---|---|
| Ala | aliphatic, hydrophobic, neutral | Met | hydrophobic, neutral |
| Cys | polar, hydrophobic, neutral | Asn | polar, hydrophilic, neutral |
| Asp | polar, hydrophilic, charged (-) | Pro | hydrophobic, neutral |
| Glu | polar, hydrophilic, charged (-) | Gln | polar, hydrophilic, neutral |
| Phe | aromatic, hydrophobic, neutral | Arg | polar, hydrophilic, charged (+) |
| Gly | aliphatic, neutral | Ser | polar, hydrophilic, neutral |
| His | aromatic, polar, hydrophilic, charged (+) | Thr | polar, hydrophilic, neutral |
| Ile | aliphatic, hydrophobic, neutral | Val | aliphatic, hydrophobic, neutral |
| Lys | polar, hydrophilic, charged(+) | Trp | aromatic, hydrophobic, neutral |
| Leu | aliphatic, hydrophobic, neutral | Tyr | aromatic, polar, hydrophobic |

**Table 2 - Hydropathy scale**

| Side Chain | Hydropathy |
|---|---|
| Ile | 4.5 |
| Val | 4.2 |
| Leu | 3.8 |
| Phe | 2.8 |
| Cys | 2.5 |
| Met | 1.9 |
| Ala | 1.8 |
| Gly | -0.4 |
| Thr | -0.7 |
| Ser | -0.8 |
| Trp | -0.9 |
| Tyr | -1.3 |
| Pro | -1.6 |
| His | -3.2 |
| Glu | -3.5 |
| Gln | -3.5 |
| Asp | -3.5 |
| Asn | -3.5 |
| Lys | -3.9 |
| Arg | -4.5 |

A mutant or modified protein, monomer or peptide can also be chemically modified in any way and at any site. A mutant or modified monomer or peptide is preferably chemically modified by attachment of a molecule to one or more cysteines (cysteine linkage), attachment of a molecule to one or more lysines, attachment of a molecule to one or more non-natural amino acids, enzyme modification of an epitope or modification of a terminus. Suitable methods for carrying out such modifications are well-known in the art. The mutant of modified protein, monomer or peptide may be chemically modified by the attachment of any molecule. For instance, the mutant of modified protein, monomer or peptide may be chemically modified by attachment of a dye or a fluorophore.

### Selectively characterizing polynucleotides having desired properties, e.g. polynucleotides of a desired length

The disclosure relates to a method of characterising a polynucleotide in a sample. Exemplary embodiments of the disclosure relate to characterising a polynucleotide or polynucleotides of a desired length. Thus, in some aspects the disclosure relates to a method for selectively characterizing polynucleotides of a desired length. However, length is only one property that can be assessed in accordance with the provided methods. This is discussed in more detail below.As discussed above, it is known that the selective characterization of polynucleotides can be achieved by comparing the "test sequence" of the initial portion of a candidate polynucleotide with a reference sequence. The similarity or lack thereof between the test sequence and the reference sequence provides a reference value which can be used to determine whether or not to continue to characterize the remainder of the candidate polynucleotide. The reference value is compared to a predetermined threshold value. If the reference signal corresponds to desired polynucleotides, then for the candidate polynucleotide to be retained then it is typically desirable that the reference value exceeds the threshold value defining similarity between the reference sequence and the test sequence. Thus, if the reference value does not exceed the threshold value then the candidate polynucleotide can be rejected and another candidate polynucleotide can be assessed. On the other hand, if the reference signal corresponds to unwanted polynucleotides (e.g. to known contaminants in the sample) then for the candidate polynucleotide to be retained it is typically desirable that the reference value does not exceed the threshold value. If it does exceed the threshold value then the candidate polynucleotide rejected.

As explained above, such methods do allow for unproductive characterization of unwanted polynucleotides to be reduced, both avoiding the unnecessary consumption of reagents and speeding up the overall characterization process. The overall characterization process is sped up at least because time required to characterize unwanted analytes is not unproductively used in the characterization.

However, the methods known in the art rely on comparing the sequence of portion of a candidate polynucleotide with a reference sequence. Determining the sequence of the candidate polynucleotide is not necessarily straight forward and is unnecessary if the candidate polynucleotide is not the desired polynucleotide. Furthermore, a reference sequence may not be available. Also, the decision whether or not to reject the candidate polynucleotide is based on a characterisation of only a part of the polynucleotide. If the candidate polynucleotide differs from the desired polynucleotide outside the part of the polynucleotide that is initially sequenced for comparison with the reference sequence, then the polynucleotide will not necessarily be rejected and thus may be unproductively characterised.

Whilst the sequence of a candidate polynucleotide can be used to determine whether the candidate polynucleotide is the desired polynucleotide, other parameters can be preferentially used which can be assessed e.g. more easily or quickly.

Any suitable properties can be assessed in accordance with the methods provided herein. Thus, as discussed in more detail herein, in some embodiments the approximate length of the polynucleotide (or of a first part of the polynucleotide) can be assessed. In some embodiments the structure of the polynucleotide (or of a first part of the polynucleotide) can be assessed. In some embodiments the composition of the polynucleotide (or of a first part of the polynucleotide) can be assessed.

One suitable parameter for assessing in order to determine whether a candidate polynucleotide in a sample of polynucleotides is a desired polynucleotide or not is the length of the polynucleotide (or of a first part of the polynucleotide). For example, in a sample of polynucleotides comprising desired long polynucleotides and unwanted short polynucleotides, an initial assessment of the length of a candidate polynucleotide (or a first part thereof) will allow unwanted short polynucleotides to be rejected and only long polynucleotides of the correct length to be characterised in more detail.

The size selection of polynucleotides in a sample of polynucleotides has been described via various means in the art. For example, gel filtration has been used to assess the size of polynucleotides in a sample. However, such techniques do not operate at the single molecule level and thus provide only bulk information about a plurality of polynucleotides in a sample. Furthermore, techniques such as gel filtration are typically slow, may modify or damage the polynucleotides being assessed, and have poor resolution. They are also not amenable to being miniaturised for use in real time during characterisation of polynucleotides in a sample by detectors such as nanopores.

The inventors have recognised that the time polynucleotide takes to move freely with respect to a nanopore (e.g. to translocate freely through or across a nanopore) provides information about the length of the polynucleotide. The inventors have found that by determining the time taken for a first part of a candidate polynucleotide to move in such manner, e.g. to translocate through or across the nanopore, the approximate length of the polynucleotide can be assessed and this assessment used as a metric to determine whether or not the polynucleotide should be rejected or characterised in more detail.

As explained above, another suitable property for assessing in order to determine whether a candidate polynucleotide in a sample of polynucleotides is a desired polynucleotide or not is the structure of the polynucleotide (or of a first part of the polynucleotide). For example, in a sample of polynucleotides comprising polynucleotides having desired polynucleotides which possess particular secondary or tertiary structural motifs and unwanted polynucleotides which do not possess such structural motifs, an initial assessment of the movement of the polynucleotide (or a first part thereof) with respect to the nanopore will allow unwanted polynucleotides to be rejected and only those polynucleotides having the relevant structural motifs to be characterised in more detail.

The detection of structural motifs in polynucleotides has been described via various means in the art. For example, circular dichroism has been used to assess the secondary structure of polynucleotides in a sample. However, such techniques do not operate at the single molecule level and thus provide only bulk information about a plurality of polynucleotides in a sample. Furthermore, techniques such as circular dichroism are typically slow and have poor resolution. They are also not amenable to being miniaturised for use in real time during characterisation of polynucleotides in a sample by detectors such as nanopores.

The inventors have recognised that the signal obtained as a polynucleotide moves freely with respect to a nanopore may provide information about the structure of the polynucleotide. For example, the baseline current level or noise characteristics recorded as a first part of a candidate polynucleotide moves with respect to a nanopore, and/or the time taken to do so, allows at least an initial assessment of the structure of the polynucleotide to be made and this assessment used as a metric to determine whether or not the polynucleotide should be rejected or characterised in more detail.

Similarly, another suitable property for assessing in order to determine whether a candidate polynucleotide in a sample of polynucleotides is a desired polynucleotide or not is the composition of the polynucleotide (or of a first part of the polynucleotide). For example, in a sample of polynucleotides comprising polynucleotides having desired compositions (e.g. percentage GC content in a strand, bulk purine vs pyrimidine composition, or RNA vs DNA) and unwanted polynucleotides which do not have such compositions, an initial assessment of the movement of the polynucleotide (or a first part thereof) with respect to the nanopore will allow unwanted polynucleotides to be rejected and only those polynucleotides having the relevant compositions to be characterised in more detail. Similarly, the identity of a known polynucleotide e.g. an amplicon from a panel may be detected such that the known polynucleotide can be recognised and characterised.

The detection of polynucleotide compositions has been described via various means in the art. One primary method used is polynucleotide sequencing, either by conventional means (e.g. Sanger sequencing) or by later generation single molecule sequencing, which allows a detailed picture of the polynucleotide composition to be determined. However, as discussed herein, conventional sequencing techniques do not operate at the single molecule level and thus provide only bulk information about a plurality of polynucleotides in a sample, whilst single molecule sequencing is relatively slow.

The inventors have recognised that the signal obtained as a polynucleotide moves freely with respect to a nanopore may provide information about the composition of the polynucleotide. For example, the baseline current level recorded as a first part of a candidate polynucleotide moves with respect to a nanopore, may allow at least an initial assessment of the composition of the polynucleotide to be made, even though such movement may be too fast for base-by-base sequencing. Such assessment can be used as a metric to determine whether or not the polynucleotide should be rejected or characterised in more detail.

In developing the claimed methods, it has been found that it is typically beneficial to allow the first part of a candidate polynucleotide to move freely with respect to the nanopore (e.g. to translocate freely through or across the nanopore) under an applied force. This is described in more detail below.

### Free translocation under an applied force

The provided methods comprise assessing one or more properties of the polynucleotide as the polynucleotide moves freely with respect to the nanopore under an applied force. For example, in some embodiments the provided methods comprise determining the time taken for a first part of a polynucleotide in the sample to translocate freely through or across the nanopore under an applied force, in order to assess the approximate length of the polynucleotide. In some preferred embodiments the methods provided herein comprise translocating the polynucleotide through the nanopore. In some embodiments the polynucleotides in the sample are double-stranded.

For the presence or absence of desired properties of the polynucleotide to be determined whilst the polynucleotide moves freely with respect to the nanopore, the speed of such movement is preferably predictable. **In** order for the approximate length of the polynucleotide to be determined from the time taken for a first part of the polynucleotide to translocate through or across the nanopore, the speed of the translocation is preferably predictable. In order for the time spent on unproductive characterisation of unwanted polynucleotides to be reduced as far as possible, it is also desirable that the speed (e.g. of the translocation of the first part of the polynucleotide through or across the nanopore is rapid. The methods provided herein thus comprise freely moving the polynucleotide with respect to the nanopore, by translocating the polynucleotide in the sample through or across the nanopore under an applied force.

Typically, if the time taken for the first portion of a polynucleotide to move with respect to the nanopore (e.g, to translocate through or across the nanopore) is below a threshold time then the length of the polynucleotide is assessed as being too low. Similarly, if the time taken for the first portion of a polynucleotide to move with respect to the nanopore (e.g. to translocate through or across the nanopore) is above a threshold time then the length of the polynucleotide is assessed as being too high. **In** such circumstances the polynucleotide may be rejected in order to avoid unproductively characterising the polynucleotide. As those skilled in the art will appreciate, if the speed at which the polynucleotide moves with respect to the nanopore (e.g. translocates through or across the nanopore) is approximately constant during the free movement, then the speed is approximately proportional to the length of the portion of the polynucleotide freely moving. Accordingly, some embodiments of the methods provided herein comprise comparing the time taken for the first portion of the polynucleotide to move with respect to the nanopore (e.g. to translocate through or across the nanopore) with a threshold time. In some embodiments the threshold time is predetermined. In some embodiments the threshold time is calculated based on the predicted length of the desired polynucleotides to be characterised. In some embodiments the threshold time is calculated based on the known or predicted length of contaminant polynucleotides which may be present in the sample. Accordingly, some embodiments of the methods provided herein comprise rejecting the polynucleotide (e.g. ejecting the polynucleotide from the nanopore) if the time taken for the first portion of the polynucleotide to move with respect to the nanopore (e.g. to translocate through or across the nanopore) is above a threshold time and/or is below a threshold time. Some embodiments of the methods provided herein comprise rejecting the polynucleotide (e.g. ejecting the polynucleotide from the nanopore) if the time taken for the first portion of the polynucleotide to move with respect to the nanopore (e.g. to translocate through or across the nanopore) is above a first threshold time and/or is below a second threshold time, wherein typically the first threshold time is greater than the second threshold time. Some embodiments thus comprise rejecting the polynucleotide (e.g. ejecting the polynucleotide from the nanopore) if the time taken for the first portion of the polynucleotide to move with respect to the nanopore (e.g. to translocate through or across the nanopore) is not between a first threshold time and a second threshold time - in other words, some embodiments comprise retaining the polynucleotide if the time taken for the first portion of the polynucleotide to move with respect to the nanopore (e.g. to translocate through or across the nanopore) is between a first threshold time and a second threshold time.

In a similar manner, other parameters apart from time may be recorded. These may include for example the aggregate current level or noise level recorded as the first portion of a polynucleotide moves with respect to the nanopore . If such levels recorded are above or below a threshold value then the structure or composition of the polynucleotide may be assessed as being incorrect, i.e. not being consistent with properties of the desired polynucleotide. In such circumstances the polynucleotide may be rejected in order to avoid unproductively characterising the polynucleotide. As those skilled in the art will appreciate, during the ultrafast free movement of the polynucleotide with respect to the nanopore (described in more detail herein) detailed noise and current information may not, in some embodiments, be obtainable; however a crude measurement of noise or current information may still be sufficient to assess a candidate polynucleotide as being correct or incorrect. In some embodiments the threshold value is predetermined. In some embodiments the threshold value is calculated based on the predicted properties of the desired polynucleotides to be characterised. In some embodiments the threshold value is calculated based on the known or predicted properties of contaminant polynucleotides which may be present in the sample. Accordingly, some embodiments of the methods provided herein comprise rejecting the polynucleotide if the current level(s) and/or noise level(s) recorded whilst the first portion of the polynucleotide moves with respect to the nanopore are above a threshold level and/or below a threshold level. Some embodiments of the methods provided herein comprise rejecting the polynucleotide if the current level(s) and/or noise level(s) recorded whilst the first portion of the polynucleotide moves with respect to the nanopore are above a first threshold level and/or below a second threshold level, wherein typically the first threshold level is greater than the second threshold level. Some embodiments thus comprise rejecting the polynucleotide if the current level(s) and/or noise level(s) recorded whilst the first portion of the polynucleotide moves with respect to the nanopore are not between a first threshold level and a second threshold level.

In some embodiments multiple threshold values can be used to define multiple windows which determine the rejection or retention of the polynucleotide. For example, in some embodiments first, second and third threshold times, current levels and/or noise levels are used. For example, the polynucleotide may be rejected (e.g. by being ejected from the nanopore) if the time taken for the first portion of the polynucleotide to move with respect to the nanopore (e.g. to translocate through or across the nanopore) is not below a first threshold time, or between a second threshold time and a third threshold time. The polynucleotide may be rejected (e.g. by being ejected from the nanopore) if the time taken for the first portion of the polynucleotide to move with respect to the nanopore (e.g. to translocate through or across the nanopore) is not above a first threshold time, or between a second threshold time and a third threshold time. Current and noise threshold levels can be used similarly.

In some embodiments first, second, third and fourth threshold times, current levels and/or noise levels are used. For example, the polynucleotide may be rejected (e.g. by being ejected from the nanopore) if the time taken for the first portion of the polynucleotide to move with respect to the nanopore (e.g. to translocate through or across the nanopore) is not between the first and second threshold times, or between the third and fourth threshold times. Current and noise threshold levels can be used similarly.

For example, the methods can be used to selectively characterise two populations of nucleotides, e.g. wherein one population has a first length (and thus freely moves with respect to the nanopore, e.g. translocates the nanopore between a first and second threshold time) and the second population has a second length (and thus freely moves with respect to the nanopore, e.g. translocates the nanopore between a third and fourth threshold time). The two populations may be of substantially the same length but differ in some other respect such as in terms of their secondary structure or composition, which may impact the time taken for the free translocation. For example, one population may have a first structure or composition (and thus freely move with respect to the nanopore , e.g. translocate the nanopore between a first and second threshold time) and the second population may have a second structure or composition (and thus freely move with respect to the nanopore , e.g. translocate the nanopore between a third and fourth threshold time). One population may have a first structure or composition (and thus freely move with respect to the nanopore and give rise to a signal having current and/or noise levels between a first and second threshold time) and the second population may have a second structure or composition (and thus freely move with respect to the nanopore and give rise to a signal having current and/or noise levels between a third and fourth threshold time).

Those skilled in the art will appreciate that further threshold values can be used. Any suitable number of threshold values can be used to selectively characterise desired polynucleotides. For example, the number of threshold values can be between 1 and 20, such as between 1 and 10, e.g. between 1 and 6, such as 1, 2, 3, 4 or 5.

Measurement of the time taken for the first portion of the polynucleotide to translocate through or across the nanopore is routine for those skilled in the art.

The binding of a molecule (e.g. a target polynucleotide) in the channel of the pore will have an effect on the open-channel ion flow through the pore, which is the essence of "molecular sensing" of pore channels. Variation in the open-channel ion flow can be measured using suitable measurement techniques by the change in electrical current (for example, WO 2000/28312 and D. Stoddart et al., Proc. Natl. Acad. Sci., 2010, 106, 7702-7 or WO 2009/077734). The degree of reduction in ion flow, as measured by the reduction in electrical current, is related to the size of the obstruction within, or in the vicinity of, the pore. Binding of a molecule of interest (e.g. the target polynucleotide) in or near the pore therefore provides a detectable and measurable event, thereby forming the basis of a "biological sensor". The event associated with binding of the polynucleotide is detectable and may provide a suitable start signal as described herein. For example, in some embodiments, the time taken for the first portion of a polynucleotide to translocate through or across the nanopore is measured from a start signal. A start signal may be for example the partial blocking of an open nanopore by capture of the first portion of the polynucleotide, which may for example be identified as an ionic current reading which changes from the open pore current to a polynucleotide-capture current. As explained herein, the polynucleotide-capture current may have a characteristic current and/or noise signature.

Any suitable start signal can be used in the methods discussed herein. A suitable start signal can be provided by a change in an ionic current or optical reading when the first part of the region to be measured beings to translocate through or across the nanopore. For example, translocation of an adapter attached to the polynucleotide to be selectively characterised may provide a start signal for determining the length of the subsequent first part of the polynucleotide. The start signal can be provided as a characteristic of the ionic current reading or optical reading, such as a change in noise level or magnitude of the signal.

In some embodiments, the time taken for the first portion of a polynucleotide to translocate through or across the nanopore is measured until a stop signal is reached. A stop signal may be for example a return to the open pore ionic current level, or may be a characteristic reading (e.g. a current reading, noise level, etc) associated with a feature in the polynucleotide such as a label or a spacer. Such features are described in more detail below.

In some embodiments a stop signal is provided by a feature on the polynucleotide. In some embodiments the stop signal is provided by a secondary or tertiary structure in the polynucleotide, such as a hairpin loop or G-quadruplex. In some embodiments the stop signal is provided by a second polynucleotide hybridised to the target polynucleotide. In some embodiments the stop signal is provided by an oligonucleotide hybridised to the target polynucleotide. In such embodiments contacting the oligonucleotide with the nanopore causes a characteristic reading as the oligonucleotide is removed from the target polynucleotide by the nanopore. The characteristic reading can comprise a stop signal as used herein.

In other embodiments a stop signal can be provided by a label on the polynucleotide such as a chemical group. Suitable chemical groups include fluorophores, streptavidin and/or biotin, cholesterol, methylene blue, dinitrophenols (DNPs), digoxigenin and/or anti-digoxigenin and dibenzylcyclooctyne groups, and the like. Other suitable labels are discussed in the context of spacers herein. The contacting of the label with the nanopore generates a characteristic reading as the label is removed from the polynucleotide or passes through or across the nanopore. Such a characteristic reading may comprise a stop signal.

In some embodiments, freely moving the first part of the polynucleotide with respect to the nanopore (e.g. by translocating the first part of the polynucleotide in the sample through or across the nanopore) comprises applying a physical or chemical force to the polynucleotide. In some embodiments the physical force is provided by an electrical (e.g. voltage) potential or a temperature gradient, etc, or is provided by a polynucleotide-handling enzyme. In some embodiments the chemical force is provided by a concentration (e.g. pH) gradient.

In some embodiments, freely moving the first part of the polynucleotide with respect to the nanopore (e.g. freely translocating the first part of the polynucleotide in the sample through or across the nanopore) comprises applying a potential across the nanopore and allowing the first part of the polynucleotide to move with respect to the nanopore (e.g. to translocate through or across the nanopore) under the influence of the applied potential. For example, polynucleotides are negatively charged molecules and so applying a voltage potential across a nanopore, e.g. a nanopore will cause the polynucleotides to move with respect to the nanopore under the influence of the applied voltage potential. For example, if a positive voltage potential is applied to the *trans* side of the nanopore relative to the *cis* side of the nanopore, then this will induce a negatively charged polynucleotide to move from the *cis* side of the nanopore to the *trans* side of the nanopore. Similarly, if a positive voltage potential is applied to the *trans* side of the nanopore relative to the *cis* side of the nanopore then this will impede the movement of a negatively charged polynucleotide from the *trans* side of the nanopore to the *cis* side of the nanopore. The opposite will occur if a negative voltage potential is applied to the *trans* side of the nanopore relative to the *cis* side of the nanopore. Apparatuses and methods of applying appropriate voltages are described in more detail herein.

In the absence of a competing force, a polynucleotide will freely translocate under the influence of an applied voltage potential. The rate of the translocation is typically proportional to the applied potential. Typically, the higher the potential the faster the rate of translocation. In some embodiments, the applied voltage potential is in the range of from about -2 V to about +2 V (suitable voltages are described in more detail below) and the speed of the voltage-driven free translocation of the polynucleotide through the nanopore is at least 1000 b/s (1 ms/base), more often at least 10kb/s (100 µs/base). Often the speed is in the range of from about 0.1 to about 10 µs/base (i.e. from about 0.1 msec /kilobase [ms/kb] to about 10 ms/kb; or in other words from about 0.1 sec/megabase [s/Mb] to about 10 s/Mb). Typically the speed of the voltage-driven free translocation of the polynucleotide through the nanopore is in the range of from about 0.5 to about 5 µs/base (i.e. from about 0.5 ms/kb to about 5 ms/kb; from about 0.5 s/Mb to about 5 s/Mb). More typically the speed of the voltage-driven free translocation of the polynucleotide through the nanopore is about 1 µs/base (i.e. about 1 ms/kb; about 1 s/Mb). Thus, by determining the time for a first portion of a polynucleotide to move with respect to the a nanopore (e.g. to translocate through or across a nanopore) under the influence of an applied voltage potential, the approximate length of the polynucleotide can be assessed.

In some embodiments, it is advantageous to control the speed of the movement of first part of the polynucleotide with respect to the nanopore (e.g. to control the speed of the translocation of the first part of the polynucleotide through or across the nanopore) rather than to solely rely on controlling an applied potential. For example, in some embodiments it is beneficial to control the speed of the translocation of the first part of the polynucleotide with respect to the nanopore (e.g. through or across the nanopore) using an ultra-fast polynucleotide-handling enzyme. In other embodiments the speed of the translocation of the first part of the polynucleotide with respect to the nanopore (e.g. through or across the nanopore) is controlled using a slider or brake. A slider may comprise a polynucleotide-handling enzyme e.g. which is modified as described herein so that it does not dissociate from the polynucleotide strand on which it is located. A brake may include modifications to the polynucleotide strand to impede (e.g. slow down) the translocation of the polynucleotide strand through a nanopore. For example, a polynucleotide strand may have single-stranded binding proteins (SSBs) attached thereto. The SSBs provide a barrier to translocation as they need to be removed from the polynucleotide strand by the nanopore to enable the polynucleotide strand to move through the pore. Any suitable brakes or sliders known in the art can be used in the methods provided herein. As used here, controlling the speed of the translocation of the first part of the polynucleotide through or across the nanopore using an ultra-fast polynucleotide-handling enzyme, a slider and/or a brake is an example of freely translocating the first part of the polynucleotide in the sample through or across the nanopore.

The speed of the translocation of the first part of the polynucleotide through or across the nanopore can also be controlled by factors such as nanopore or solvent effects. Charges inside the nanopore (e.g. at the solvent-accessible surface of a channel through the nanopore) can be used to interact with the nucleotide and control the speed of translocation, e.g. by retarding the speed. Constrictions and other steric blocks within the channel of the nanopore can be similarly used. Solvent parameters and effects such as viscosity, electro-osmosis, sterics, charge, etc can all be chosen or controlled to control the speed of the free translocation.

Some prior art techniques have sought to increase sequencing accuracy by using a polynucleotide-handling enzyme to control the movement of a first part of a polynucleotide to be characterised through a pore and by using the same polynucleotide-handling enzyme or a different polynucleotide-handling enzyme to control the movement of a second part of a polynucleotide through the pore so that the sequence of the first part of the polynucleotide and the sequence of the second part of the polynucleotide (e.g. the sequence of the template strand and the complement strand of a double-stranded polynucleotide) can be determined, with the sequence information from both strands being combined to improve the sequencing accuracy. For example, WO 2013/014451 describes how two strands of a double-stranded polynucleotide can be linked via a hairpin loop so that translocation of the forward (template) strand is followed by translocation of the reverse (complement) strand, with the sequences of both the forward and reverse strands being determined and the obtained sequence information from the forward and reverse strands being combined in order to provide higher confidence observations than may be achieved from measurement of template strands only. Similarly, WO 2018/100370 describes how both strands of a double stranded polynucleotide can be sequentially translocated through a nanopore to provide sequence information without the need to covalently link two strands via a bridging moiety, with the sequences of both strands being determined and the obtained sequence information from the two strands being combined in order to provide higher confidence observations of the polynucleotide sequence. Those skilled in the art will appreciate that such techniques (e.g. those techniques described in WO 2013/014451 and WO 2018/100370) are very different to the techniques described herein, in which the first part of the polynucleotide is freely translocated through the nanopore at speeds typically too fast to permit sequencing information to be obtained. The methods described herein do not require the sequence of the first part of the polynucleotide to be determined - rather, a relevant observable is the time required for the first part of the polynucleotide to translocate through the pore in order to assess the approximate length of the polynucleotide.

For example, an ultra-fast polynucleotide-handling enzyme may control the movement of the polynucleotide with respect to the nanopore at a rate of several thousand bases per second, e.g. around 10 kb/s or faster, such as at least 5 kb/s, e.g. at least 8 kb/s, for example at least 10 kb/s, e.g. at least 15 kb/s, such as at least 20 kb/s. An ultra-fast polynucleotide-handling enzyme is typically unsuitable for controlling the movement of a polynucleotide whilst the polynucleotide is being characterised in detail, e.g. whilst the sequence of the polynucleotide is being determined, because the movement of the polynucleotide with respect to the nanopore is typically too fast (e.g. too fast relative to data acquisition limits e.g. as used to sequence a polynucleotide). However, an ultra-fast polynucleotide-handling enzyme can be used to control the translocation of a first part of the polynucleotide with respect to a nanopore (e.g. through or across the nanopore) in order to assess its length or other properties described herein as detailed characterisation is not required. In such embodiments it can be beneficial that the processing of the polynucleotide by the polynucleotide-handling enzyme is too fast for the detailed characterisation of the polynucleotide in order to minimise the time required to assess the length of the polynucleotide and thus to minimise any time lost in unproductive characterisation of unwanted polynucleotides. As such, in some embodiments the methods provided herein do not comprise determining the sequence of the first part of the polynucleotide as it moves with respect to the nanopore (e.g. through or across the nanopore).

Ultra-fast polynucleotide-handling enzymes suitable for controlling the movement of the polynucleotide with respect to the nanopore (e.g. for controlling the translocation of a first part of a polynucleotide through or across a nanopore) are known in the art. Suitable examples include translocases (e.g. DNA translocases) such as those in the FtsK and SpoIIIE families; packaging motors such as the phi29 packaging motor; and helicases and helicase-nucleases such as AddAB and RecBCD.

Ultrafast translocation is typically too fast for characteristics such as the sequence of the polynucleotide to be determined, whilst still allowing the approximate length of the polynucleotide, or other relevant properties such as its structure or composition, to be determined. The controlled translocation in step (iii) is slower than the ultrafast translocation in step (ii) and allows characteristics of the polynucleotide such as its sequence to be determined.

In some embodiments, the relationship between the time taken to translocate the first part of the polynucleotide strand through or across the nanopore and the length of the polynucleotide is approximately linear; i.e. is directly proportional to the force applied on the polynucleotide.

### Rejecting undesired polynucleotides, e.g. by ejecting undesired polynucleotides from a nanopore

As explained above, the provided methods comprise assessing one or more properties of the first part of the polynucleotide. When the first part of the polynucleotide has one or more desired properties, the polynucleotide is further characterised and when the first part of the polynucleotide does not have one or more desired properties, the provided methods comprise rejecting the polynucleotide. For example, the provided methods may comprise assessing the approximate length of the polynucleotide in order to selectively characterise polynucleotides of a desired length. When the polynucleotide is not of the desired length, the provided methods may comprise ejecting the polynucleotide from the nanopore. When the first part of the polynucleotide does not have one or more desired properties, step (iii) of the disclosed methods typically comprises repeating steps (i), (ii a) and (ii b) with further polynucleotides from the sample until a polynucleotide having a first part having one or more desired properties is identified.

If the polynucleotide does not have one or more desired properties, it can be rejected from the nanopore by any suitable means. For example, it may be ejected from the nanopore . Any suitable means for ejecting the polymer may be used and are readily available to those skilled in the art. For example, in some embodiments, ejection of the polymer from the nanopore comprises reversing the bias of an applied potential across the nanopore. For example, in some embodiments capture and/or characterisation of a polynucleotide may be conducted at a positive applied potential and ejecting the polynucleotide if the polynucleotide is not of the desired length may comprise applying a negative potential. In other embodiments capture and/or characterisation of a polynucleotide may be conducted at a negative applied potential and ejecting the polynucleotide if the polynucleotide is not of the desired length may comprise applying a positive potential.

In some embodiments when ejection of non-selected polynucleotides comprises applying a voltage potential, the magnitude of the voltage potential used to eject the polynucleotides is greater than that applied during the assessment of the length of the first part of the polynucleotide. In other embodiments when ejection of non-selected polynucleotides comprises applying a voltage potential, the magnitude of the voltage potential used to eject the polynucleotides is lower than that applied during the assessment of the length of the first part of the polynucleotide. In yet other embodiments when ejection of non-selected polynucleotides comprises applying a voltage potential, the magnitude of the voltage potential used to eject the polynucleotides is the same as that applied during the assessment of the length of the first part of the polynucleotide.

The capture and/or characterisation of a polynucleotide may be conducted without an applied potential and ejecting the polynucleotide if the polynucleotide is not of the desired length may comprise applying a positive or a negative potential depending on the configuration of the apparatus used to perform the methods. In some embodiments the translocation of the first part of the polynucleotide is controlled by a polynucleotide-handling enzyme and ejection of the polynucleotide is achieved by altering the conditions in which the enzyme operates e.g. by controlling the fuel available to the enzyme, altering the pH, temperature, etc.

### Controlling movement of second part of polynucleotides

When the assessment of the first part of the polynucleotide confirms that the polynucleotide has one or more desired properties, the disclosed methods comprise controlling the movement of a second part of the polynucleotide with respect to a nanopore. For example, when the assessment of the length of the polynucleotide confirms that the polynucleotide is of the desired length, the disclosed methods may comprise controlling the movement of a second part of the polynucleotide through or across the nanopore.

Any suitable method can be used to control the movement of a second part of the polynucleotide with respect to a nanopore, e.g. through or across a nanopore. In some embodiments, the movement of the polynucleotide (e.g. through or across the nanopore) is controlled by controlling the force applied to the polynucleotide. The application of force to the polynucleotide is discussed above.

The movement of a second part of the polynucleotide with respect to a nanopore, e.g. through or across a nanopore, is controlled using a polynucleotide binding protein capable of controlling the movement of a polynucleotide. The polynucleotide binding protein is capable of controlling the movement of the polynucleotide with respect to a nanopore, e.g. controlling the movement of the polynucleotide through the nanopore. A polynucleotide binding protein is also known as a motor protein. As described in more detail herein, in some embodiments the polynucleotide binding protein is present on an adaptor prior to performing the methods described herein. In some embodiments the polynucleotide binding protein is stalled on an adapter prior to performing the methods described herein. In some embodiments the polynucleotide binding protein is stalled on the polynucleotide analyte prior to performing the methods described herein.

Suitable polynucleotide binding proteins are described in more detail herein. For example, in some embodiments the polynucleotides in the sample are double stranded and a polynucleotide binding protein capable of controlling the movement of a polynucleotide (i.e. a motor protein) is bound to at least one end of at least one strand of the double stranded polynucleotides.

In some embodiments the first part of the polynucleotide is translocated through or across a nanopore. In some preferred embodiments the first part of the polynucleotide is translocated through the nanopore. In some embodiments the second part of the polynucleotide is translocated through or across a nanopore. In some preferred embodiments the second part of the polynucleotide is translocated through the nanopore. In some embodiments the first part and the second part of the polynucleotide are sequentially translocated through the nanopore.

As explained in more detail here, the first part of the polynucleotide and the second part of the polynucleotide are, in some embodiments, the same - i.e. a portion of the polynucleotide is freely translocated through or across a nanopore (comprised in the first part of the polynucleotide) and the same portion is characterised e.g. sequenced (comprised in the second part). Embodiments in which the first and second parts of the polynucleotide are the same are described in more detail herein. For example, in one such embodiment, a polynucleotide binding protein may be bound to one end of a polynucleotide strand, the first part of the polynucleotide may be between a start point and the polynucleotide binding protein (the start point may for example by an end of the strand or provided by an adapter attached to the strand); and the second part may be the same as the first part. In an exemplary mode of this embodiment, the free translocation of the first part of the polynucleotide is used to assess the length of the polynucleotide and then the polynucleotide binding protein is used to control the movement of the second part of the polynucleotide with respect to the nanopore in order to characterise the second part of the polynucleotide, and the second part is the same as the first part.

In other embodiments, the first part of the polynucleotide is not the same as the second part of the polynucleotide. In yet other embodiments the first part of the polynucleotide overlaps with the second part of the polynucleotide. For example, the first part of the polynucleotide may comprise a portion of the second part of the polynucleotide. For instance, for a long polynucleotide strand to be characterised, the first part may be a portion of that strand between a start point and an end point and the second part may be the whole or part of the strand comprising the first part and additional nucleotides of the polynucleotide strand.

### Adapters

As explained in more detail below, a polynucleotide adapter can be attached to a target polynucleotide in order to characterise the target polynucleotide. In some embodiments of the methods provided herein, an adapter is attached to one or both ends of the polynucleotides in the sample prior to step (i) (i.e. before the nanopore, e.g. the transmembrane nanopore is contacted with the sample of polynucleotides).

An adapter typically comprises a polynucleotide strand capable of being attached to the end of a target polynucleotide. The target polynucleotide is typically intended for characterisation in accordance with methods disclosed herein.

A polynucleotide adapter may be added to both ends of the target polynucleotide. Alternatively, different adapters may be added to the two ends of the target polynucleotide. An adapter may be added to just one end of the target polynucleotide. An adapter may be added to both strands of a double stranded polynucleotide. An adapter may be added to just one strand of a polynucleotide. Methods of adding adapters to polynucleotides are known in the art. Adapters may be attached to polynucleotides, for example, by ligation, by click chemistry, by tagmentation, by topoisomerisation or by any other suitable method.

In one embodiment, the or each adapter is synthetic or artificial. Typically, the or each adapter comprises a polymer as described herein. In some embodiments, the or each adapter comprises a spacer as described herein.

In some embodiments, the or each adapter comprises a polynucleotide. The or each polynucleotide adapter may comprise DNA, RNA, modified DNA (such as a basic DNA), PNA, LNA, BNA and/or PEG. Usually, the or each adapter comprises single stranded and/or double stranded DNA or RNA. The adapter may comprise the same type of polynucleotide as the polynucleotide strand to which it is attached. The adapter may comprise a different type of polynucleotide to the polynucleotide strand to which it is attached. In some embodiments the polynucleotide strand assessed and characterised in the methods described herein is a double stranded DNA strand and the adapter comprises DNA or RNA, e.g. double or single stranded DNA.

In some embodiments, an adapter may be a bridging moiety. A bridging moiety may be used to connect the two strands of a double-stranded polynucleotide. For example, in some embodiments a bridging moiety is used to connect the template strand of a double stranded polynucleotide to the complement strand of the double stranded polynucleotide.

A bridging moiety typically covalently links the two strands of the target polynucleotide. The bridging moiety can be anything that is capable of linking the two strands of the target polynucleotide, provided that the bridging moiety does not interfere with movement of the single stranded polynucleotide through the transmembrane pore. Suitable bridging moieties include, but are not limited to a polymeric linker, a chemical linker, a polynucleotide or a polypeptide. Preferably, the bridging moiety comprises DNA, RNA, modified DNA (such as abasic DNA), RNA, PNA, LNA or PEG. The bridging moiety is more preferably DNA or RNA.

In some embodiments a bridging moiety is a hairpin adapter. Thus, in some embodiments, the polynucleotide is a double-stranded polynucleotide comprising a first strand connected to a second strand by a hairpin or hairpin adapter. A hairpin adapter is an adapter comprising a single polynucleotide strand, wherein the ends of the polynucleotide strand are capable of hybridising to each other, or are hybridized to each other, and wherein the middle section of the polynucleotide forms a loop. Suitable hairpin adapters can be designed using methods known in the art. In some embodiments a hairpin loop is typically 4 to 100 nucleotides in length, e.g. from 4 to 50 such as from 4 to 20 e.g. from 4 to 8 nucleotides in length.

In some embodiments, the bridging moiety (e.g. hairpin adapter) is linked to the target polynucleotide by any suitable means known in the art. The bridging moiety (e.g. hairpin adapter) may be synthesized separately and chemically attached or enzymatically ligated to the target polynucleotide. Alternatively, the bridging moiety (e.g. hairpin adapter) may be generated in the processing of the target polynucleotide. In some embodiments, the bridging moiety (e.g. hairpin adapter) is linked to the target polynucleotide at or near one end of the target polynucleotide. In some embodiments, the bridging moiety (e.g. hairpin adapter) is linked to the target polynucleotide within 50, e.g. within 20 for example within 10 nucleotides of an end of the target polynucleotide. In some embodiments the bridging moiety (e.g. hairpin adapter) is linked to the target polynucleotide at a terminus of the target polynucleotide.

In some embodiments the bridging moiety (e.g. hairpin adapter) is attached at one end of the target polynucleotide. A bridging moiety (e.g. hairpin adapter) is typically not attached at both ends of the target polynucleotide. When a bridging moiety (e.g. hairpin adapter) is linked to the target polynucleotide the bridging moiety may comprise the same type of nucleotides as the target polynucleotide or may comprise different nucleotides to the target polynucleotide.

In some embodiments, an adapter may be a linear adapter. A linear adapter may be bound to either or both ends of a single stranded polynucleotide. When the polynucleotide is a double stranded polynucleotide, a linear adapter may be bound to either or both ends of either or both strands of the double stranded polynucleotide. A linear adapter may comprise a leader sequence as described herein. A linear adapter may comprise a portion for hybridisation with a tag (such as a pore tag) as described herein. A linear adapter may be 10 to 150 nucleotides in length, such as from 20 to 120, e.g. 30 to 100, for example 40 to 80 such as 50 to 70 nucleotides in length. A linear adapter may be single stranded. A linear adapter may be double stranded.

A linear adapter may be linked to the target polynucleotide by any suitable means known in the art. The linear adapter may be synthesized separately and chemically attached or enzymatically ligated to the target polynucleotide. Alternatively, the linear adapter may be generated in the processing of the target polynucleotide. In some embodiments, the linear adapter is linked to the target polynucleotide at or near one end of the target polynucleotide. In some embodiments, the linear adapter is linked to the target polynucleotide within 50, e.g. within 20 for example within 10 nucleotides of an end of the target polynucleotide. In some embodiments the linear adapter is linked to the target polynucleotide at a terminus of the target polynucleotide. When a linear adapter is linked to the target polynucleotide the linear adapter may comprise the same type of nucleotides as the target polynucleotide or may comprise different nucleotides to the target polynucleotide.

In some embodiments, an adapter may be a Y adapter. A Y adapter is typically a polynucleotide adapter. A Y adapter is typically double stranded and comprises (a) at one end, a region where the two strands are hybridised together and (b), at the other end, a region where the two strands are not complementary. The non-complementary parts of the strands typically form overhangs. The presence of a non-complementary region in the Y adapter gives the adapter its Y shape since the two strands typically do not hybridise to each other unlike the double stranded portion. The two single-stranded portions of the Y adapter may be the same length, or may be different lengths. For example, one single-stranded portion of the Y adapter may be 10 to 150 nucleotides in length, such as from 20 to 120, e.g. 30 to 100, for example 40 to 80 such as 50 to 70 nucleotides in length and the other single stranded portion of the Y adapter may independently by 10 to 150 nucleotides in length, such as from 20 to 120, e.g. 30 to 100, for example 40 to 80 such as 50 to 70 nucleotides in length. The double-stranded "stem" portion of the Y adapter may be e.g. from 10 to 150 nucleotides in length, such as from 20 to 120, e.g. 30 to 100, for example 40 to 80 such as 50 to 70 nucleotides in length.

In some embodiments a Y adapter is attached at each end of a double stranded polynucleotide. In some embodiments each Y adapter is the same. In other embodiments the Y adapter attached to one end of the double stranded polynucleotide is different from the Y adapter attached at the other end of the double stranded polynucleotide. In some embodiments a Y adapter is attached at one end of a double stranded polynucleotide but not at the other end (i.e. is attached at just one end of a double stranded polynucleotide).

In some embodiments, one of the non-complementary strands of a polynucleotide adapter such as a linear adapter or a Y adapter comprises a leader sequence, which when contacted with a transmembrane pore is capable of threading into a nanopore. The leader sequence typically comprises a polymer such as a polynucleotide, for instance DNA or RNA, a modified polynucleotide (such as abasic DNA), PNA, LNA, polyethylene glycol (PEG) or a polypeptide. In some embodiments, the leader sequence comprises a single strand of DNA, such as a poly dT section. The leader sequence can be any length, but is typically 10 to 150 nucleotides in length, such as from 20 to 120, e.g. 30 to 100, for example 40 to 80 such as 50 to 70 nucleotides in length.

In some embodiments, the Y adapter is linked to the target polynucleotide at or near one end of the target polynucleotide. In some embodiments, the Y adapter is linked to the target polynucleotide within 50, e.g. within 20 for example within 10 nucleotides of an end of the target polynucleotide. In some embodiments the Y adapter is linked to the target polynucleotide at a terminus of the target polynucleotide. When a Y adapter is linked to the target polynucleotide the Y adapter may comprise the same type of nucleotides as the target polynucleotide or may comprise different nucleotides to the target.

Those skilled in the art will also appreciate that when an adapter comprises a polynucleotide strand, the sequence of the adapter is typically not determinative and can be controlled or chosen according to experimental conditions such as the polynucleotide to be characterised. Exemplary sequences are provided solely by way of illustration in the examples. For example, the adapter may comprise a sequence such as one or more of SEQ ID NOs: 11, 12, 15 or 16 or a polynucleotide sequence having at least 20%, such as at least 30%, e.g. at least 40% such as at least 50%, e.g. at least 60% such as at least 70%, e.g. at least 80%, for example at least 90% e.g. at least 95% sequence similarity or identity to one or more of SEQ ID NOs: 11, 12, 15 or 16. The sequence of the adapter can typically be altered without negatively affecting the efficacy of the methods provided herein.

In one embodiment an adapter may comprise a membrane anchor or a pore anchor as described in more detail herein. In some embodiments, the anchor may be attached to a polynucleotide that is complementary to and hence that is hybridised to the overhang to which an nucleic acid handling enzyme is bound. An anchor such as a membrane anchor may be attached to the first part and/or to the second part of the polynucleotide.

In some embodiments, a polynucleotide binding protein (discussed below) may bind to or be bound to an adapter. A polynucleotide binding protein may be bound to or stalled at an adapter prior to step (i) of the disclosed methods. In some embodiments, a polynucleotide binding protein capable of controlling the movement of a polynucleotide is bound to the adapter attached to at least one end of the polynucleotide. For example, a polynucleotide binding protein may bind to an overhang of an adapter such as a Y adapter. In some embodiments, a polynucleotide binding protein may be bound to and/or stalled at a hairpin or hairpin adapter. In some embodiments a polynucleotide binding protein is bound to and/or stalled at the hairpin or hairpin adapter prior to step (i) of the disclosed methods.

In another embodiment, a polynucleotide binding protein may bind to the double stranded region. In other embodiments, a polynucleotide binding protein may bind to a single-stranded and/or a double-stranded region of the adapter. In other embodiments, a first polynucleotide binding protein may bind to the single-stranded region of such an adapter and a second polynucleotide binding protein may bind to the double-stranded region of the adapter.

In some embodiments, a polynucleotide adapter may comprise one or more spacers (as described herein). Spacers may be used for example to provide a characteristic signal when the portion of the adapter comprising the spacers is translocated through or across a nanopore and/or to position a polynucleotide binding protein on an adapter.

Typically, in the methods disclosed herein, the polynucleotides to be selectively characterised are double stranded polynucleotides.

In some embodiments a binding moiety such as a hairpin adapter is attached to one end of the double stranded polynucleotides. In some embodiments, a first part of the double stranded polynucleotide is a first strand. In some embodiments, the methods comprise freely translocating the first part (i.e. the first strand) of the double stranded polynucleotide through or across a nanopore and determining the time taken for the first part (i.e. the first strand) of the double stranded polynucleotide to translocate through or across the nanopore in order to assess the approximate length of the polynucleotide. In some embodiments the second part of the double stranded polynucleotide is the second strand and is attached to the first part (first strand) by the hairpin adapter. Once the first part (first strand) has been used to assess the approximate length of the polynucleotide and the hairpin adapter has been reached, then if the polynucleotide is of the desired length then the movement of the second part (second strand) of the polynucleotide though or across the nanopore is controlled so that the second part (second strand) of the polynucleotide can be characterised, e.g. can be sequenced. In other embodiments the second part is the same as the first part (e.g. the second part is the first strand) and is characterised after the approximate length of the first part has been assessed. In other embodiments, the composition or structure of the first part of the polynucleotide is assessed similarly, by monitoring the current and/or noise during the translocation of the first part of the polynucleotide.

The controlled movement of the second part (e.g. second strand) of the polynucleotide through or across the nanopore is controlled by a polynucleotide binding protein capable of controlling the movement of the polynucleotide with respect to the nanopore, as described in more detailed herein. In some embodiments the polynucleotide binding protein capable of controlling the movement of a polynucleotide is bound to the hairpin adapter. In some embodiments, the polynucleotide binding protein is bound to the hairpin adapter before the adapter is attached to the double stranded polynucleotides. In some embodiments the adapter is attached to the double stranded polynucleotides before the polynucleotide binding protein is bound to the adapter. In some embodiments the polynucleotide binding protein may be contacted with the polynucleotide and thereby bind to the adapter once the first part of the double stranded polynucleotide has passed through or across the nanopore.

In some embodiments, an adapter comprising a single stranded leader sequence is attached to the double stranded polynucleotides. In some embodiments, an adapter comprising a single stranded leader sequence is attached to one strand of the double stranded polynucleotide. In some embodiments, an adapter comprising a single stranded leader sequence is attached to the first part of the double stranded polynucleotide. In some embodiments an adapter comprising a single stranded leader sequence is attached to both strands of the double stranded polynucleotide. Typically in such embodiments the leader sequence attached to the first part (e.g. the first strand) of the double stranded polynucleotide does not hybridise to a leader sequence attached to the second part (e.g. the second strand) of the double stranded polynucleotide. In some embodiments, the leader sequence may be part of a Y adapter as described herein.

In some embodiments, an adapter comprising a single stranded leader sequence is attached to one end of a double stranded polynucleotide. In some embodiments, an adapter comprising a single stranded leader sequence is attached to each end of a double stranded polynucleotide. In some embodiments, an adapter is attached to only one strand of the double stranded polynucleotide. In some embodiments, an adapter is attached to each strand of a double stranded polynucleotide. For example, in some embodiments, a Y adapter may be attached to one or both ends of the double stranded polynucleotide.

In some embodiments a polynucleotide binding protein is bound to the adapter. In some embodiments the polynucleotide binding protein is bound to the same strand of the double stranded polynucleotide as the adapter. In some embodiments the polynucleotide binding protein is bound to the other strand of the polynucleotide as the adapter. In some embodiments a polynucleotide binding protein is bound to both strands of the polynucleotide. In some embodiments, the polynucleotide comprises a single stranded leader sequence at one end and has a polynucleotide binding protein bound thereto at the other end on the same strand of the polynucleotide.

In some embodiments the first strand of the double stranded polynucleotide having an adapter bound thereto is freely translocated through or across the nanopore in order to assess the approximate length of the polynucleotide. In some embodiments free translocation is stopped by the polynucleotide binding protein on the adapter. If the polynucleotide is of the desired length then the polynucleotide binding protein can control the movement of the polynucleotide through or across the nanopore whilst measurements are taken so that the polynucleotide can be characterised. In other embodiments, the composition or structure of the first part of the polynucleotide is assessed similarly, by monitoring the current and/or noise during the translocation of the first part of the polynucleotide.

In some embodiments, both strands of a double stranded polynucleotide are translocated through or across a nanopore without being covalently linked. In such embodiments, the first part of the polynucleotide is a first strand of the double stranded polynucleotide (e.g. the template strand) and the second part of the polynucleotide is a second strand of the polynucleotide (e.g. the complement strand). In some embodiments, the second part (e.g. the second strand) of the double stranded polynucleotide remains in the vicinity of the nanopore once the first part (e.g. the first strand) of the double stranded polynucleotide has been freely translocated through or across the nanopore. In some embodiments the second strand is preferentially captured by the nanopore e.g. due to an increased local concentration of the second strand in the vicinity of the nanopore. A tag can be used to retain the second part of the polynucleotide in the vicinity of the nanopore. Tags are described in more detail herein.

In some embodiments a portion of the second strand of the double stranded polynucleotide comprises a sequence complementary to a tag sequence. In some embodiments an oligonucleotide is hybridised to the second strand of the double stranded polynucleotide and the oligonucleotide comprises (a) a portion complementary to a portion of the second strand and (b) a portion complementary to a tag sequence. In some embodiments an oligonucleotide is hybridised to the second strand of the double stranded polynucleotide and the oligonucleotide comprises (a) a portion complementary to a portion of the second strand and (b) an affinity molecule capable of binding to a tag. In some embodiments, hybridisation of a portion of the second strand of the double stranded polynucleotide to a tag sequence comprises hybridisation of at least 50%, e.g. at least 60%, such as at least 70%, e.g. at least 80% e.g. at least 90% such as at least 95% or more of the nucleotide residues in the portion of the second strand to the tag sequence. A portion may be for example from 10 to 50 nucleotides, such as from 20 to 30 nucleotides in length.

In some embodiments the portion of the second strand which binds to and/or hybridises to the tag sequence is a portion of an adapter which is attached to the target polynucleotide.

In some embodiments, the oligonucleotide or the second strand binds to a tag or tag sequence attached to or in the vicinity of, e.g. adjacent to, the nanopore. In some embodiments, the oligonucleotide or the second strand is bound to a tag or tag sequence attached or in the vicinity of, e.g. adjacent to, to the nanopore. In some embodiments the second strand or a portion thereof hybridises to a tag sequence as the first strand moves through or across the nanopore.

In some embodiments, the first part of the polynucleotide is at least a portion of the first strand and the second part of the polynucleotide is at least a portion of the second strand. In some embodiments the first part of the polynucleotide is at least 10% by length of the first strand, e.g. at least 20%, such as at least 40%, e.g. at least 60%, e.g at least 80%, e.g. at least 90%, e.g. at least 95% or more such as at least 97%, at least 98%, or at least 99% by length of the first strand. In some embodiments the second part of the polynucleotide is at least 10% by length of the second strand, e.g. at least 20%, such as at least 40%, e.g. at least 60%, e.g at least 80%, e.g. at least 90%, e.g. at least 95% or more such as at least 97%, at least 98%, or at least 99% by length of the second strand.

In some embodiments, the method comprises pausing the first part of the double stranded polynucleotide once it has translocated wholly or partially through or across the nanopore. In some embodiments pausing the first part of the double stranded polynucleotide in this way promotes binding of the portion of the second part of the double stranded polynucleotide comprising a sequence complementary to a tag sequence to the tag sequence. In some embodiments movement of the first strand through the transmembrane nanopore is temporarily paused to allow hybridisation of the second strand to the tag sequence.

Any suitable method can be used to pause the translocation of the first part of the polynucleotide in order to promote the binding of the second part of the polynucleotide to the tag. In some embodiments, the applied force is temporarily paused or halted such that the translocation of the first part of the polynucleotide is paused. In some embodiments a pausing moiety is positioned on the first part of the polynucleotide. In some embodiments the pausing moiety is a polynucleotide handling enzyme. In some embodiments the pausing moiety is a chemical group. Suitable chemistry for the pausing moiety is similar to that for the tag moiety as discussed below. For example a weakly bound avidin moiety (e.g. strepatividin, neutravidin etc), a biotin group, a G-quadruplex or similar secondary structures such as thrombin binding aptamers, a BNA group etc may be used to impede translocation of the first part of the polynucleotide in order to promote the binding of the second part of the polynucleotide to the tag.

In some embodiments the pausing moiety is present within the polynucleotide sequence. In some embodiments the pausing moiety is located in a loop portion. A loop portion may be formed by a portion of the first strand of polynucleotide which does not hybridise to the second strand of polynucleotide. In some embodiments a loop portion may comprise a homo-oligomeric polynucleotide. A homo-oligomeric polynucleotide may comprise poly(T), poly(A), poly(C) or poly(G) residues. Poly(T) residues are typically used. A loop portion may comprise one or more non-nucleotide groups. A loop portion may comprise one or more spacer groups as defined herein. A loop portion may comprise one or more spacer groups selected from spacer 9 (iSp9) [(OCH₂CH₂)₃OPO₃] groups and spacer 18 (iSp18) [(OCH₂CH₂)₆OPO₃] groups as described in more detail herein. A loop portion may comprise both regions of non-hybridising polynucleotides and one or more spacer groups. **In** some embodiments a loop portion comprises one or more poly(T) portions and one or more spacer groups.

**In** some embodiments a loop portion comprising a pausing moiety is located on a first part of the polynucleotide adjacent to a loop portion comprising a polynucleotide handling enzyme located on the second part of the polynucleotide. **In** some embodiments a loop portion comprising a pausing moiety is located on a first part of the polynucleotide offset from a loop portion comprising a polynucleotide handling enzyme located on the second part of the polynucleotide.

The pausing moiety may be bound to the first part of the polynucleotide at the opposite end of the polynucleotide to the leader sequence if present on the first part of the polynucleotide. **In** other words, the first part of the polynucleotide may have a leader sequence at one end and a pausing moiety such as a polynucleotide handling enzyme at or near the opposite end. The pausing moiety such as a polynucleotide handling enzyme may impede the free translocation of the first part of the polynucleotide thereby pausing the free translocation of the first part of the polynucleotide, promoting binding of the second part of the polynucleotide to the tag.

In some embodiments the pausing moiety is provided on an adapter. In some embodiments the adapter is part of the same adapter comprising the polynucleotide handling enzyme on the second part of the polynucleotide. For example, a double stranded polynucleotide may be attached to an adapter comprising a pausing moiety on the first strand of the double stranded polynucleotide and an polynucleotide handling enzyme on the second strand of the polynucleotide. The adapter may comprise a portion comprising a sequence complementary to a tag sequence. In some embodiments the adapter is a Y adapter attached to both the first and second strands of the double stranded polynucleotide, so that the portion of the Y adapter attached to the second strand of the double stranded polynucleotide comprises a leader sequence.

In view of the above discussion, it will be apparent to those skilled in the art that the methods of the invention can be readily applied.

The first part of the polynucleotide is the same as the second part of the polynucleotide. The free translocation of the first part of the polynucleotide is in a first direction with respect to the nanopore. A polynucleotide binding protein typically moves the polynucleotide through or across the nanopore in a second direction opposite to the first direction. For example, if the polynucleotide is freely translocated from the *cis* to the *trans* side of a nanopore then the polynucleotide binding protein typically controls the movement of the polynucleotide from the *trans* side to the *cis* side of the nanopore whilst the polynucleotide is being characterised. Typically, therefore, in such embodiments the polynucleotide binding protein is orientated on the polynucleotide such that the polynucleotide binding protein controls the movement of the second part of the polynucleotide back through the nanopore against the applied force. Suitable polynucleotide binding proteins for use in this manner are disclosed in WO 2013/057495.

For example, a leader sequence may be attached to one end of the first strand of a double stranded polynucleotide and a polynucleotide binding protein may be attached to the other end of the first strand. In other words, in some embodiments the polynucleotide comprises a single stranded leader sequence at one end and has a polynucleotide binding protein bound thereto at the other end on the same strand of the polynucleotide. The free translocation of the first strand may be impeded by the polynucleotide binding protein. The polynucleotide binding protein then controls the movement of the first strand in the opposite direction to its free translocation whilst the polynucleotide is characterised.

In some embodiments the leader sequence is comprised in a linear adapter attached to one end of a first strand of a double stranded polynucleotide. In some embodiments the leader sequence is comprised in a Y adapter attached to one end of a first strand of a double stranded polynucleotide. In some embodiments the polynucleotide binding protein is attached to a linear adapter attached to the opposite end of the first strand of a double stranded polynucleotide. In some embodiments the polynucleotide binding protein is attached to a Y adapter attached to the opposite end of the first strand of a double stranded polynucleotide from the leader sequence. In some embodiments a Y adapter is attached to both ends of a double stranded polynucleotide with one strand of the Y adapter comprising a leader sequence and the other strand of the adapter having a polynucleotide binding protein bound thereto.

In some embodiments, therefore, a single stranded leader sequence is captured by the transmembrane nanopore under the applied force, the first part of the polynucleotide is the part between the leader polynucleotide and the polynucleotide binding protein, the polynucleotide binding protein is orientated on the polynucleotide such that the polynucleotide binding protein controls the movement of the second part of the polynucleotide back through the nanopore against the applied force, and the second part of the polynucleotide is the same as the first part of the polynucleotide. In some embodiments the polynucleotide binding protein (i.e. the motor protein) is bound directly to the polynucleotide. In some embodiments the polynucleotide binding protein (i.e. the motor protein) is bound to an adapter which is attached to the polynucleotide.

In some embodiments, the signal caused by the capture of the leader sequence on the first part of the polynucleotide is a start signal for determining the time taken for the first part of the polynucleotide to translocate through or across the nanopore. In some embodiments the signal caused by the portion of the polynucleotide on which the polynucleotide binding protein is bound (e.g. the adapter on which the polynucleotide binding protein is bound) generates a signal which is a stop signal for determining the time taken for the first part of the polynucleotide to translocate through or across the nanopore. In some embodiments the portion of the polynucleotide on which the polynucleotide binding protein is bound (e.g. the adapter on which the polynucleotide binding protein is bound) comprises a recognisable motif such as a barcode or label which generates a signal which is a stop signal for determining the time taken for the first part of the polynucleotide to translocate through or across the nanopore. Such labels can include hybridised oligonucleotides and/or chemical groups such as fluorophores, streptavidin and/or biotin, cholesterol, methylene blue, dinitrophenols (DNPs), digoxigenin and/or anti-digoxigenin and dibenzylcyclooctyne groups, etc, as described herein. In some embodiments the sequence of the portion of the polynucleotide on which the polynucleotide binding protein is bound (e.g. the adapter on which the polynucleotide binding protein is bound) generates a known signal which corresponds to the stop signal. In some embodiments the sequence of the portion of the polynucleotide on which the polynucleotide binding protein is bound generates a secondary structure in the polynucleotide e.g. a hairpin loop which generates a stop signal when contacted with the nanopore. In some embodiments the contacting of the polynucleotide binding protein with the nanopore generates a stop signal.

In some preferred embodiments, a hairpin adapter is attached to one end of the double stranded polynucleotide and an adapter comprising a single stranded leader sequence is attached to the other end of the double stranded polynucleotide. In some embodiments, a hairpin adapter is attached to one end of the double stranded polynucleotides and an adapter comprising a single stranded leader sequence is attached to the other end of the double stranded polynucleotides, and a polynucleotide binding protein capable of controlling the movement of a polynucleotide is bound to the hairpin adapter. The single stranded leader sequence permits the capture of the first part of the double stranded polynucleotide by the nanopore and under the influence of an applied force the first part of the double stranded polynucleotide is freely translocated through or across the nanopore so that the approximate length of the polynucleotide can be assessed. The movement of the second part of the double stranded polynucleotide through or across the pore is controlled by the polynucleotide binding protein on the hairpin adapter so that the second part of the double stranded polynucleotide can be characterised as it moves with respect to the nanopore; e.g. to permit its sequencing as it moves with respect to (e.g. through or across) the nanopore.

In some embodiments the leader sequence is comprised in a linear adapter attached to one end of a first strand of a double stranded polynucleotide. In some embodiments the leader sequence is comprised in a Y adapter attached to one end of a first strand of a double stranded polynucleotide. In some embodiments the polynucleotide binding protein is attached to the hairpin adapter. In some embodiments the polynucleotide binding protein is attached to the end of the first strand of the double stranded polynucleotide in the vicinity of the hairpin adapter, e.g. abutting the hairpin adapter. In some embodiments the polynucleotide binding protein is attached to the end of the second strand of the double stranded polynucleotide in the vicinity of the hairpin adapter, e.g. abutting the hairpin adapter.

In some embodiments, therefore, the single stranded leader sequence is captured by the transmembrane nanopore under the applied force, the first part of the polynucleotide is a first strand of the double stranded polynucleotide, the second part of the polynucleotide is the second strand of the double stranded polynucleotide and the polynucleotide binding protein controls the movement of the second part of the polynucleotide through the transmembrane nanopore. In some other embodiments, the single stranded leader sequence is captured by the transmembrane nanopore under the applied force, the first part of the polynucleotide is a first strand of the double stranded polynucleotide, the second part of the polynucleotide is the same as the first part of the polynucleotide, and the polynucleotide binding protein controls the movement of the second part of the polynucleotide back through the transmembrane nanopore against the applied force.

In some embodiments, the signal caused by the capture of the first part of the polynucleotide is a start signal for determining the time taken for the first part of the polynucleotide to translocate through or across the nanopore. In some embodiments the hairpin adapter comprises a recognisable motif such as a barcode or label which generates a signal which is a stop signal for determining the time taken for the first part of the polynucleotide to translocate through or across the nanopore. In some embodiments the sequence of the hairpin adapter generates a known signal which corresponds to the stop signal. In some embodiments the hairpin adapter is modified to generate a stop signal. In some embodiments the contacting of the polynucleotide binding protein with the nanopore generates a stop signal.

In some preferred embodiments a double stranded polynucleotide comprises a leader sequence at one end of a first part (e.g. a first strand) and a portion of a second part (e.g. the second strand of the double stranded polynucleotide) comprises a sequence complementary to a tag sequence. In some embodiments a polynucleotide handling enzyme is bound to the second part (e.g. the second strand) of the polynucleotide. In some embodiments no polynucleotide handling enzyme is bound to the first part. In some embodiments the free translocation of the first part (e.g. the first strand) of the polynucleotide through or across the nanopore causes the portion of a second part (e.g. the second strand of the double stranded polynucleotide) comprising a sequence complementary to a tag sequence to bind to the tag sequence. In some embodiments the tag sequence is located on or in the vicinity of (e.g. adjacent to) the nanopore. Thus, the second part (e.g. the second strand of the double stranded polynucleotide) is held in the vicinity of the pore. After the free translocation of the first part of the polynucleotide has allowed the approximate length of the polynucleotide to be assessed, if the length is a desired length then the polynucleotide handling enzyme controls the movement of the second part of the polynucleotide through or across the nanopore, thus allowing the second part of the polynucleotide to be characterised, e.g. sequenced.

In some embodiments the portion of the second part (e.g. the second strand of the double stranded polynucleotide) comprising a sequence complementary to a tag sequence is a part of the polynucleotide to be selectively characterised. In some embodiments the portion of the second part comprising a sequence complementary to a tag sequence is a portion of an adapter attached to the second part of the polynucleotide.

In some embodiments the portion of the second part (e.g. the second strand of the double stranded polynucleotide) comprising a sequence complementary to a tag sequence is attached to a leader sequence as defined herein. In some embodiments the binding of the tag sequence to a tag on or near the nanopore causes the leader sequence on the second polynucleotide to be captured by the nanopore.

In some embodiments, the polynucleotide is double stranded and comprises a single stranded leader sequence at one end of a first strand of the double stranded polynucleotide and has polynucleotide binding protein bound thereto at one end of the second strand of the double stranded polynucleotide. In some embodiments the single stranded leader sequence and the polynucleotide binding protein are positioned at the same end of the polynucleotide. In some embodiments the single stranded leader sequence and the polynucleotide binding protein are positioned at different ends of the polynucleotide.

In some embodiments the leader sequence of the adapter attached to the second strand of the polynucleotide does not hybridise to a single-stranded portion of the adapter attached to the first strand of the polynucleotide. This promotes facile capture of the second strand of the polynucleotide for characterisation, e.g. sequencing. In some embodiments the leader sequence of the adapter attached to the second strand of the polynucleotide is longer than the single-stranded portion of the adapter attached to the first strand of the polynucleotide and so the hybridisation is prevented. Thus, in some embodiments the polynucleotide is double stranded and comprises a single stranded leader sequence at one end of a first strand of the double stranded polynucleotide and has polynucleotide binding protein bound thereto at one end of the second strand of the double stranded polynucleotide, and the portion of the second strand on which the polynucleotide binding protein is bound does not hybridise with the first strand. In some embodiments the portion of the second strand on which the polynucleotide binding protein is bound is part of the second strand of the polynucleotide. In some embodiments the portion of the second strand on which the polynucleotide binding protein is bound is part of an adapter such as a Y adapter. In some embodiments the Y adaptor comprises a pausing moiety on the first strand. In some embodiments the second strand comprises a sequence complementary to a tag sequence. An example of this is shown in Figure 3.

In some embodiments the leader sequence of the adapter attached to the second strand of the polynucleotide hybridises to a single-stranded portion of the first strand or an adapter attached to the first strand of the polynucleotide. This prevents capture of the second strand of the polynucleotide until the free translocation of the first strand has completed. For example, in some embodiments the second strand comprises a leader sequence that is hybridised to the first strand such that it is captured by the nanopore only after the first strand has moved through the nanopore. The leader sequence of the second strand may therefore be occluded by the first strand, e.g. by a leader sequence on the first strand.

In some embodiments the leader sequence of the adapter attached to the second strand of the polynucleotide is the same length as the single-stranded portion of the adapter attached to the first strand of the polynucleotide. Thus, in some embodiments the polynucleotide is double stranded and comprises a single stranded leader sequence at one end of a first strand of the double stranded polynucleotide and has polynucleotide binding protein bound thereto at one end of the second strand of the double stranded polynucleotide, and the portion of the second strand on which the polynucleotide binding protein is bound hybridises with the first strand. In some embodiments the portion of the second strand on which the polynucleotide binding protein is bound is part of the second strand of the polynucleotide. In some embodiments the portion of the second strand on which the polynucleotide binding protein is bound is part of an adapter such as a Y adapter. In some embodiments the Y adaptor comprises a pausing moiety on the first strand. In some embodiments the second strand comprises a sequence complementary to a tag sequence. An example of this is shown in Figure 4 (B) and (C).

In some embodiments a double stranded polynucleotide comprises a leader sequence at one end of a first part (e.g. a first strand) and an oligonucleotide is hybridised to the second strand, wherein the oligonucleotide comprises (a) a portion complementary to a portion of the second strand and (b) (i) a portion complementary to a tag sequence or (ii) an affinity molecule capable of binding to a tag. In some embodiments a polynucleotide handling enzyme is bound to the second part (e.g. the second strand) of the polynucleotide. In some embodiments no polynucleotide handling enzyme is bound to the first part, although in other embodiments a polynucleotide may be bound to the first part as discussed below. In some embodiments the leader sequence on the first part of the double stranded polynucleotide is at the same end of the polynucleotide as the polynucleotide handling enzyme on the second strand of the polynucleotide. In some embodiments the polynucleotide is modified with an adapter only at one end of the polynucleotide.

In some embodiments the second strand of the polynucleotide comprises a leader sequence which is inaccessible to the nanopore when the first strand is hybridised to the second strand, e.g. by being occluded by the first strand. For example, in some embodiments the leader sequence on the first part of the double stranded polynucleotide hybridises to the leader sequence on the second strand thereby preventing capture of the leader sequence on the second strand until the first strand has translocated through or across the nanopore.

In some embodiments the free translocation of the first part (e.g. the first strand) of the polynucleotide through or across the nanopore causes the portion of the oligonucleotide complementary to the tag (or the affinity molecule capable of binding to the tag) to bind to the tag or tag sequence. In some embodiments the tag or tag sequence is located on or in the vicinity of (e.g. adjacent to) the nanopore. Thus, the second part (e.g. the second strand of the double stranded polynucleotide) is held in the vicinity of the pore. After the free translocation of the first part of the polynucleotide has allowed the approximate length of the polynucleotide to be assessed, if the length is a desired length then the polynucleotide handling enzyme controls the movement of the second part of the polynucleotide through or across the nanopore, thus allowing the second part of the polynucleotide to be characterised, e.g. sequenced. An example of this is shown in Figure 6.

In some embodiments the translocation of a first strand of a double stranded polynucleotide can be temporarily paused in order to promote binding of the second strand or an oligonucleotide attached thereto to the nanopore. Pausing the translocation of the first strand of the double stranded polynucleotide does not prevent the free translocation of the first strand of the polynucleotide. For example, a pausing moiety may be provided on an adapter attached to the first strand so that the pausing moiety impedes translocation of the adapter and thus delays translocation of the first strand of the polynucleotide, but does not impede the translocation of the first strand of the polynucleotide in the region that is being assessed for length. An adapter may comprise a leader sequence and a pausing moiety and be attached to the first strand. Suitable pausing moieties are described herein, and include e.g. G-quadruplexes and similar secondary structures such as thrombin binding aptamers, and weak avidin groups as well as polynucleotide handling enzymes.

In some embodiments a double stranded polynucleotide comprises a leader sequence at one end of a first part (e.g. a first strand); and (i) an oligonucleotide is hybridised to the second strand, wherein the oligonucleotide comprises (a) a portion complementary to a portion of the second strand and (b) (i) a portion complementary to a tag sequence or (ii) an affinity molecule capable of binding to a tag or (ii) a portion of the second strand of the double stranded polynucleotide comprises a sequence complementary to a tag sequence; and a pausing moiety is attached to the first strand. The pausing moiety may be any pausing moiety as described herein. In some embodiments the pausing moiety is bound to an adapter. In some embodiments the portion of the adapter on the first strand may comprise a pausing moiety and the portion of the adapter on the second strand may have a polynucleotide binding enzyme bound thereto in order to control the movement of the second part of the polynucleotide through or across the nanopore. An example wherein an adapter is attached at only one end of the double stranded polynucleotide and an oligonucleotide is hybridised to the second strand, wherein the oligonucleotide comprises (a) a portion complementary to a portion of the second strand and (b) (i) a portion complementary to a tag sequence or (ii) an affinity molecule capable of binding to a tag is shown in Figure 8. An example wherein an adapter is attached at only one end of the double stranded polynucleotide and a portion of the second strand of the double stranded polynucleotide comprises a sequence complementary to a tag sequence is shown in Figure 10.

In some embodiments a double stranded polynucleotide is modified with an adapter at one end of the strand. In some embodiments a double stranded polynucleotide is modified with an adapter at each end of the strand. In some embodiments the adapter at each end of the strand is the same so that the first strand is identical to the second strand. As explained above, the or each adapter may comprise a pausing moiety and/or a polynucleotide handling enzyme.

For example, in some embodiments a double stranded polynucleotide comprises an adapter at each end of the double stranded polynucleotide, wherein in each adapter:
- a leader sequence is present on a first strand;
- (i) an oligonucleotide is hybridised to a second strand, wherein the oligonucleotide comprises (a) a portion complementary to a portion of the second strand and (b) (i) a portion complementary to a tag sequence or (ii) an affinity molecule capable of binding to a tag or (ii) a portion of the second strand of the adapter comprises a sequence complementary to a tag sequence;
- a polynucleotide handling enzyme is present on the second strand; an
- optionally a pausing moiety is attached to the first strand.

Examples of such embodiments are shown in Figures 7, 9 and 11. For example, in some embodiments capture of the leader sequence on a first strand by the nanopore commences translocation of the first strand which is optionally delayed e.g. temporarily paused by the pausing moiety if present, allowing the oligonucleotide or portion of the second strand comprising a sequence complementary to a tag sequence to bind to a tag or tag sequence on or in the vicinity of (e.g. adjacent to) the nanopore. The first strand of the polynucleotide can then freely translocate until the polynucleotide handling enzyme at the end of the first strand is reached. After the first strand is ejected from the nanopore the bound second strand can be characterised under the control of the polynucleotide handling enzyme on the second strand, as described above. In some embodiments, the signal caused by the capture of the first part of the polynucleotide, e.g. by the leader sequence on the first strand of the double stranded polynucleotide, is a start signal for determining the time taken for the first part of the polynucleotide to translocate through or across the nanopore. In some embodiments the contacting of a polynucleotide binding protein on the first strand with the nanopore generates a stop signal.

### Tags

The interaction between a tag on a nanopore and the binding site on a polynucleotide (*e.g*., the binding site present in an adaptor attached to a polynucleotide, wherein the binding site can be provided by an anchor or a leader sequence of an adaptor or by a capture sequence within the duplex stem of an adaptor) may be reversible. For example, a polynucleotide can bind to a tag on a nanopore, *e.g*., via its adaptor, and release at some point, *e.g*., during characterization of the polynucleotide by the nanopore and/or during processing by the polymerase. A strong non-covalent bond (*e.g*., biotin/avidin) is still reversible and can be useful in some embodiments of the methods described herein. For example, to ensure processing of a complement of a double-stranded polynucleotide following the processing of a template, it may be desirable to design the pair of pore tag and polynucleotide adaptor to provide a sufficient interaction between the complement of a double stranded polynucleotide (or a portion of an adaptor that is attached to the complement) and the nanopore such that the complement is held close to the nanopore (without detaching from the nanopore and diffusing away) but is able to release from the nanopore as it is processed.

Accordingly, in some embodiments, the pair of pore tag and polynucleotide adaptor can be configured such that the binding strength or affinity of a binding site on the polynucleotide (*e.g*., a binding site provided by an anchor or a leader sequence of an adaptor or by a capture sequence within the duplex stem of an adaptor) to a tag on a nanopore is sufficient to maintain the coupling between the nanopore and polynucleotide until an applied force is placed on it to release the bound polynucleotide from the nanopore. In some embodiments where the analyte is a double stranded polynucleotide, the applied force may be processing of a complement strand by a polymerase.

In some embodiments, the tags or tethers are uncharged. This can ensure that the tags or tethers are not drawn into the nanopore under the influence of a potential difference.

One or more molecules that attract or bind the polynucleotide or adaptor may be linked to the nanopore . Any molecule that hybridizes to the adaptor and/or target polynucleotide may be used. The molecule attached to the pore may be selected from a PNA tag, a PEG linker, a short oligonucleotide, a positively charged amino acid and an aptamer. Pores having such molecules linked to them are known in the art. For example, pores having short oligonucleotides attached thereto are disclosed in Howarka et al (2001) Nature Biotech. 19: 636-639 and WO 2010/086620, and pores comprising PEG attached within the lumen of the pore are disclosed in Howarka et al (2000) J. Am. Chem. Soc. 122(11): 2411-2416.

A short oligonucleotide attached to the nanopore (e.g. a transmembrane pore), which oligonucleotide comprises a sequence complementary to a sequence in the leader sequence or another single stranded sequence in the adaptor may be used to enhance capture of the target polynucleotide in the methods described herein.

In some embodiments, the tag or tether may comprise or be an oligonucleotide (*e.g*., DNA, RNA, LNA, BNA, PNA, or morpholino). The oligonucleotide *(e.g.,* DNA, RNA, LNA, BNA, PNA, or morpholino) can have about 10-30 nucleotides in length or about 10-20 nucleotides in length. An exemplary oligonucleotide (*e.g.*, DNA, RNA, LNA, BNA, PNA, or morpholino) may comprise a sequence as set forth in SEQ ID NO: 9. In some embodiments, the oligonucleotide (*e.g*., DNA, RNA, LNA, BNA, PNA, or morpholino) for use in the tag or tether can have at least one end (*e.g.,* 3'- or 5'-end) modified for conjugation to other modifications or to a solid substrate surface including, *e.g.,* a bead. The end modifiers may add a reactive functional group which can be used for conjugation. Examples of functional groups that can be added include, but are not limited to amino, carboxyl, thiol, maleimide, aminooxy, and any combinations thereof. The functional groups can be combined with different length of spacers (*e.g.*, C3, C9, C12, Spacer 9 and 18) to add physical distance of the functional group from the end of the oligonucleotide sequence. In some embodiments, the tag or tether may be an oligonucleotide (*e.g*., DNA, RNA, LNA, BNA, PNA, or morpholino) having a sequence as set forth in SEQ ID NO: 9 with a 5'-malemide modification. In some embodiments, the tag or tether may be an oligonucleotide (*e.g*., DNA, RNA, LNA, BNA, PNA, or morpholino) having a sequence as set forth in SEQ ID NO: 9 with a 3'-malemide modification. In some embodiments, the tag or tether may be an oligonucleotide (*e.g.*, DNA, RNA, or PNA) having a sequence as set forth in SEQ ID NO: 9 with a 5'-C9-Thiol modification. In some embodiments, the tag or tether may be an oligonucleotide (*e.g*., DNA, RNA, LNA, BNA, PNA, or morpholino) having a sequence as set forth in SEQ ID NO: 9 with a 3'-C9-Thiol modification. In some embodiments, the tag or tether may be an oligonucleotide (*e.g*., DNA, RNA, LNA, BNA, PNA, or morpholino) having a sequence as set forth in SEQ ID NO: 9 with a 5'-Thiol modification. In some embodiments, the tag or tether may be an oligonucleotide (*e.g*., DNA, RNA, LNA, BNA, PNA, or morpholino) having a sequence as set forth in SEQ ID NO: 9 with a 3'-Thiol modification.

In some embodiments, the tag or tether may comprise or be a morpholino oligonucleotide. The morpholino oligonucleotide can have about 10-30 nucleotides in length or about 10-20 nucleotides in length. An exemplary morpholino oligonucleotide may comprise a sequence as set forth in SEQ ID NO: 9. The morpholino oligonucleotides can be modified or unmodified. For example, in some embodiments, the morpholino oligonucleotide can be modified on the 3' and/or 5' ends of the oligonucleotides. Examples of modifications on the 3' and/or 5' end of the morpholino oligonucleotides include, but are not limited to 3' affinity tag and functional groups for chemical linkage (including, *e.g.,* 3'-biotin, 3'-primary amine, 3'-disulfide amide, 3'-pyridyl dithio, and any combinations thereof); 5' end modifications (including, *e.g.,* 5'-primary ammine, and/or 5'-dabcyl), modifications for click chemistry (including, *e.g.,* 3'-azide, 3'-alkyne, 5'-azide, 5'-alkyne), and any combinations thereof. In some embodiments, the tag or tether may be a morpholino oligonucleotide having a sequence as set forth in SEQ ID NO: 9 with a 5'-azide modification. In some embodiments, the tag or tether may be a morpholino oligonucleotide having a sequence as set forth in SEQ ID NO: 9 with a 3'-azide modification. In some embodiments, the tag or tether may be a morpholino oligonucleotide having a sequence as set forth in SEQ ID NO: 9 with a 5'-alkyne modification. In some embodiments, the tag or tether may be a morpholino oligonucleotide having a sequence as set forth in SEQ ID NO: 9 with a 3'-alkyne modification. In some embodiments, the tag or tether may be a morpholino oligonucleotide having a sequence as set forth in SEQ ID NO: 9 with a 3'-pyridyl dithio modification.

In some embodiments, the tag or tether may further comprise a polymeric linker, *e.g.,* to facilitate coupling to a nanopore. An exemplary polymeric linker includes, but is not limited to polyethylene glycol (PEG). The polymeric linker may have a molecular weight of about 500 Da to about 10 kDa (inclusive), or about 1 kDa to about 5 kDa (inclusive). The polymeric linker (*e.g.,* PEG) can be functionalized with different functional groups including, *e.g.,* but not limited to maleimide, NHS ester, dibenzocyclooctyne (DBCO), azide, biotin, amine, alkyne, aldehyde, and any combinations thereof. In some embodiments, the tag or tether may further comprise a 1 kDa PEG with a 5'-maleimide group and a 3'-DBCO group. In some embodiments, the tag or tether may further comprise a 2 kDa PEG with a 5'-maleimide group and a 3'-DBCO group. In some embodiments, the tag or tether may further comprise a 3 kDa PEG with a 5'-maleimide group and a 3'-DBCO group. In some embodiments, the tag or tether may further comprise a 5 kDa PEG with a 5'-maleimide group and a 3'-DBCO group.

Other examples of a tag or tether include, but are not limited to His tags, biotin or streptavidin, antibodies that bind to analytes, aptamers that bind to analytes, analyte binding domains such as DNA binding domains (including, *e.g.,* peptide zippers such as leucine zippers, single-stranded DNA binding proteins (SSB)), and any combinations thereof.

The tag or tether may be attached to the external surface of a nanopore, *e.g*., on the *cis* side of a membrane, using any methods known in the art. For example, one or more tags or tethers can be attached to the nanopore via one or more cysteines (cysteine linkage), one or more primary amines such as lysines, one or more non-natural amino acids, one or more histidines (His tags), one or more biotin or streptavidin, one or more antibody-based tags, one or more enzyme modification of an epitope (including, *e.g.,* acetyl transferase), and any combinations thereof. Suitable methods for carrying out such modifications are well-known in the art. Suitable non-natural amino acids include, but are not limited to, 4-azido-L-phenylalanine (Faz) and any one of the amino acids numbered 1-71 in Figure 1 of Liu C. C. and Schultz P. G., Annu. Rev. Biochem., 2010, 79, 413-444.

In some embodiments where one or more tags or tethers are attached to a nanopore via cysteine linkage(s), the one or more cysteines can be introduced to one or more monomers that form the nanopore by substitution. In some embodiments, the nanopore may be chemically modified by attachment of (i) Maleimides including diabromomaleimides such as: 4-phenylazomaleinanil, 1.N-(2-Hydroxyethyl)maleimide, N-Cyclohexylmaleimide, 1.3-Maleimidopropionic Acid, 1.1-4-Aminophenyl-1H-pyrrole,2,5,dione, 1.1-4-Hydroxyphenyl-1H-pyrrole,2,5,dione, N-Ethylmaleimide, N-Methoxycarbonylmaleimide, N-tert-Butylmaleimide, N-(2-Aminoethyl)maleimide , 3-Maleimido-PROXYL , N-(4-Chlorophenyl)maleimide, 1-[4-(dimethylamino)-3,5-dinitrophenyl]-1H-pyrrole-2,5-dione, N-[4-(2-Benzimidazolyl)phenyl]maleimide, N-[4-(2-benzoxazolyl)phenyl]maleimide, N-(1-naphthyl)-maleimide, N-(2,4-xylyl)maleimide, N-(2,4-difluorophenyl)maleimide , N-(3-chloro-para-tolyl)-maleimide, 1-(2-amino-ethyl)-pyrrole-2,5-dione hydrochloride, 1-cyclopentyl-3-methyl-2,5-dihydro-1H-pyrrole-2,5-dione, 1-(3-aminopropyl)-2,5-dihydro-1H-pyrrole-2,5-dione hydrochloride, 3-methyl-1-[2-oxo-2-(piperazin-1-yl)ethyl]-2,5-dihydro-1H-pyrrole-2,5-dione hydrochloride, 1-benzyl-2,5-dihydro-1H-pyrrole-2,5-dione, 3-methyl-1-(3,3,3-trifluropropyl)-2,5-dihydro-1H-pyrrole-2,5-dione, 1-[4-(methylamino)cyclohexyl]-2,5-dihydro-1H-pyrrole-2,5-dione trifluroacetic acid, SMILES O=C1C=CC(=O)N1CC=2C=CN=CC2, SMILES O=C1C=CC(=O)N1CN2CCNCC2, 1-benzyl-3-methyl-2,5-dihydro-1H-pyrrole-2,5-dione, 1-(2-fluorophenyl)-3-methyl-2,5-dihydro 1H-pyrrole-2,5-dione, N-(4-phenoxyphenyl)maleimide , N-(4-nitrophenyl)maleimide (ii) Iodocetamides such as :3-(2-Iodoacetamido)-proxyl, N-(cyclopropylmethyl)-2-iodoacetamide, 2-iodo-N-(2-phenylethyl)acetamide, 2-iodo-N-(2,2,2-trifluoroethyl)acetamide, N-(4-acetylphenyl)-2-iodoacetamide, N-(4-(aminosulfonyl)phenyl)-2-iodoacetamide, N-(1,3-benzothiazol-2-yl)-2-iodoacetamide, N-(2,6-diethylphenyl)-2-iodoacetamide, N-(2-benzoyl-4-chlorophenyl)-2-iodoacetamide, (iii) Bromoacetamides: such as N-(4-(acetylamino)phenyl)-2-bromoacetamide , N-(2-acetylphenyl)-2-bromoacetamide , 2-bromo-n-(2-cyanophenyl)acetamide, 2-bromo-N-(3-(trifluoromethyl)phenyl)acetamide, N-(2-benzoylphenyl)-2-bromoacetamide , 2-bromo-N-(4-fluorophenyl)-3-methylbutanamide, N-Benzyl-2-bromo-N-phenylpropionamide, N-(2-bromo-butyryl)-4-chloro-benzenesulfonamide, 2-Bromo-N-methyl-N-phenylacetamide, 2-bromo-N-phenethyl-acetamide,2-adamantan-1-yl-2-bromo-N-cyclohexyl-acetamide, 2-bromo-N-(2-methylphenyl)butanamide, Monobromoacetanilide, (iv) Disulphides such as: aldrithiol-2 , aldrithiol-4 , isopropyl disulfide, 1-(Isobutyldisulfanyl)-2-methylpropane, Dibenzyl disulfide, 4-aminophenyl disulfide, 3-(2-Pyridyldithio)propionic acid, 3-(2-Pyridyldithio)propionic acid hydrazide, 3-(2-Pyridyldithio)propionic acid N-succinimidyl ester, am6amPDP1-βCD and (v) Thiols such as: 4-Phenylthiazole-2-thiol, Purpald, 5,6,7,8-tetrahydro-quinazoline-2-thiol.

In some embodiments, the tag or tether may be attached directly to a nanopore or via one or more linkers. The tag or tether may be attached to the nanopore using the hybridization linkers described in WO 2010/086602. Alternatively, peptide linkers may be used. Peptide linkers are amino acid sequences. The length, flexibility and hydrophilicity of the peptide linker are typically designed such that it does not to disturb the functions of the monomer and pore. Preferred flexible peptide linkers are stretches of 2 to 20, such as 4, 6, 8, 10 or 16, serine and/or glycine amino acids. More preferred flexible linkers include (SG)₁, (SG)₂, (SG)₃, (SG)₄, (SG)₅ and (SG)₈ wherein S is serine and G is glycine. Preferred rigid linkers are stretches of 2 to 30, such as 4, 6, 8, 16 or 24, proline amino acids. More preferred rigid linkers include (P)₁₂ wherein P is proline.

### Spacers

In some embodiments, a polynucleotide or adapter may comprise a spacer. For example, one or more spacers may be present in the polynucleotide adapter. For example, the polynucleotide adapter may comprise from one to about 10 spacers, e.g. from 1 to about 5 spacers, e.g. 1, 2, 3, 4 or 5 spacers. The spacer may comprise any suitable number of spacer units. A spacer provides an energy barrier which impedes movement of a polynucleotide binding protein. For example, a spacer may stall the polynucleotide binding protein by reducing the traction of the polynucleotide binding protein. This may be achieved for instance by using an abasic spacer i.e. a spacer in which the bases are removed from one or more nucleotides in the polynucleotide adapter. A spacer may physically block movement of the polynucleotide binding protein, for instance by introducing a bulky chemical group to physically impede the movement of the polynucleotide binding protein.

In some embodiments, one or more spacers are included in the polynucleotide or in an adapter as used in the methods claimed herein in order to provide a distinctive signal when they pass through or across a nanopore. Suitable signals include start signals and stop signals as described in more detail herein.

In some embodiments, a spacer may comprise a linear molecule, such as a polymer. Typically, such a spacer has a different structure from the target polynucleotide. For instance, if the target polynucleotide is DNA, the or each spacer typically does not comprise DNA. In particular, if the target polynucleotide is deoxyribonucleic acid (DNA) or ribonucleic acid (RNA), the or each spacer preferably comprises peptide nucleic acid (PNA), glycerol nucleic acid (GNA), threose nucleic acid (TNA), locked nucleic acid (LNA), bridged nucleic acid (BNA) or a synthetic polymer with nucleotide side chains. In some embodiments, a spacer may comprise one or more nitroindoles, one or more inosines, one or more acridines, one or more 2-aminopurines, one or more 2-6-diaminopurines, one or more 5-bromo-deoxyuridines, one or more inverted thymidines (inverted dTs), one or more inverted dideoxy-thymidines (ddTs), one or more dideoxy-cytidines (ddCs), one or more 5-methylcytidines, one or more 5-hydroxymethylcytidines, one or more 2'-O-Methyl RNA bases, one or more Iso-deoxycytidines (Iso-dCs), one or more Iso-deoxyguanosines (Iso-dGs), one or more C3 (OC₃H₆OPO₃) groups, one or more photo-cleavable (PC) [OC₃H₆-C(O)NHCH₂-C₆H₃NO₂-CH(CH₃)OPO₃] groups, one or more hexandiol groups, one or more spacer 9 (iSp9) [(OCH₂CH₂)₃OPO₃] groups, or one or more spacer 18 (iSp18) [(OCH₂CH₂)₆OPO₃] groups; or one or more thiol connections. A spacer may comprise any combination of these groups. Many of these groups are commercially available from IDT^{®} (Integrated DNA Technologies^{®}). For example, C3, iSp9 and iSp18 spacers are all available from IDT^{®}. A spacer may comprise any number of the above groups as spacer units.

In some embodiments, a spacer may comprise one or more chemical groups which cause the polynucleotide binding protein to stall. In some embodiments, suitable chemical groups are one or more pendant chemical groups. The one or more chemical groups may be attached to one or more nucleobases in the polynucleotide adapter. The one or more chemical groups may be attached to the backbone of the polynucleotide adapter. Any number of appropriate chemical groups may be present, such as 2, 3, 4, 5, 6, 7, 8, 9, 10, 11, 12 or more. Suitable groups include, but are not limited to, fluorophores, streptavidin and/or biotin, cholesterol, methylene blue, dinitrophenols (DNPs), digoxigenin and/or anti-digoxigenin and dibenzylcyclooctyne groups. In some embodiments, a spacer may comprise a polymer. In some embodiments the spacer may comprise a polymer which is a polypeptide or a polyethylene glycol (PEG).

In some embodiments, a spacer may comprise one or more abasic nucleotides (i.e. nucleotides lacking a nucleobase), such as 2, 3, 4, 5, 6, 7, 8, 9, 10, 11, 12 or more abasic nucleotides. The nucleobase can be replaced by -H (idSp) or -OH in the abasic nucleotide. Abasic spacers can be inserted into target polynucleotides by removing the nucleobases from one or more adjacent nucleotides. For instance, polynucleotides may be modified to include 3-methyladenine, 7-methylguanine, 1,N6-ethenoadenine inosine or hypoxanthine and the nucleobases may be removed from these nucleotides using Human Alkyladenine DNA Glycosylase (hAAG). Alternatively, polynucleotides may be modified to include uracil and the nucleobases removed with Uracil-DNA Glycosylase (UDG). In one embodiment, the one or more spacers do not comprise any abasic nucleotides.

Suitable spacers can be designed or selected depending on the nature of the polynucleotide adapter, the polynucleotide binding protein and the conditions under which the method is to be carried out. For example, many polynucleotide binding proteins process DNA *in vivo* and such polynucleotide binding proteins may typically be stalled using anything that is not DNA.

### Polynucleotide binding protein

As described above, when the assessment of the first part of the polynucleotide confirms that the polynucleotide has one or more desired properties, the disclosed methods comprise controlling the movement of a second part of the polynucleotide with respect to a nanopore. For example, when assessing the size of the polynucleotide, and assessment of the length of the polynucleotide confirms that the polynucleotide is of the desired length, the disclosed methods comprise controlling the movement of a second part of the polynucleotide through or across the nanopore. The movement of the second part of the polynucleotide is controlled using a polynucleotide binding protein capable of controlling the movement of a polynucleotide with respect to the nanopore.

In some embodiments, when a polynucleotide binding protein is present on an adapter, or otherwise present on a portion of the polynucleotide, the polynucleotide binding protein is modified to prevent the polynucleotide binding protein disengaging from the polynucleotide or adapter (other than by passing off the end of the polynucleotide/adapter). The polynucleotide binding protein can be adapted in any suitable way. For example, the polynucleotide binding protein can be loaded onto the adapter or polynucleotide and then modified in order to prevent it from disengaging. Alternatively, the polynucleotide binding protein can be modified to prevent it from disengaging before it is loaded onto the adapter or polynucleotide. Modification of a polynucleotide binding protein in order to prevent it from disengaging from a polynucleotide or adapter can be achieved using methods known in the art, such as those discussed in WO 2014/013260, and with particular reference to passages describing the modification of polynucleotide binding proteins such as helicases in order to prevent them from disengaging with polynucleotide strands.

For example, the polynucleotide binding protein may have a polynucleotide-unbinding opening; e.g. a cavity, cleft or void through which a polynucleotide strand may pass when the polynucleotide binding protein disengages from the strand. In some embodiments, the polynucleotide-unbinding opening for a given polynucleotide binding protein can be determined by reference to its structure, e.g. by reference to its X-ray crystal structure. The X-ray crystal structure may be obtained in the presence and/or the absence of a polynucleotide substrate. In some embodiments, the location of a polynucleotide-unbinding opening in a given polynucleotide binding protein may be deduced or confirmed by molecular modelling using standard packages known in the art. In some embodiments, the polynucleotide-unbinding opening may be transiently produced by movement of one or more parts e.g. one or more domains of the polynucleotide binding protein.

The polynucleotide binding protein may be modified by closing the polynucleotide-unbinding opening. Closing the polynucleotide-unbinding opening may therefore prevent the polynucleotide binding protein from disengaging from the polynucleotide or adapter. For example, the polynucleotide binding protein may be modified by covalently closing the polynucleotide-unbinding opening. In some embodiments, a preferred polynucleotide binding protein for addressing in this way is a helicase.

In one embodiment, a polynucleotide binding protein may be any protein that is capable of binding to a polynucleotide and controlling its movement with respect to a nanopore, e.g. through the pore.

In one embodiment, a polynucleotide binding protein is or is derived from a polynucleotide handling enzyme. A polynucleotide handling enzyme is a polypeptide that is capable of interacting with and modifying at least one property of a polynucleotide. The enzyme may modify the polynucleotide by cleaving it to form individual nucleotides or shorter chains of nucleotides, such as di- or trinucleotides. The enzyme may modify the polynucleotide by orienting it or moving it to a specific position.

In one embodiment, the polynucleotide binding protein is derived from a member of any of the Enzyme Classification (EC) groups 3.1.11, 3.1.13, 3.1.14, 3.1.15, 3.1.16, 3.1.21, 3.1.22, 3.1.25, 3.1.26, 3.1.27, 3.1.30 and 3.1.31.

Typically, the polynucleotide binding protein is a helicase, a polymerase, an exonuclease, a topoisomerase, or a variant thereof.

In one embodiment, the polynucleotide binding protein is an exonuclease. Suitable enzymes include, but are not limited to, exonuclease I from *E. coli* (SEQ ID NO: 1), exonuclease III enzyme from *E. coli* (SEQ ID NO: 2), RecJ from *T. thermophilus* (SEQ ID NO: 3) and bacteriophage lambda exonuclease (SEQ ID NO: 4), TatD exonuclease and variants thereof. Three subunits comprising the sequence shown in SEQ ID NO: 3 or a variant thereof interact to form a trimer exonuclease.

In one embodiment, the polynucleotide binding protein is a polymerase. The polymerase may be PyroPhage^{®} 3173 DNA Polymerase (which is commercially available from Lucigen^{®} Corporation), SD Polymerase (commercially available from Bioron^{®}), Klenow from NEB or variants thereof. In one embodiment, the enzyme is Phi29 DNA polymerase (SEQ ID NO: 5) or a variant thereof. Modified versions of Phi29 polymerase that may be used in the invention are disclosed in US Patent No. 5,576,204.

In one embodiment the polynucleotide binding protein is a topoisomerase. In one embodiment, the topoisomerase is a member of any of the Moiety Classification (EC) groups 5.99.1.2 and 5.99.1.3. The topoisomerase may be a reverse transcriptase, which are enzymes capable of catalysing the formation of cDNA from a RNA template. They are commercially available from, for instance, New England Biolabs^{®} and Invitrogen^{®}.

In one embodiment, the polynucleotide binding protein is a helicase. Any suitable helicase can be used in accordance with the methods provided herein. The helicase may be a helicase selected from SF1, SF2,SF3, SF4, SF5 or SF6 families. Helicases in families SF1-SF6 are well known to those skilled in the art. For example, the or each polynucleotide binding protein used in accordance with the present disclosure may be independently selected from a Hel308 helicase, a RecD helicase, a TraI helicase, a TrwC helicase, an XPD helicase, and a Dda helicase, or a variant thereof. Monomeric helicases may comprise several domains attached together. For instance, TraI helicases and TraI subgroup helicases may contain two RecD helicase domains, a relaxase domain and a C-terminal domain. The domains typically form a monomeric helicase that is capable of functioning without forming oligomers. Particular examples of suitable helicases include Hel308, NS3, Dda, UvrD, Rep, PcrA, Pif1 and TraI. These helicases typically work on single stranded DNA. Examples of helicases that can move along both strands of a double stranded DNA include FtsK and hexameric enzyme complexes, or multisubunit complexes such as RecBCD. In some embodiments a given polynucleotide binding protein may be used in the free translocation of the first part of the polynucleotide and/or in the controlled translocation of the second part of the polynucleotide. The operation of the polynucleotide binding protein can be controlled or selected by altering the experimental conditions, for example, e.g. by controlling the presence or absence of fuel molecules.

Hel308 helicases are described in publications such as WO 2013/057495. RecD helicases are described in publications such as WO 2013/098562. XPD helicases are described in publications such as WO 2013/098561. Dda helicases are described in publications such as WO 2015/055981 and WO 2016/055777.

In one embodiment the helicase comprises the sequence shown in SEQ ID NO: 6 (Trwc Cba) or a variant thereof, the sequence shown in SEQ ID NO: 7 (Hel308 Mbu) or a variant thereof or the sequence shown in SEQ ID NO: 8 (Dda) or a variant thereof. Variants may differ from the native sequences in any of the ways discussed herein. An example variant of SEQ ID NO: 8 comprises E94C/A360C. A further example variant of SEQ ID NO: 8 comprises E94C/A360C and then (ΔM1)G1G2 (i.e. deletion of M1 and then addition of G1 and G2).

Typically, the polynucleotide binding protein is not an ultra-fast polynucleotide handling enzyme capable of controlling the free movement of the first part of the polynucleotide with respect to a nanopore as described herein, Typically, the polynucleotide binding protein is not an ultra-fast polynucleotide handling enzyme capable of controlling the free translocation of the first part of the polynucleotide through or across the nanopore in the methods provided herein. In some embodiments the polynucleotide binding protein controls the rate of translocation of the second part of the polynucleotide through or across the nanopore at a rate of less than 20 kb/s, such as less than 10 kb/s, e.g up to about 5 kb/s, e.g. at most 4 kb/s e.g. at most 2 kb/s, such as at most 1 kb/s. Thus, in some embodiments, polynucleotide binding proteins (e.g. polynucleotide binding proteins which have been used in the art to sequence the first and second part of a polynucleotide strand) are suitable for controlling the movement of the second part of the polynucleotide strand but are not suitable for freely translocating the first part of the polynucleotide through or across the nanopore.

In some embodiments a polynucleotide binding protein (e.g. a helicase) can control the movement of polynucleotides in at least two active modes of operation (when the polynucleotide binding protein is provided with all the necessary components to facilitate movement, e.g. fuel and cofactors such as ATP and Mg²⁺ discussed herein) and one inactive mode of operation (e.g. when the polynucleotide binding protein is not provided with the necessary components to facilitate movement or when the polynucleotide binding protein is to altered to prevent such movement).

When provided with all the necessary components to facilitate movement (i.e. in the active modes), the polynucleotide binding protein (e.g. helicase) moves along the polynucleotide in a 5' to 3' or a 3' to 5' direction (depending on the polynucleotide binding protein). In embodiments in which the polynucleotide binding protein is used to control the movement of a polynucleotide strand with respect to a nanopore, the polynucleotide binding protein can be used to either move the polynucleotide away from (e.g. out of) the pore (e.g. against an applied field) or the polynucleotide towards (e.g. into) the pore (e.g. with an applied field). For example, when the end of the polynucleotide towards which the polynucleotide binding protein moves is captured by a pore, the polynucleotide binding protein works against the direction of the field resulting from the applied potential and pulls the threaded polynucleotide out of the pore (e.g. into the *cis* chamber). However, when the end away from which the polynucleotide binding protein moves is captured in the pore, the polynucleotide binding protein works with the direction of the field resulting from the applied potential and pushes the threaded polynucleotide into the pore (e.g. into the *trans* chamber).

When the polynucleotide binding protein (e.g. helicase) is not provided with the necessary components to facilitate movement (i.e. in the inactive mode) it can bind to the polynucleotide and act as a brake slowing the movement of the polynucleotide when it is moved with respect to a nanopore, e.g. by being pulled into the pore by a field resulting from an applied potential. In the inactive mode, it does not matter which end of the polynucleotide is captured, it is the applied field which determines the movement of the polynucleotide with respect to the pore, and the polynucleotide binding protein acts as a brake. When in the inactive mode, the movement control of the polynucleotide by the polynucleotide binding protein can be described in a number of ways including ratcheting, sliding and braking.

A polynucleotide binding protein typically requires fuel in order to handle the processing of polynucleotides. Fuel is typically free nucleotides or free nucleotide analogues. The free nucleotides may be one or more of, but are not limited to, adenosine monophosphate (AMP), adenosine diphosphate (ADP), adenosine triphosphate (ATP), guanosine monophosphate (GMP), guanosine diphosphate (GDP), guanosine triphosphate (GTP), thymidine monophosphate (TMP), thymidine diphosphate (TDP), thymidine triphosphate (TTP), uridine monophosphate (UMP), uridine diphosphate (UDP), uridine triphosphate (UTP), cytidine monophosphate (CMP), cytidine diphosphate (CDP), cytidine triphosphate (CTP), cyclic adenosine monophosphate (cAMP), cyclic guanosine monophosphate (cGMP), deoxyadenosine monophosphate (dAMP), deoxyadenosine diphosphate (dADP), deoxyadenosine triphosphate (dATP), deoxyguanosine monophosphate (dGMP), deoxyguanosine diphosphate (dGDP), deoxyguanosine triphosphate (dGTP), deoxythymidine monophosphate (dTMP), deoxythymidine diphosphate (dTDP), deoxythymidine triphosphate (dTTP), deoxyuridine monophosphate (dUMP), deoxyuridine diphosphate (dUDP), deoxyuridine triphosphate (dUTP), deoxycytidine monophosphate (dCMP), deoxycytidine diphosphate (dCDP) and deoxycytidine triphosphate (dCTP). The free nucleotides are usually selected from AMP, TMP, GMP, CMP, UMP, dAMP, dTMP, dGMP or dCMP. The free nucleotides are typically adenosine triphosphate (ATP).

A cofactor for the polynucleotide binding protein is a factor that allows the polynucleotide binding protein to function. The cofactor is preferably a divalent metal cation. The divalent metal cation is preferably Mg²⁺, Mn²⁺, Ca²⁺ or Co²⁺. The cofactor is most preferably Mg²⁺. In embodiments provided herein, the divalent metal cation can be present in solution present either or both of the *cis* and/or *trans* sides of the nanopore.

### Measurements and characteristics that can be determined

In one embodiment, the presence, absence or one or more characteristics of a target polynucleotide are determined. The methods may be for determining the presence, absence or one or more characteristics of at least one target polynucleotide. The methods may concern determining the presence, absence or one or more characteristics of two or more target polynucleotide. The methods may comprise determining the presence, absence or one or more characteristics of any number of target polynucleotides, such as 2, 5, 10, 15, 20, 30, 40, 50, 100 or more target polynucleotides. Any number of characteristics of the one or more target polynucleotides may be determined, such as 1, 2, 3, 4, 5, 10 or more characteristics.

The one or more characteristics of the polynucleotide which are selectively determined in the methods provided herein typically comprise the sequence of the polynucleotides. Other characteristics that can be selectively determined in the methods provided herein include determining whether or not the selected polynucleotides are modified and the extent and/or number of any such modifications that may be present; the identity of the selected polynucleotides, and the secondary structure of the polynucleotides. Modifications that may be characterised in the methods of the invention include whether and to what extend the polynucleotide is modified by methylation, by oxidation, by damage, with one or more proteins or with one or more labels, tags or spacer.

### Sample of polynucleotides

Detecting the presence of biological molecules and characterising the detected biological molecules finds application in personalised drug development, medicine, diagnostics, life science research, environmental monitoring and in the security and/or the defence industry. As such, the methods described herein find application in at least these industries.

The polynucleotides in the sample to be selectively characterised in the methods described herein are also referred to herein as "target nucleotide(s)". The polynucleotide in the sample to be selectively characterised in the methods described herein may thus be referred to as the "target nucleotide". In some embodiments the sample of polynucleotides assessed in the methods described herein may be formed from an impure mixture of one or more target polynucleotides and one or more impurities. Impurities may comprise truncated forms of target polynucleotides and/or polynucleotides which are distinct from the target polynucleotides. For example the target polynucleotide may be genomic DNA and undesired polynucleotides may comprise fractions of the genomic DNA, plasmids, etc. The target polynucleotide may be a coding region of genomic DNA and undesired polynucleotides may comprise non-coding regions of DNA. For example, the human genome comprises approximately 50 Mb of coding DNA and about 3000 Mb of non-coding DNA, so that the target polynucleotide may be coding regions of the human genome and undesired polynucleotides may comprise non-coding regions of the human genome.

The polynucleotides in the sample may be secreted from cells. Alternatively, the polynucleotides in the sample may be present inside cells such that they must be extracted from the cells before the method can be carried out.

In one embodiment, the target polynucleotide is a nucleic acid. A polynucleotide is defined as a macromolecule comprising two or more nucleotides. The naturally-occurring nucleic acid bases in DNA and RNA may be distinguished by their physical size. As a nucleic acid molecule, or individual base, passes through the channel of a nanopore, the size differential between the bases causes a directly correlated reduction in the ion flow through the channel. The variation in ion flow may be recorded as described above. Suitable electrical measurement techniques for recording ion flow variations are described in, for example, WO 2000/28312 and D. Stoddart et al., Proc. Natl. Acad. Sci., 2010, 106, pp 7702-7 (single channel recording equipment); and, for example, in WO 2009/077734 (multi-channel recording techniques). Through suitable calibration, the characteristic reduction in ion flow can be used to identify the particular nucleotide and associated base traversing the channel in real-time. In typical nanopore nucleic acid sequencing, the open-channel ion flow is reduced as the individual nucleotides of the nucleic sequence of interest sequentially pass through the channel of the nanopore due to the partial blockage of the channel by the nucleotide. It is this reduction in ion flow that is measured using the suitable recording techniques described above. The reduction in ion flow may be calibrated to the reduction in measured ion flow for known nucleotides through the channel resulting in a means for determining which nucleotide is passing through the channel, and therefore, when done sequentially, a way of determining the nucleotide sequence of the nucleic acid passing through the nanopore. For the accurate determination of individual nucleotides, it has typically required for the reduction in ion flow through the channel to be directly correlated to the size of the individual nucleotide passing through the constriction (or "reading head"). It will be appreciated that sequencing may be performed upon an intact nucleic acid polymer that is 'threaded' through the pore via the action of an associated polynucleotide binding protein such as a polymerase or helicase, for example. Alternatively, sequences may be determined by passage of nucleotide triphosphate bases that have been sequentially removed from a target nucleic acid in proximity to the pore (see for example WO 2014/187924).

The polynucleotide or nucleic acid may comprise any combination of any nucleotides. The nucleotides can be naturally occurring or artificial. One or more nucleotides in the polynucleotide can be oxidized or methylated. One or more nucleotides in the polynucleotide may be damaged. For instance, the polynucleotide may comprise a pyrimidine dimer. Such dimers are typically associated with damage by ultraviolet light and are the primary cause of skin melanomas. One or more nucleotides in the polynucleotide may be modified, for instance with a label or a tag, for which suitable examples are known by a skilled person. The polynucleotide may comprise one or more spacers. A nucleotide typically contains a nucleobase, a sugar and at least one phosphate group. The nucleobase and sugar form a nucleoside. The nucleobase is typically heterocyclic. Nucleobases include, but are not limited to, purines and pyrimidines and more specifically adenine (A), guanine (G), thymine (T), uracil (U) and cytosine (C). The sugar is typically a pentose sugar. Nucleotide sugars include, but are not limited to, ribose and deoxyribose. The sugar is preferably a deoxyribose. The polynucleotide preferably comprises the following nucleosides: deoxyadenosine (dA), deoxyuridine (dU) and/or thymidine (dT), deoxyguanosine (dG) and deoxycytidine (dC). The nucleotide is typically a ribonucleotide or deoxyribonucleotide. The nucleotide typically contains a monophosphate, diphosphate or triphosphate. The nucleotide may comprise more than three phosphates, such as 4 or 5 phosphates. Phosphates may be attached on the 5' or 3' side of a nucleotide. The nucleotides in the polynucleotide may be attached to each other in any manner. The nucleotides are typically attached by their sugar and phosphate groups as in nucleic acids. The nucleotides may be connected via their nucleobases as in pyrimidine dimers. The polynucleotide may be single stranded or double stranded. At least a portion of the polynucleotide is preferably double stranded. The polynucleotide is most preferably ribonucleic nucleic acid (RNA) or deoxyribonucleic acid (DNA). In particular, said method using a polynucleotide as an analyte alternatively comprises determining one or more characteristics selected from (i) the length of the polynucleotide, (ii) the identity of the polynucleotide, (iii) the sequence of the polynucleotide, (iv) the secondary structure of the polynucleotide and (v) whether or not the polynucleotide is modified.

The polynucleotide can be any length (i). For example, the polynucleotide can be at least 10, at least 50, at least 100, at least 150, at least 200, at least 250, at least 300, at least 400 or at least 500 nucleotides or nucleotide pairs in length. The polynucleotide can be 1000 or more nucleotides or nucleotide pairs, 5000 or more nucleotides or nucleotide pairs in length or 100000 or more nucleotides or nucleotide pairs in length. The first part of the polynucleotide may be any suitable length. The first part of the polynucleotide assessed in the disclosed methods is may be at least 1 kb (i.e. 1000 nucleotides or nucleotide pairs) in length. In some embodiments, the first part of the polynucleotide is at least 2 kb, e.g. at least 5 kb, e.g. at least 10 kb, such as at least 20 kb, e.g. at least 50 kb, e.g. at least 100 kb in length, e.g. at least 1000 kb, e.g. at least 10,000 kb such as at least 50,000 kb or more.

Any number of polynucleotides can be investigated. For instance, the method may concern characterising 2, 3, 4, 5, 6, 7, 8, 9, 10, 20, 30, 50, 100 or more polynucleotides. If two or more polynucleotides are characterised, they may be different polynucleotides or two instances of the same polynucleotide. The polynucleotide can be naturally occurring or artificial. For instance, the method may be used to verify the sequence of a manufactured oligonucleotide. The method is typically carried out *in vitro.*

Nucleotides can have any identity (ii), and include, but are not limited to, adenosine monophosphate (AMP), guanosine monophosphate (GMP), thymidine monophosphate (TMP), uridine monophosphate (UMP), 5-methylcytidine monophosphate, 5-hydroxymethylcytidine monophosphate, cytidine monophosphate (CMP), cyclic adenosine monophosphate (cAMP), cyclic guanosine monophosphate (cGMP), deoxyadenosine monophosphate (dAMP), deoxyguanosine monophosphate (dGMP), deoxythymidine monophosphate (dTMP), deoxyuridine monophosphate (dUMP), deoxycytidine monophosphate (dCMP) and deoxymethylcytidine monophosphate. The nucleotides are preferably selected from AMP, TMP, GMP, CMP, UMP, dAMP, dTMP, dGMP, dCMP and dUMP. A nucleotide may be abasic (i.e. lack a nucleobase). A nucleotide may also lack a nucleobase and a sugar (i.e. is a C3 spacer). The sequence of the nucleotides (iii) is determined by the consecutive identity of following nucleotides attached to each other throughout the polynucleotide strain, in the 5' to 3' direction of the strand.

The target polynucleotide may comprise the products of a PCR reaction, genomic DNA, the products of an endonuclease digestion and/or a DNA library. The target polynucleotide may be obtained from or extracted from any organism or microorganism. The target polynucleotide is often obtained from a human or animal, e.g. from urine, lymph, saliva, mucus, seminal fluid or amniotic fluid, or from whole blood, plasma or serum. The target polynucleotide may be obtained from a plant e.g. a a cereal, legume, fruit or vegetable. The target polynucleotide may comprise genomic DNA. The genomic DNA may be fragmented. The DNA may be fragmented by any suitable method. For example, methods of fragmenting DNA are known in the art, Such methods may use a transposase, such as a MuA transposase. Often the genomic DNA is not fragmented. In some embodiments, the target polynucleotide may be DNA, RNA and/or a DNA/RNA hybrid.

### Detection methods

The polynucleotide may be characterised in the methods provided herein in any suitable manner. In one embodiment the polynucleotide is characterised by detecting an ionic current or optical signal as the polynucleotide moves with respect to a nanopore. This is described in more detail herein. The method is amenable to these and other methods of detecting polynucleotides.

In another non-limiting example, in one embodiment the polynucleotide is characterised by detecting the by-products of a polynucleotide-processing reaction, such as a sequencing by synthesis reaction. The method may thus involve detecting the product of the sequential addition of (poly)nucleotides by an enzyme such as a polymerase to the nucleic acid strand. The product may be a change in one or more properties of the enzyme such as in the conformation of the enzyme. Such methods may thus comprise subjecting an enzyme such as polymerase or a reverse transcriptase to a double-stranded polynucleotide under conditions such that the template-dependent incorporation of nucleotide bases into a growing oligonucleotide strand causes conformational changes in the enzyme in response to sequentially encountering template strand nucleic acid bases and/or incorporating template-specified natural or analog bases (i.e., an incorporation event), detecting the conformational changes in the enzyme in response to such incorporation events, and thereby detecting the sequence of the template strand. In such methods the polynucleotide strand may be moved in accordance with the methods provided herein. Such methods may involve detecting and/or measuring incorporation events using methods known to those skilled in the art, such as those described in US 2017/0044605.

In another embodiment, by-products may be labelled so that a phosphate labelled species is released upon the addition of a nucleotide to a synthesised nucleic acid strand that is complementary to the template strand, and the phosphate labelled species is detected e.g. using a nanopore as described herein. The polynucleotide being characterised in this way may be moved in accordance with the methods herein. Suitable labels may be optical labels that are detected using a nanopore, or a zero mode wave guide, or by Raman spectroscopy, or other detectors. Suitable labels may be non-optical labels that are detected using a nanopore, or other detectors.

In another approach, nucleoside phosphates (nucleotides) are not labelled and upon the addition of a nucleotide to a synthesised nucleic acid strand that is complementary to the template strand, a natural by-product species is detected. Suitable detectors may be ion-sensitive field-effect transistors, or other detectors.

These and other detection methods are suitable for use in the methods described herein. Any suitable measurements can be taken using a nanopore as the polynucleotide moves with respect to the nanopore.

### Nanopore

Any suitable transmembrane nanopore can be used.

A transmembrane nanopore is a structure that crosses the membrane to some degree. It permits hydrated ions driven by an applied potential to flow across or within the membrane. The transmembrane nanopore typically crosses the entire membrane so that hydrated ions may flow from one side of the membrane to the other side of the membrane. However, the transmembrane nanopore does not have to cross the membrane. It may be closed at one end. For instance, the nanopore may be a well, gap, channel, trench or slit in the membrane along which or into which hydrated ions may flow.

Any transmembrane nanopore may be used in the methods provided herein. The nanopore may be biological or artificial. Suitable pores include, but are not limited to, protein pores, polynucleotide pores and solid state pores. The nanopore may be a DNA origami pore (Langecker et al., Science, 2012; 338: 932-936). Suitable DNA origami pores are disclosed in WO2013/083983.

In one embodiment, the nanopore is a transmembrane protein pore. A transmembrane protein pore is a polypeptide or a collection of polypeptides that permits hydrated ions, such as polynucleotides, to flow from one side of a membrane to the other side of the membrane. In the methods provided herein, the transmembrane protein pore is capable of forming a pore that permits hydrated ions driven by an applied potential to flow from one side of the membrane to the other. The transmembrane protein pore preferably permits polynucleotides to flow from one side of the membrane, such as a triblock copolymer membrane, to the other. The transmembrane protein pore typically allows a polynucleotide to be moved through the pore.

In one embodiment, the nanopore is a transmembrane protein pore which is a monomer or an oligomer. The pore is preferably made up of several repeating subunits, such as at least 6, at least 7, at least 8, at least 9, at least 10, at least 11, at least 12, at least 13, at least 14, at least 15, or at least 16 subunits. The pore is preferably a hexameric, heptameric, octameric or nonameric pore. The pore may be a homo-oligomer or a hetero-oligomer.

In one embodiment, the transmembrane protein pore comprises a barrel or channel through which the ions may flow. The subunits of the pore typically surround a central axis and contribute strands to a transmembrane β-barrel or channel or a transmembrane α-helix bundle or channel.

Typically, the barrel or channel of the transmembrane protein pore comprises amino acids that facilitate interaction with an analyte, such as a target polynucleotide (as described herein). These amino acids are preferably located near a constriction of the barrel or channel. The transmembrane protein pore typically comprises one or more positively charged amino acids, such as arginine, lysine or histidine, or aromatic amino acids, such as tyrosine or tryptophan. These amino acids typically facilitate the interaction between the pore and nucleotides, polynucleotides or nucleic acids.

In one embodiment, the nanopore is a transmembrane protein pore derived from β-barrel pores or α-helix bundle pores. β-barrel pores comprise a barrel or channel that is formed from β-strands. Suitable β-barrel pores include, but are not limited to, β-toxins, such as α-hemolysin, anthrax toxin and leukocidins, and outer membrane proteins/porins of bacteria, such as *Mycobacterium smegmatis* porin (Msp), for example MspA, MspB, MspC or MspD, CsgG, outer membrane porin F (OmpF), outer membrane porin G (OmpG), outer membrane phospholipase A and *Neisseria* autotransporter lipoprotein (NalP) and other pores, such as lysenin. α-helix bundle pores comprise a barrel or channel that is formed from α-helices. Suitable α-helix bundle pores include, but are not limited to, inner membrane proteins and α outer membrane proteins, such as WZA and ClyA toxin.

In one embodiment the nanopore is a transmembrane pore derived from or based on Msp, α-hemolysin (α-HL), lysenin, CsgG, ClyA, Sp1 or haemolytic protein fragaceatoxin C (FraC).

In one embodiment, the nanopore is a transmembrane protein pore derived from CsgG, e.g. from CsgG from *E. coli* Str. K-12 substr. MC4100. Such a pore is oligomeric and typically comprises 7, 8, 9 or 10 monomers derived from CsgG. The pore may be a homo-oligomeric pore derived from CsgG comprising identical monomers. Alternatively, the pore may be a hetero-oligomeric pore derived from CsgG comprising at least one monomer that differs from the others. Examples of suitable pores derived from CsgG are disclosed in WO 2016/034591.

In one embodiment, the nanopore is a transmembrane pore derived from lysenin. Examples of suitable pores derived from lysenin are disclosed in WO 2013/153359.

In one embodiment, the nanopore is a transmembrane pore derived from or based on α-hemolysin (α-HL). The wild type α-hemolysin pore is formed of 7 identical monomers or sub-units (i.e., it is heptameric). An α-hemolysin pore may be α-hemolysin-NN or a variant thereof. The variant preferably comprises N residues at positions E111 and K147.

In one embodiment, the nanopore is a transmembrane protein pore derived from Msp, e.g. from MspA. Examples of suitable pores derived from MspA are disclosed in WO 2012/107778.

In one embodiment, the nanopore is a transmembrane pore derived from or based on ClyA.

### Membrane

In the disclosed methods, the nanopore is typically present in a membrane. Any suitable membrane may be used.

The membrane is preferably an amphiphilic layer. An amphiphilic layer is a layer formed from amphiphilic molecules, such as phospholipids, which have both hydrophilic and lipophilic properties. The amphiphilic molecules may be synthetic or naturally occurring. Non-naturally occurring amphiphiles and amphiphiles which form a monolayer are known in the art and include, for example, block copolymers (Gonzalez-Perez et al., Langmuir, 2009, 25, 10447-10450). Block copolymers are polymeric materials in which two or more monomer sub-units that are polymerized together to create a single polymer chain. Block copolymers typically have properties that are contributed by each monomer sub-unit. However, a block copolymer may have unique properties that polymers formed from the individual sub-units do not possess. Block copolymers can be engineered such that one of the monomer sub-units is hydrophobic (i.e. lipophilic), whilst the other sub-unit(s) are hydrophilic whilst in aqueous media. In this case, the block copolymer may possess amphiphilic properties and may form a structure that mimics a biological membrane. The block copolymer may be a diblock (consisting of two monomer sub-units), but may also be constructed from more than two monomer sub-units to form more complex arrangements that behave as amphipiles. The copolymer may be a triblock, tetrablock or pentablock copolymer. The membrane is preferably a triblock copolymer membrane.

Archaebacterial bipolar tetraether lipids are naturally occurring lipids that are constructed such that the lipid forms a monolayer membrane. These lipids are generally found in extremophiles that survive in harsh biological environments, thermophiles, halophiles and acidophiles. Their stability is believed to derive from the fused nature of the final bilayer. It is straightforward to construct block copolymer materials that mimic these biological entities by creating a triblock polymer that has the general motif hydrophilic-hydrophobic-hydrophilic. This material may form monomeric membranes that behave similarly to lipid bilayers and encompass a range of phase behaviours from vesicles through to laminar membranes. Membranes formed from these triblock copolymers hold several advantages over biological lipid membranes. Because the triblock copolymer is synthesised, the exact construction can be carefully controlled to provide the correct chain lengths and properties required to form membranes and to interact with pores and other proteins.

Block copolymers may also be constructed from sub-units that are not classed as lipid sub-materials; for example a hydrophobic polymer may be made from siloxane or other non-hydrocarbon based monomers. The hydrophilic sub-section of block copolymer can also possess low protein binding properties, which allows the creation of a membrane that is highly resistant when exposed to raw biological samples. This head group unit may also be derived from non-classical lipid head-groups.

Triblock copolymer membranes also have increased mechanical and environmental stability compared with biological lipid membranes, for example a much higher operational temperature or pH range. The synthetic nature of the block copolymers provides a platform to customise polymer based membranes for a wide range of applications.

In some embodiments, the membrane is one of the membranes disclosed in International Application No. WO2014/064443 or WO2014/064444.

The amphiphilic molecules may be chemically-modified or functionalised to facilitate coupling of the polynucleotide. The amphiphilic layer may be a monolayer or a bilayer. The amphiphilic layer is typically planar. The amphiphilic layer may be curved. The amphiphilic layer may be supported.

Amphiphilic membranes are typically naturally mobile, essentially acting as two dimensional fluids with lipid diffusion rates of approximately 10⁻⁸ cm s⁻¹. This means that the pore and coupled polynucleotide can typically move within an amphiphilic membrane.

The membrane may be a lipid bilayer. Lipid bilayers are models of cell membranes and serve as excellent platforms for a range of experimental studies. For example, lipid bilayers can be used for *in vitro* investigation of membrane proteins by single-channel recording. Alternatively, lipid bilayers can be used as biosensors to detect the presence of a range of substances. The lipid bilayer may be any lipid bilayer. Suitable lipid bilayers include, but are not limited to, a planar lipid bilayer, a supported bilayer or a liposome. The lipid bilayer is preferably a planar lipid bilayer. Suitable lipid bilayers are disclosed in WO 2008/102121, WO 2009/077734 and WO 2006/100484.

Methods for forming lipid bilayers are known in the art. Lipid bilayers are commonly formed by the method of Montal and Mueller (Proc. Natl. Acad. Sci. USA., 1972; 69: 3561-3566), in which a lipid monolayer is carried on aqueous solution/air interface past either side of an aperture which is perpendicular to that interface. The lipid is normally added to the surface of an aqueous electrolyte solution by first dissolving it in an organic solvent and then allowing a drop of the solvent to evaporate on the surface of the aqueous solution on either side of the aperture. Once the organic solvent has evaporated, the solution/air interfaces on either side of the aperture are physically moved up and down past the aperture until a bilayer is formed. Planar lipid bilayers may be formed across an aperture in a membrane or across an opening into a recess.

The method of Montal & Mueller is popular because it is a cost-effective and relatively straightforward method of forming good quality lipid bilayers that are suitable for protein pore insertion. Other common methods of bilayer formation include tip-dipping, painting bilayers and patch-clamping of liposome bilayers.

Tip-dipping bilayer formation entails touching the aperture surface (for example, a pipette tip) onto the surface of a test solution that is carrying a monolayer of lipid. Again, the lipid monolayer is first generated at the solution/air interface by allowing a drop of lipid dissolved in organic solvent to evaporate at the solution surface. The bilayer is then formed by the Langmuir-Schaefer process and requires mechanical automation to move the aperture relative to the solution surface.

For painted bilayers, a drop of lipid dissolved in organic solvent is applied directly to the aperture, which is submerged in an aqueous test solution. The lipid solution is spread thinly over the aperture using a paintbrush or an equivalent. Thinning of the solvent results in formation of a lipid bilayer. However, complete removal of the solvent from the bilayer is difficult and consequently the bilayer formed by this method is less stable and more prone to noise during electrochemical measurement.

Patch-clamping is commonly used in the study of biological cell membranes. The cell membrane is clamped to the end of a pipette by suction and a patch of the membrane becomes attached over the aperture. The method has been adapted for producing lipid bilayers by clamping liposomes which then burst to leave a lipid bilayer sealing over the aperture of the pipette. The method requires stable, giant and unilamellar liposomes and the fabrication of small apertures in materials having a glass surface.

Liposomes can be formed by sonication, extrusion or the Mozafari method (Colas et al. (2007) Micron 38:841-847).

In some embodiments, a lipid bilayer is formed as described in International Application No. WO 2009/077734. Advantageously in this method, the lipid bilayer is formed from dried lipids. In a most preferred embodiment, the lipid bilayer is formed across an opening as described in WO2009/077734.

A lipid bilayer is formed from two opposing layers of lipids. The two layers of lipids are arranged such that their hydrophobic tail groups face towards each other to form a hydrophobic interior. The hydrophilic head groups of the lipids face outwards towards the aqueous environment on each side of the bilayer. The bilayer may be present in a number of lipid phases including, but not limited to, the liquid disordered phase (fluid lamellar), liquid ordered phase, solid ordered phase (lamellar gel phase, interdigitated gel phase) and planar bilayer crystals (lamellar sub-gel phase, lamellar crystalline phase).

Any lipid composition that forms a lipid bilayer may be used. The lipid composition is chosen such that a lipid bilayer having the required properties, such surface charge, ability to support membrane proteins, packing density or mechanical properties, is formed. The lipid composition can comprise one or more different lipids. For instance, the lipid composition can contain up to 100 lipids. The lipid composition preferably contains 1 to 10 lipids. The lipid composition may comprise naturally-occurring lipids and/or artificial lipids.

The lipids typically comprise a head group, an interfacial moiety and two hydrophobic tail groups which may be the same or different. Suitable head groups include, but are not limited to, neutral head groups, such as diacylglycerides (DG) and ceramides (CM); zwitterionic head groups, such as phosphatidylcholine (PC), phosphatidylethanolamine (PE) and sphingomyelin (SM); negatively charged head groups, such as phosphatidylglycerol (PG); phosphatidylserine (PS), phosphatidylinositol (PI), phosphatic acid (PA) and cardiolipin (CA); and positively charged headgroups, such as trimethylammonium-Propane (TAP). Suitable interfacial moieties include, but are not limited to, naturally-occurring interfacial moieties, such as glycerol-based or ceramide-based moieties. Suitable hydrophobic tail groups include, but are not limited to, saturated hydrocarbon chains, such as lauric acid (*n*-Dodecanolic acid), myristic acid (*n-*Tetradecononic acid), palmitic acid (*n*-Hexadecanoic acid), stearic acid (*n*-Octadecanoic) and arachidic (*n*-Eicosanoic); unsaturated hydrocarbon chains, such as oleic acid (*cis-*9-Octadecanoic); and branched hydrocarbon chains, such as phytanoyl. The length of the chain and the position and number of the double bonds in the unsaturated hydrocarbon chains can vary. The length of the chains and the position and number of the branches, such as methyl groups, in the branched hydrocarbon chains can vary. The hydrophobic tail groups can be linked to the interfacial moiety as an ether or an ester. The lipids may be mycolic acid.

The lipids can also be chemically-modified. The head group or the tail group of the lipids may be chemically-modified. Suitable lipids whose head groups have been chemically-modified include, but are not limited to, PEG-modified lipids, such as 1,2-Diacyl-sn-Glycero-3-Phosphoethanolamine-N-[Methoxy(Polyethylene glycol)-2000]; functionalised PEG Lipids, such as 1,2-Distearoyl-sn-Glycero-3 Phosphoethanolamine-N-[Biotinyl(Polyethylene Glycol)2000]; and lipids modified for conjugation, such as 1,2-Dioleoyl-sn-Glycero-3-Phosphoethanolamine-N-(succinyl) and 1,2-Dipalmitoyl-sn-Glycero-3-Phosphoethanolamine-N-(Biotinyl). Suitable lipids whose tail groups have been chemically-modified include, but are not limited to, polymerisable lipids, such as 1,2-bis(10,12-tricosadiynoyl)-sn-Glycero-3-Phosphocholine; fluorinated lipids, such as 1-Palmitoyl-2-(16-Fluoropalmitoyl)-sn-Glycero-3-Phosphocholine; deuterated lipids, such as 1,2-Dipalmitoyl-D62-sn-Glycero-3-Phosphocholine; and ether linked lipids, such as 1,2-Di-O-phytanyl-sn-Glycero-3-Phosphocholine. The lipids may be chemically-modified or functionalised to facilitate coupling of the polynucleotide.

The amphiphilic layer, for example the lipid composition, typically comprises one or more additives that will affect the properties of the layer. Suitable additives include, but are not limited to, fatty acids, such as palmitic acid, myristic acid and oleic acid; fatty alcohols, such as palmitic alcohol, myristic alcohol and oleic alcohol; sterols, such as cholesterol, ergosterol, lanosterol, sitosterol and stigmasterol; lysophospholipids, such as 1-Acyl-2-Hydroxy-sn- Glycero-3-Phosphocholine; and ceramides.

In another embodiment, the membrane comprises a solid state layer. Solid state layers can be formed from both organic and inorganic materials including, but not limited to, microelectronic materials, insulating materials such as Si₃N₄, Al₂O₃, and SiO, organic and inorganic polymers such as polyamide, plastics such as Teflon^{®} or elastomers such as two-component addition-cure silicone rubber, and glasses. The solid state layer may be formed from graphene. Suitable graphene layers are disclosed in WO 2009/035647. If the membrane comprises a solid state layer, the pore is typically present in an amphiphilic membrane or layer contained within the solid state layer, for instance within a hole, well, gap, channel, trench or slit within the solid state layer. The skilled person can prepare suitable solid state/amphiphilic hybrid systems. Suitable systems are disclosed in WO 2009/020682 and WO 2012/005857. Any of the amphiphilic membranes or layers discussed above may be used.

The methods disclosed herein are typically carried out using (i) an artificial amphiphilic layer comprising a pore, (ii) an isolated, naturally-occurring lipid bilayer comprising a pore, or (iii) a cell having a pore inserted therein. The methods are typically carried out using an artificial amphiphilic layer, such as an artificial triblock copolymer layer. The layer may comprise other transmembrane and/or intramembrane proteins as well as other molecules in addition to the pore. Suitable apparatus and conditions are discussed below. The methods provided herein are typically carried out *in vitro.*

### Anchor

In one embodiment, the polynucleotide or polynucleotide adapter comprises a membrane anchor or a transmembrane pore anchor e.g. attached to the adapter. In one embodiment the anchor aids in characterisation of a target polynucleotide in accordance with the methods disclosed herein. For example, a membrane anchor or transmembrane pore anchor may promote localisation of the selected polynucleotides around the transmembrane pore.

The anchor may be a polypeptide anchor and/or a hydrophobic anchor that can be inserted into the membrane. In one embodiment, the hydrophobic anchor is a lipid, fatty acid, sterol, carbon nanotube, polypeptide, protein or amino acid, for example cholesterol, palmitate or tocopherol. The anchor may comprise thiol, biotin or a surfactant.
In one aspect the anchor may be biotin (for binding to streptavidin), amylose (for binding to maltose binding protein or a fusion protein), Ni-NTA (for binding to poly-histidine or poly-histidine tagged proteins) or peptides (such as an antigen).

In one embodiment, the anchor comprises a linker, or 2, 3, 4 or more linkers. Preferred linkers include, but are not limited to, polymers, such as polynucleotides, polyethylene glycols (PEGs), polysaccharides and polypeptides. These linkers may be linear, branched or circular. For instance, the linker may be a circular polynucleotide. The adapter may hybridise to a complementary sequence on a circular polynucleotide linker. The one or more anchors or one or more linkers may comprise a component that can be cut or broken down, such as a restriction site or a photolabile group. The linker may be functionalised with maleimide groups to attach to cysteine residues in proteins. Suitable linkers are described in WO 2010/086602.

In one embodiment, the anchor is cholesterol or a fatty acyl chain. For example, any fatty acyl chain having a length of from 6 to 30 carbon atom, such as hexadecanoic acid, may be used. Examples of suitable anchors and methods of attaching anchors to adapters are disclosed in WO 2012/164270 and WO 2015/150786.

In another embodiment the anchor may consist or comprise a hydrophobic modification to the polynucleotide or polynucleotide adapter. The hydrophobic modification may comprise a modified phosphate group comprised within the polynucleotide or polynucleotide anchor. The hydrophobic modification may for example comprise a phosphorothioate such as a charge-neutralized alkyl-phosphorothioate (PPT) as described in Jones et al, J. Am. Chem. Soc. 2021, 143, 22, 8305. Suitable alkyl groups include for example C₁-C₁₀ alkyl groups such as C₂-C₆ alkyl groups; e.g. methyl, ethyl, propyl, butyl, pentyl and hexyl groups. Incorporation of the charge-neutralized alkyl-phosphorothioate into a polynucleotide allows for the polynucleotide to anchor to a hydrophobic region such as a lipid bilayer.

### Characterisation methods

As discussed above, the methods provided herein relate to selectively characterising polynucleotides having one or more desired properties. For example, the method may involve characterising a polynucleotide of a desired length.

The characterisation methods may be carried out using any apparatus that is suitable for investigating a membrane/pore system in which a pore is inserted into a membrane. The characterisation method may be carried out using any apparatus that is suitable for transmembrane pore sensing. For example, the apparatus may comprise a chamber comprising an aqueous solution and a barrier that separates the chamber into two sections. The barrier may have an aperture in which a membrane containing a transmembrane pore is formed. Transmembrane pores are described herein.

The characterisation methods may be carried out using the apparatus described in WO 2008/102120, WO 2010/122293 or WO 00/28312.

The characterisation methods may involve measuring the ion current flow through the pore, typically by measurement of a current. Alternatively, the ion flow through the pore may be measured optically, e.g. such as disclosed by Heron et al: J. Am. Chem. Soc. 9 Vol. 131, No. 5, 2009. Therefore the apparatus may also comprise an electrical circuit capable of applying a potential and measuring an electrical signal across the membrane and pore. The characterisation methods may be carried out using a patch clamp or a voltage clamp. The characterisation methods preferably involve the use of a voltage clamp.

The methods may involve measuring an optical signal as described in Chen et al, Nature Communications (2018)9:1733. For example, a nanopore such as an optically engineered nanopore structure (e.g. a plasmonic nanoslit) may be used to locally enable single-molecule surface enhanced Raman spectroscopy (SERS) to allow the characterisation of the polynucleotide through direct Raman spectroscopic detection.

The characterisation methods may be carried out on a silicon-based array of wells where each array comprises 128, 256, 512, 1024, 2000, 3000, 4000, 6000, 10000, 12000, 15000 or more wells.

The characterisation methods may involve the measuring of a current flowing through the pore. The method is typically carried out with a voltage applied across the membrane and pore. The voltage used is typically from +2 V to -2 V, typically -400 mV to +400mV. The voltage used is preferably in a range having a lower limit selected from -400 mV, -300 mV, -200 mV, -150 mV, -100 mV, -50 mV, -20mV and 0 mV and an upper limit independently selected from +10 mV, + 20 mV, +50 mV, +100 mV, +150 mV, +200 mV, +300 mV and +400 mV. The voltage used is more preferably in the range 100 mV to 240mV and most preferably in the range of 120 mV to 220 mV. It is possible to increase discrimination between different nucleotides by a pore by using an increased applied potential.

The characterisation methods are typically carried out in the presence of any charge carriers, such as metal salts, for example alkali metal salts, halide salts, for example chloride salts, such as alkali metal chloride salt. Charge carriers may include ionic liquids or organic salts, for example tetramethyl ammonium chloride, trimethylphenyl ammonium chloride, phenyltrimethyl ammonium chloride, or 1-ethyl-3-methyl imidazolium chloride. In the exemplary apparatus discussed above, the salt is present in the aqueous solution in the chamber. Potassium chloride (KCl), sodium chloride (NaCl) or caesium chloride (CsCl) is typically used. KCl is preferred. The salt may be an alkaline earth metal salt such as calcium chloride (CaCl2). The salt concentration may be at saturation. The salt concentration may be 3M or lower and is typically from 0.1 to 2.5 M, from 0.3 to 1.9 M, from 0.5 to 1.8 M, from 0.7 to 1.7 M, from 0.9 to 1.6 M or from 1 M to 1.4 M. The salt concentration is preferably from 150 mM to 1 M. The characterisation method is preferably carried out using a salt concentration of at least 0.3 M, such as at least 0.4 M, at least 0.5 M, at least 0.6 M, at least 0.8 M, at least 1.0 M, at least 1.5 M, at least 2.0 M, at least 2.5 M or at least 3.0 M. High salt concentrations provide a high signal to noise ratio and allow for currents indicative of binding/no binding to be identified against the background of normal current fluctuations.

The characterisation methods are typically carried out in the presence of a buffer. In the exemplary apparatus discussed above, the buffer is present in the aqueous solution in the chamber. Any suitable buffer may be used. Typically, the buffer is HEPES. Another suitable buffer is Tris-HCl buffer. The methods are typically carried out at a pH of from 4.0 to 12.0, from 4.5 to 10.0, from 5.0 to 9.0, from 5.5 to 8.8, from 6.0 to 8.7 or from 7.0 to 8.8 or 7.5 to 8.5. The pH used is preferably about 7.5.

The characterisation methods may be carried out at from 0 °C to 100 °C, from 15 °C to 95 °C, from 16 °C to 90 °C, from 17 °C to 85 °C, from 18 °C to 80 °C, 19 °C to 70 °C, or from 20 °C to 60 °C. The characterisation methods are typically carried out at room temperature. The characterisation methods are optionally carried out at a temperature that supports enzyme function, such as about 37 °C.

### Construct

As discussed above, the movement of a polynucleotide (e.g. the movement of the second part of the target polynucleotide) may be controlled using a polynucleotide binding protein such as a motor protein described herein. The polynucleotide binding protein may be stalled on the polynucleotide. For example, as described herein, when the target polynucleotide is a double-stranded polynucleotide the two strands of the double-stranded polynucleotide may be linked by a hairpin or hairpin adapter, and the polynucleotide binding protein may be stalled on the hairpin or hairpin adapter.

It is to be understood that although particular embodiments, specific configurations as well as materials and/or molecules, have been discussed herein for methods according to the present invention, various changes or modifications in form and detail may be made without departing from the scope of this invention. The following examples are provided to better illustrate particular embodiments, and they should not be considered limiting the application. The application is limited only by the claims.

### Examples

### Example 1

This example describes a method of measuring a polynucleotide freely translocating through the nanopore. The time taken for the polynucleotide to freely translocate through or across the pore is measured. A decision is made whether to reject the polynucleotide based on the time taken for the polynucleotide to freely translocate through or across the pore. If accepted, the polynucleotide is sequenced by measuring the current through the nanopore as the polynucleotide binding protein controls the movement of the polynucleotide out of the nanopore. If rejected, the polynucleotide is ejected from the nanopore.

### Method

A double stranded DNA analyte is subjected to NEBNext end repair and NEBNext dA-tailing modules (New England Biolabs (NEB)), to generate 3' A overhangs.

A Y adapter is prepared by annealing DNA. A polynucleotide binding protein is loaded onto the annealed Y adapter DNA. The Y adapter contains a leader that is captured by the nanopore and a 3' T to enable ligation to the DNA analyte. The Y adapter contains a binding site and a stalling chemistry for the polynucleotide binding protein. The protein bound Y adapter is purified on an anion exchange column.

The double stranded DNA analyte is ligated to the Y adapter using LNB from Oxford Nanopore Technologies sequencing kit (SQK-LSK109; see https://community.nanoporetech.com/protocols/gDNA-sqk-lsk109/v/gde_9063_v109_revt_14aug2019 for details) and T4 DNA Ligase (NEB). The sample is purified using Agencourt AMPure XP (Beckman Coulter) beads, with two washes with LFB from Oxford Nanopore Technologies sequencing kit (SQK-LSK109). The ligated substrate is eluted into EB from Oxford Nanopore Technologies sequencing kit (SQK-LSK109), this will be referred to as the DNA library.

Electrical measurements are acquired on a FLO-MIN106 MinION flowcell and MinION Mk1b from Oxford Nanopore Technologies. The raw data is collected using MinKNOW software (Oxford Nanopore Technologies).

The duration of the free translocation of the first part of the polynucleotide is calculated by measuring the duration of the signal after the drop from the open pore level when the first strand is captured by the nanopore and before a current threshold that is induced when the Y-adapter ligated to the distal end of the polynucleotide is reached.

The MinKNOW software uses the duration of the freely translocating polynucleotide to decide whether to reject the second strand. The second strand is ejected by reversing the applied voltage. In this example, strands of a desired length having free translocation times above a predetermined threshold duration are accepted. If the MinKNOW software decides to accept the strand, the movement of the second strand of the polynucleotide with respect to the nanopore is controlled by the polynucleotide binding protein and the second strand is characterised, e.g. by being sequenced.

Compared to the control for which no duration rejection criteria are set in MinKNOW, the N50 of the sequence length distribution is greater.

### Example 2

This example describes a method of measuring the first strand of a double stranded polynucleotide freely translocating through the nanopore. The method is shown schematically in Figure 3.

A sequence is revealed in the second strand of the double stranded polynucleotide as the first strand translocates through the nanopore. This sequence hybridises to a complementary oligonucleotide chemically attached to the nanopore, localising the second strand to the nanopore. The sequencing adapter from the second strand is captured by the nanopore. The time taken for the first strand to freely translocate through or across the pore is measured. A decision is made whether to reject the second strand of the polynucleotide based on the time taken for the first strand of the polynucleotide to freely translocate through or across the pore. If accepted, the movement of the second strand of the polynucleotide is controlled by a polynucleotide binding protein. If rejected, the second strand is ejected from the nanopore.

### Method

A double stranded DNA analyte is subjected to NEBNext end repair and NEBNext dA-tailing modules (New England Biolabs (NEB)), to generate 3' A overhangs. The double stranded DNA analyte is ligated to a 1D^2 adapter from Oxford Nanopore Technologies sequencing kit (SQK-LSK308) using LNB from Oxford Nanopore Technologies sequencing kit (SQK-LSK109) and T4 DNA Ligase (NEB). The sample is purified using Agencourt AMPure XP (Beckman Coulter) beads, with two washes with LFB from Oxford Nanopore Technologies sequencing kit (SQK-LSK109). The ligated substrate is eluted into EB from Oxford Nanopore Technologies sequencing kit (SQK-LSK109), this will be referred to as the 1D^2 DNA analyte.

A Y adapter is prepared by annealing DNA. The Y adapter contains a leader that is captured by the nanopore and an overhang to enable ligation to the 1D^2 DNA analyte. The Y adapter may contain a tether to promote capture of the Y adapter by the nanopore. A sequencing adapter is prepared by annealing DNA. A polynucleotide binding protein is loaded onto the annealed sequencing adapter DNA. The sequencing adapter contains a leader that is captured by the nanopore and an overhang to enable ligation to the 1D^2 DNA analyte. The sequencing adapter contains a binding site and a stalling chemistry for the polynucleotide binding protein. The protein bound sequencing adapter is purified on an anion exchange column.

The 1D^2 DNA analyte is ligated to the Y adapter and the sequencing adapter using LNB from Oxford Nanopore Technologies sequencing kit (SQK-LSK109) and T4 DNA Ligase (NEB). The sample is purified using Agencourt AMPure XP (Beckman Coulter) beads, with two washes with LFB from Oxford Nanopore Technologies sequencing kit (SQK-LSK109). The ligated substrate is eluted into EB from Oxford Nanopore Technologies sequencing kit (SQK-LSK109), this will be referred to as the DNA library. Electrical measurements are acquired on a FLO-MIN107 MinION flowcell and MinION Mk1b from Oxford Nanopore Technologies. The raw data is collected using MinKNOW software (Oxford Nanopore Technologies).

The duration of the free translocation of the first part of the polynucleotide is calculated by measuring the duration of the signal after the drop from the open pore level when the first strand is captured by the nanopore and before a current threshold that is induced (a) when the open pore level is reached again; (b) by a signal in the sequencing adapter ligated to the distal end of the first strand of the polynucleotide; and/or (c) by a signal from the capture of the second strand.

The MinKNOW software uses the duration of the freely translocating polynucleotide to decide whether to reject the second strand. The second strand is ejected by reversing the applied voltage. In this example, strands of a desired length having free translocation times above a predetermined threshold duration are accepted. If the MinKNOW software decides to accept the strand, the movement of the second strand of the polynucleotide with respect to the nanopore is controlled by the polynucleotide binding protein and the second strand is characterised, e.g. by being sequenced.

Compared to the control for which no duration rejection criteria are set in MinKNOW, the N50 of the sequence length distribution (ie the median strand length) is greater.

### Example 3

This example describes a method of measuring the first strand of an asymmetric double stranded polynucleotide freely translocating through the nanopore. The method is shown schematically in Figure 5.

The first strand of the polynucleotide freely translocates through the nanopore. The nanopore separates the duplex. A stall such as a polynucleotide binding protein, a G-quadruplex (TBA), a BNA / LNA stall, a spacer such as a C3 or Sp18 spacer, or biotin/desthiobiotin attached to streptavidin or monovalent streptavidin is provided at the distal end of the freely translocating polynucleotide, creating a pause and a signal. A sequence is revealed on the second strand of the double stranded polynucleotide, this sequence hybridises to a complementary oligonucleotide chemically attached to the nanopore, whilst the first strand is paused. The first strand fully translocates through the nanopore. The time taken for the first strand to freely translocate through or across the pore is measured. A decision is made whether to reject the second strand of the polynucleotide based on the time taken for the first strand of the polynucleotide to freely translocate through or across the pore. If accepted, the movement of the second strand of the polynucleotide is controlled by a polynucleotide binding protein. If rejected, the second strand is ejected from the nanopore.

### Method

A double stranded DNA analyte is subjected to NEBNext end repair and NEBNext dA-tailing modules (New England Biolabs (NEB)), to generate 3' A overhangs. The double stranded DNA analyte is ligated to a 1D^2 adapter from Oxford Nanopore Technologies sequencing kit (SQK-LSK308) using LNB from Oxford Nanopore Technologies sequencing kit (SQK-LSK109) and T4 DNA Ligase (NEB). The sample is purified using Agencourt AMPure XP (Beckman Coulter) beads, with two washes with LFB from Oxford Nanopore Technologies sequencing kit (SQK-LSK109). The ligated substrate is eluted into EB from Oxford Nanopore Technologies sequencing kit (SQK-LSK109), this will be referred to as the 1D^2 DNA analyte.

A Y adapter is prepared by annealing DNA. The Y adapter contains a leader that is captured by the nanopore and an overhang to enable ligation to the 1D^2 DNA analyte. The Y adapter may contain a tether to promote capture of the Y adapter by the nanopore. A sequencing adapter is prepared by annealing DNA. The sequencing adapter contains a leader that is captured by the nanopore and an overhang to enable ligation to the 1D^2 DNA analyte. The sequencing adapter contains a binding site and a stalling chemistry for the (first) polynucleotide binding protein. The sequencing adapter contains a stall selected from
a. Binding site and stalling chemistry for a second polynucleotide binding protein on the opposite strand to which the first polynucleotide binding protein is bound
b. G-quadruplex (TBA) on the opposite strand to which the polynucleotide binding protein is bound
c. BNA / LNA stall on the opposite strand to which the polynucleotide binding protein is bound
d. C3 or Sp18 stall on the opposite strand to which the polynucleotide binding protein is bound
e. Biotin or desthiobiotin stall on the opposite strand to which the polynucleotide binding protein is bound

A polynucleotide binding protein is loaded onto the annealed sequencing adapter DNA. The protein bound sequencing adapter is purified on an anion exchange column. The 1D^2 DNA analyte is ligated to the Y adapter and the sequencing adapter using LNB from Oxford Nanopore Technologies sequencing kit (SQK-LSK109) and T4 DNA Ligase (NEB). The sample is purified using Agencourt AMPure XP (Beckman Coulter) beads, with two washes with LFB from Oxford Nanopore Technologies sequencing kit (SQK-LSK109). The ligated substrate is eluted into EB from Oxford Nanopore Technologies sequencing kit (SQK-LSK109), this will be referred to as the DNA library.

Electrical measurements are acquired on a FLO-MIN107 MinION flowcell and MinION Mk1b from Oxford Nanopore Technologies. The raw data is collected using MinKNOW software (Oxford Nanopore Technologies).

The duration of the free translocation of the first part of the polynucleotide is calculated by measuring the duration of the signal after the drop from the open pore level when the first strand is captured by the nanopore and before a current threshold that is induced (a) when the open pore level is reached again; (b) by a signal in the sequencing adapter ligated to the distal end of the first strand of the polynucleotide; and/or (c) by a signal from the capture of the second strand.

The MinKNOW software uses the duration of the freely translocating polynucleotide to decide whether to reject the second strand. The second strand can be ejected by reversing the applied voltage. In this example, strands of a desired length having free translocation times above a predetermined threshold are accepted. If the MinKNOW software decides to accept the strand, the movement of the second strand of the polynucleotide with respect to the nanopore is controlled by the polynucleotide binding protein and the second strand is characterised, e.g. by being sequenced.

Compared to the control for which no duration rejection criteria are set in MinKNOW, the N50 of the sequence length distribution is greater.

### Example 4

This example describes a method of measuring the first strand of an asymmetric double stranded polynucleotide freely translocating through the nanopore. The method is shown schematically in Figure 6.

A sequence on the Y adapter hybridises to a complementary oligonucleotide chemically attached to the nanopore, keeping the second strand localised to the nanopore. The leader of the first strand is captured by the nanopore. The first strand freely translocates through the nanopore. The nanopore separates the duplex, revealing a leader for capture of the second strand. The first strand fully translocates through the nanopore. The time taken for the first strand to freely translocate through or across the pore is measured. The adapter of the second strand of the polynucleotide is captured by the nanopore. A decision is made whether to reject the second strand of the polynucleotide based on the time taken for the first strand of the polynucleotide to freely translocate through or across the pore. If accepted, the movement of the second strand of the polynucleotide is controlled by a polynucleotide binding protein. If rejected, the second strand is ejected from the nanopore.

### Method

A double stranded DNA analyte is subjected to NEBNext end repair and NEBNext dA-tailing modules (New England Biolabs (NEB)), to generate 3' A overhangs, this will be referred to as the DNA analyte.

A Y adapter is prepared by annealing DNA. The Y adapter contains a leader that is captured by the nanopore and an overhang to enable ligation to the DNA analyte. The Y adapter contains a binding site for hybridisation to an oligonucleotide chemically attached to the nanopore. The sequencing adapter contains a binding site and a stalling chemistry for the polynucleotide binding protein. A polynucleotide binding protein is loaded onto the annealed Y adapter DNA. The protein bound sequencing adapter is purified on an anion exchange column.

A distal end adapter is prepared by annealing DNA. The adapter contains an overhang to enable ligation to the DNA analyte. Optionally, a stall such as a polynucleotide binding protein, a G-quadruplex (TBA), a BNA / LNA stall, a spacer such as a C3 or Sp18 spacer, or biotin/desthiobiotin attached to streptavidin or monovalent streptavidin may be present in the distal end adapter.

The DNA analyte is ligated to the Y adapter and the distal end adapter using LNB from Oxford Nanopore Technologies sequencing kit (SQK-LSK109) and T4 DNA Ligase (NEB). The sample is purified using Agencourt AMPure XP (Beckman Coulter) beads, with two washes with LFB from Oxford Nanopore Technologies sequencing kit (SQK-LSK109). The ligated substrate is eluted into EB from Oxford Nanopore Technologies sequencing kit (SQK-LSK109), this will be referred to as the DNA library.

Electrical measurements are acquired on a FLO-MIN107 MinION flowcell and MinION Mk1b from Oxford Nanopore Technologies. The raw data is collected using MinKNOW software (Oxford Nanopore Technologies).

The duration of the free translocation of the first part of the polynucleotide is calculated by measuring the duration of the signal after the drop from the open pore level when the first strand is captured by the nanopore and before a current threshold that is induced (a) when the open pore level is reached again; (b) by a signal in the distal end adapter; and/or (c) by a signal from the capture of the second strand.

The MinKNOW software uses the duration of the freely translocating polynucleotide to decide whether to reject the second strand. The second strand can be ejected by reversing the applied voltage. In this example, strands of a desired length having free translocation times above a predetermined threshold w are accepted.

If the MinKNOW software decides to accept the strand, the movement of the second strand of the polynucleotide with respect to the nanopore is controlled by the polynucleotide binding protein and the second strand is characterised, e.g. by being sequenced.

Compared to the control for which no duration rejection criteria are set in MinKNOW, the N50 of the sequence length distribution is greater.

### Example 5

This example describes a method of measuring the time duration taken for a first part of a polynucleotide to freely translocate through a nanopore, and controlling the movement of the second part of the polynucleotide with a polynucleotide binding protein bound to a hairpin adapter. The time taken for the first part of the double stranded polynucleotide to translocate through or across the pore is measured. The length of the second part of the polynucleotide is measured. A correlation between time taken for the first part of the double stranded polynucleotide to translocate through or across the pore and the length of the second part of the polynucleotide is observed.

### Materials and methods

### Hairpin Adapter

A hairpin adapter was made by annealing SEQ ID NO: 11 with SEQ ID NO: 12. A polynucleotide binding protein (a Dda helicase) was loaded onto the hairpin adapter. The protein bound hairpin adapter was purified on an anion exchange column.

### 3.6 kb Analyte Preparation

A double stranded 3.6 kb DNA analyte (SEQ ID NO: 13) was prepared using specific primers and PCR. The PCR product was subjected to NEBNext end repair, NEBNext dA-tailing modules and USER^{®} Enzyme (New England Biolabs (NEB)), to generate a 3' A overhang and a 3' AGGA overhang.

### 10 kb Analyte Preparation

A double stranded 10 kb DNA analyte (SEQ ID NO: 14) was prepared using specific primers and PCR. The PCR product was subjected to NEBNext end repair, NEBNext dA-tailing modules and USER^{®} Enzyme (New England Biolabs (NEB)), to generate a 3' A overhang and a 3' AGGA overhang.

### Y Adapter Preparation

A Y adapter was prepared by annealing SEQ ID NO: 15 with SEQ ID NO: 16

### Ligation of Y adapter and hairpin adapter to 3.6 kb analyte

2 µg of 3.6 kb analyte was ligated to 30 nM hairpin adapter and 50 nM Y adapter in a 100 µL volume using LNB from Oxford Nanopore Technologies sequencing kit (SQK-LSK109) and T4 DNA Ligase (NEB). The sample was purified using Agencourt AMPure XP (Beckman Coulter) beads. With two washes with ABB from Oxford Nanopore Technologies sequencing kit (SQK-LSK108). The ligated substrate was eluted into ELB from Oxford Nanopore Technologies sequencing kit (SQK-LSK108), this will be referred to as the 3.6 kb library.

### Ligation of Y adapter and hairpin adapter to 10 kb analyte

2 µg of 10 kb analyte was ligated to 30 nM hairpin adapter and 50 nM Y adapter in a 100 µL volume using LNB from Oxford Nanopore Technologies sequencing kit (SQK-LSK109) and T4 DNA Ligase (NEB). The sample was purified using Agencourt AMPure XP (Beckman Coulter) beads. With two washes with ABB from Oxford Nanopore Technologies sequencing kit (SQK-LSK108). The ligated substrate was eluted into ELB from Oxford Nanopore Technologies sequencing kit (SQK-LSK108), this will be referred to as the 10 kb library.

### Electrical Measurements

Electrical measurements were acquired on a FLO-MIN107 MinION flowcell and MinION Mk1b from Oxford Nanopore Technologies. 500 µL of RBF from Oxford Nanopore Technologies sequencing kit (SQK-LSK 108) prepared with nuclease free water (Ambion^{™}) was flowed through the system, 5 minute wait, then 300 µL of RBF was flowed through the system 5 minute wait, then 200 µL of RBF1 was flowed through the system. 37.5 µL RBF from Oxford Nanopore Technologies sequencing kit (SQK-LSK108), 12.5 µL of the recovered bead purified Library and 25.5 µL of LLB (Library Loading Beads) from Oxford Nanopore Technologies sequencing kit (SQK-LSK108) were mixed. 75 µL of the 3.6 kb sequencing mix was added to a MinION flowcell, using the SpotOn Flowcell Port. 75 µL of the 10 kb sequencing mix was added to a MinION flowcell, using the SpotOn Flowcell Port The raw data was collected using MinKNOW software (Oxford Nanopore Technologies) at -180 mV and 4000 kHz acquisition frequency.

### Data Analysis

The second part of the polynucleotide with movement controlled with a polynucleotide binding protein was sequenced using a basecalling algorithm in Guppy v3.1.5 (Oxford Nanopore Technologies). The basecalled sequence of the second part of the polynucleotide was aligned to a reference using minimap2 (https://github.com/lh3/minimap2, version 2.14-r883) to calculate the length of the second part of the polynucleotide in base pairs (bp).

### Results

The duration of the free translocation of the first part of the polynucleotide was calculated by taking the duration of the signal after the drop from the open pore level and before a 120 pA current threshold was crossed (Figure 12). The first 10 datapoints were removed from the measurement to avoid any artefacts arising from signal from the drop from open pore level to the freely translocating polynucleotide level. The length of the first part of the polynucleotide determined the time duration of the free translocation of the first part of the polynucleotide as shown in the table below.

The second part of the polynucleotide was characterised by sequencing during its translocation and the alignment length is shown in the table below. A correlation between time taken for the first part of the double stranded polynucleotide to translocate through or across the pore and the length of the second part of the polynucleotide was observed.

| input | alignment length (bp) | First part duration (ms) |
|---|---|---|
| 3.6kb | 3435 | 11.75 |
| 10kb | 6707 | 39.75 |

### Example 6

This example describes a method of measuring the first strand of a double stranded polynucleotide freely translocating through the nanopore.

A double stranded polynucleotide is contacted with an ultra-fast polynucleotide handling enzyme which enables the free translocation of the first strand of the double-stranded polynucleotide through or across the pore. A decision is made whether to reject the second strand of the polynucleotide based on the time taken for the first strand of the polynucleotide to freely translocate through or across the pore. If accepted, the movement of the second strand of the polynucleotide is controlled by a polynucleotide binding protein. If rejected, the second strand is ejected from the nanopore.

### Method

A double stranded DNA analyte is prepared as described in the previous examples.

The double stranded DNA analyte is contacted with an ultra-fast polynucleotide-handling enzyme such as a FtsK or SpoIIIE translocase; phi29 packaging motor; or AddAB or RecBCD helicase / helicase-nuclease.

The first strand of the double-stranded polynucleotide is allowed to translocate freely through a nanopore. Electrical measurements are acquired on a FLO-MIN107 MinION flowcell and MinION Mk1b from Oxford Nanopore Technologies. The raw data is collected using MinKNOW software (Oxford Nanopore Technologies).

The duration of the free translocation of the first part of the polynucleotide under the control of the ultra-fast polynucleotide-handling enzyme is calculated by measuring the duration of the signal after the drop from the open pore level when the first strand is captured by the nanopore and before a current threshold that is induced (a) when the open pore level is reached again; (b) by a signal in a sequencing adapter ligated to the distal end of the first strand of the polynucleotide; and/or (c) by a signal from the capture of the second strand.

The MinKNOW software uses the duration of the freely translocating first strand of the polynucleotide to decide whether to reject the second strand. The second strand may be ejected by reversing the applied voltage. In this example, strands of a desired length having free translocation times above a predetermined threshold duration are accepted. If the MinKNOW software decides to accept the strand, the movement of the second strand of the polynucleotide with respect to the nanopore is controlled by a polynucleotide binding protein and the second strand is characterised, e.g. by being sequenced.

Compared to the control for which no duration rejection criteria are set in MinKNOW, the N50 of the sequence length distribution (ie the median strand length) is greater.

### Example 7

This example demonstrates how the duration of the signal from the initial, enzyme-free portion of DNA translocation (3'-5') through a nanopore may be used to estimate the size of a double-stranded DNA molecule whose template and complement strands are joined by a hairpin moiety, before a 5'-3' DNA motor on the distal end actively translocates the DNA strand out of the nanopore in the opposite direction. Additionally, this example shows how markers added to the hairpin may be used to demarcate the signal.

The DNA motor was initially stalled on a Y-adapter ligated to the polynucleotide. The template and complement strands were linked together via a hairpin moiety. Optionally, the hairpin moiety contained a bulky fluorophore group or an abasic group, and/or an additional oligonucleotide was hybridised to the hairpin.

An asymmetric 3.6-kilobase double-stranded DNA analyte (a fragment of bacteriophage lambda DNA; SEQ ID NO: 20) was obtained by PCR using primers, one of which contained multiple dUTP bases, and was end-repaired and dA-tailed by NEBNext end repair and NEBNext dA-tailing modules (New England Biolabs (NEB)), followed by NEB USER digest, to generate a 3' dA overhang at one end and leaving a 3' AGGA overhang at the opposite end.

A random library of *Escherichia coli* double-stranded DNA was generated by ligating generic adapters to *E. coli* SCS110 DNA which had been sheared using a Covaris gTube to a shear size of ~20 kb and amplifying by PCR. Fragments were end-repaired and dA-tailed by NEBNext end repair and NEBNext dA-tailing modules (New England Biolabs (NEB)) to generate 3' dA overhangs at both ends.

A Y-adapter was prepared by annealing DNA oligonucleotides (SEQ ID NO: 21, SEQ ID NO: 22). A DNA motor (Dda helicase) was loaded onto the adapter. Monomeric traptavidin was added to the adapter to bind to the 5' biotin moiety as blocker, to (1) prevent diffusion of the DNA motor backwards off the 5' end and (2) prevent unintentional capture of the 5' end of the library by the nanopore.

Hairpins bearing 3'-TCCT or 3'-T overhangs were prepared by heating DNAs SEQ ID NO: 24, SEQ ID NO: 25 or SEQ ID NO: 26 at 1 µM to 95 °C for 2 min in duplex-annealing buffer (Integrated DNA Technologies, Inc.), followed by snap-cooling on wet ice.

The asymmetric 3.6-kilobase double-stranded DNA analyte and the hairpin (SEQ ID NO: 24 or SEQ ID NO: 26) were ligated to the Y-adapter using LNB from Oxford Nanopore Technologies sequencing kit (SQK-LSK109) and T4 DNA Ligase (NEB). The sample was purified using Agencourt AMPure XP (Beckman Coulter) beads, with two washes with LFB from Oxford Nanopore Technologies sequencing kit (SQK-LSK109). The ligated substrate was eluted into 10 mM Tris-Cl, 50 mM NaCl (pH 8.0), yielding a '3.6 kb DNA library'.

The *Escherichia coli* double-stranded DNA and the hairpin (SEQ ID NO: 25) were ligated to the Y-adapter using LNB from Oxford Nanopore Technologies sequencing kit (SQK-LSK109) and T4 DNA Ligase (NEB). The sample was purified using Agencourt AMPure XP (Beckman Coulter) beads, with two washes with LFB from Oxford Nanopore Technologies sequencing kit (SQK-LSK109). The ligated substrate was eluted into 10 mM Tris-Cl, 50 mM NaCl (pH 8.0), yielding a 'random *E. coli* test library'.

Electrical measurements were acquired on a FLO-MIN106 MinION flow cell and MinION Mk1b from Oxford Nanopore Technologies. To 1200 µL FB (from Oxford Nanopore Technologies sequencing kit (SQK-LSK109)), 50 nM of DNA tether was added, yielding tether mix. 800 µL of tether mix was flowed through the system, followed by a 5 minute wait, then a further 200 µL of tether mix flowed through the system with the SpotON port open. 37.5 µL SQB from Oxford Nanopore Technologies sequencing kit (SQK-LSK109), 15 µL of either the 3.6 kb library or the random *E. coli* test library, 0.7 µL excess monomeric traptavidin (~100 nM tetramer) and 22.5 µL of LB from Oxford Nanopore Technologies sequencing kit (SQK-LSK109) were mixed, yielding "sequencing mix". To a portion of the reactions, oligonucleotide SEQ ID NO: 27 was also added at 50 nM. 75 µL of the sequencing mix were added to a MinION flowcell via the SpotON flow cell port.

The two libraries were tested with different run scripts. The 3.6 kb library was run with a custom sequencing script to control the applied potential as follows: 10 sec capture phase (+120 mV); 0.5 sec de-stalling phase (-40 mV); 85.5 seconds sequencing (+120 mV); eject phase (0 mV, 1 sec; -120 mV, 3 sec). This sequence of applied potentials was repeated multiple times.

The random *E. coli* test library was run with a custom active de-stalling script, with capture/sequencing voltage of 120 mV and eject/unblock voltage of -48 mV. Classifications for the stall level and strand (sequencing) level were programmed into a configuration file in the MinKNOW instrument control software that enabled the detection of the stalled species and applied an unblock potential that would not cause full ejection of the strand. The script functioned as follows: if MinKNOW detected that a strand was at the stall level, it would apply the unblock potential first for 5 seconds, then return to the sequencing potential of 120 mV to check five times for actively sequencing strands. If the stall level was still present, it would apply the unblock potential for a further 25 seconds, and repeat five times. A rest period of 3 seconds was incorporated between each unblock attempt. If upon returning the sequencing potential, MinKNOW detected an actively sequencing strand, it would stop attempting to unblock and apply only the sequencing potential. If this entire process did not yield an actively sequencing strand, MinKNOW would turn off the channel. Every 15 minutes, a "mux scan" was applied to reset the system, which globally unblocked all channels on the flow cell and checked for active nanopores at 120 mV.

Raw data was collected in a bulk FAST5 file using MinKNOW software (Oxford Nanopore Technologies).

Figure 13 shows the hairpin and oligonucleotide combinations which were used in this Example. The 3.6 kb DNA library was used to first characterise the capture-phase signals.

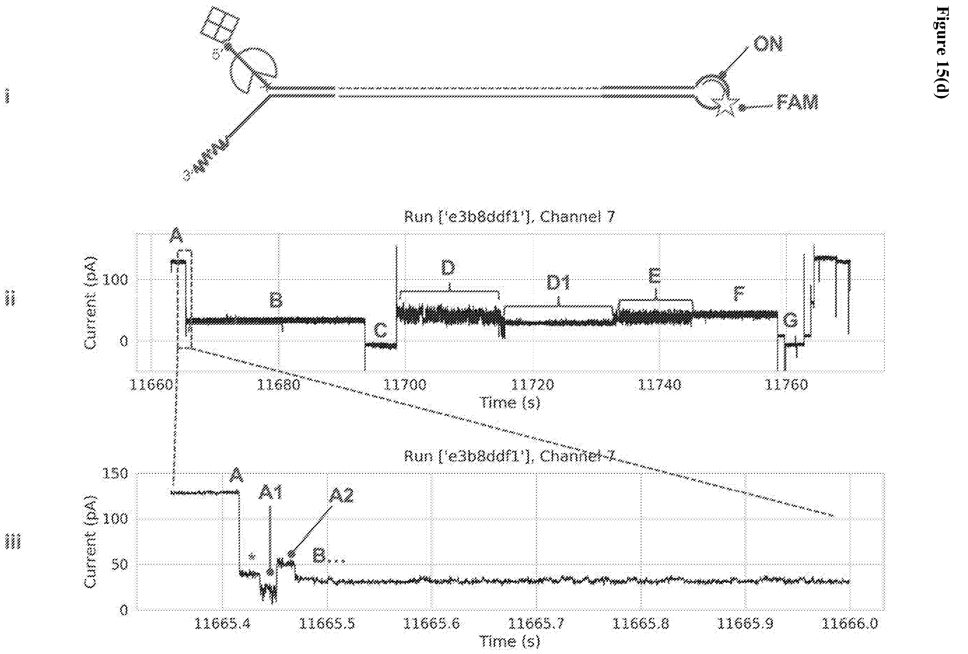

Figure 14 shows a schematic of the intermediates that would be expected to be detected in electrical measurements of enzyme-free and enzyme-mediated translocation. Two states A1 and A2, corresponding to a bulky group in the nanopore and the blocker oligonucleotide atop the nanopore respectively (shown in Figure 14), would be expected during the initial enzyme-free capture. An additional state D1, corresponding to the enzyme translocating over a bulky group in the hairpin moiety, would be expected between the template (D) and complement (E) phases of enzyme-mediated translocation. Figures 15a through 15d show example traces for each hairpin-oligonucleotide combination. A hairpin-only moiety (Figure 15a) exhibited a relatively flat, yet detectable capture phase (marked by an asterisk). Addition of an oligonucleotide hybridised to the hairpin moiety introduced an additional uptick intermediate (marked as A2 in Figure 15b), and the three bulky fluorescein-dT bases introduced a downtick (marked as A1 in Figure 15c). The combination of oligonucleotide hybridised to the hairpin and the fluorescein-dT bases introduced both types of signal (seen in Figure 15d). The introduction of an additional signal enabled the duration of the enzyme-free capture/entry phase of the polynucleotide to be measured (denoted by an asterisk in Figures 15a-d).

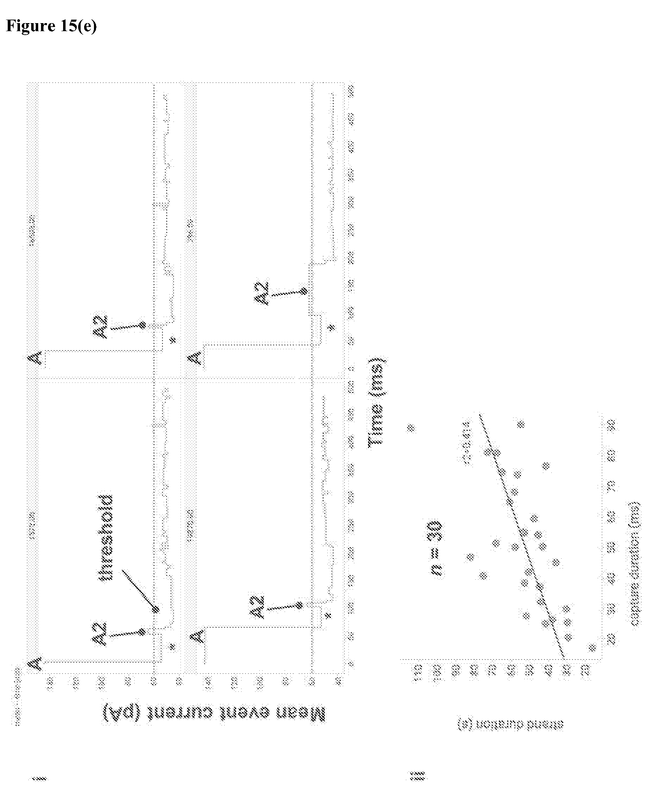

Examples using the scheme shown in Figure 15b (hairpin plus hybridised oligonucleotide) were used to measure the enzyme-free capture phases for a random *E. coli* test library (Figure 15e). Figure 15e, i shows simplified (event-fitted) raw data for four examples. A threshold of 60 pA was used to measure the enzyme-free capture duration between states A and A2, denoted by an asterisk. Figure 15e, ii shows the duration of the enzyme-mediated translocation plotted against the capture duration for thirty molecules. Linear regression analysis shows that the enzyme-free capture duration is correlated with the enzyme-mediated strand duration, confirming that it is possible to estimate the size of a strand using this method before decoding its sequence.

### Example 8

This example demonstrates how the duration of the signal from the initial, enzyme-free portion of DNA translocation (3'-5') through a nanopore may be used to estimate the size of one strand of a double-stranded DNA molecule before it is fully characterised, based solely on the duration of the capture/entry phase.

A Y-adapter was prepared by annealing DNA oligonucleotides (SEQ ID NO: 28, SEQ ID NO: 33, SEQ ID NO: 30 and SEQ ID NO: 32). A DNA motor (Dda helicase) was loaded onto the adapter.

A 10 kb fragment was obtained from bacteriophage lambda by PCR. Bacteriophages lambda DNA (~48 kb) and T4 DNA (~169 kb) were obtained from commercial sources. These double-stranded analytes were end-repaired and dA-tailed by NEBNext end repair and NEBNext dA-tailing modules (New England Biolabs (NEB)) to generate 3' dA overhangs at both ends of each fragment. Each sample was ligated (separately) to the dA-tailed end of the Y-adapter using LNB from Oxford Nanopore Technologies sequencing kit (SQK-LSK109) and T4 DNA Ligase (NEB). The samples were purified using Agencourt AMPure XP (Beckman Coulter) beads, with two washes with LFB from Oxford Nanopore Technologies sequencing kit (SQK-LSK109). The ligated substrates were eluted into 10 mM Tris-Cl, 50 mM NaCl (pH 8.0), yielding a '10 kb library', a 'lambda library' and a 'T4 library'.

Electrical measurements were acquired on a FLO-MIN106 MinION flow cell and MinION Mk1b from Oxford Nanopore Technologies. To 1170 µL FB (from Oxford Nanopore Technologies sequencing kit (SQK-LSK109)), 30 µL of FLT was added, yielding tether mix. 800 µL of tether mix was flowed through the system, followed by a 5 minute wait, then a further 200 µL of tether mix flowed through the system with the SpotON port open. 37.5 µL SQB from Oxford Nanopore Technologies sequencing kit (SQK-LSK109), 15 µL of the DNA library and 22.5 µL of LB from Oxford Nanopore Technologies sequencing kit (SQK-LSK109) were mixed, yielding "sequencing mix". 75 µL of the sequencing mix were added to a MinION flowcell via the SpotON flow cell port.

Data were collected using a custom script similar to that described in Example 7, with a capture/sequencing voltage of 120 mV.

Figure 16a shows the experimental schematic. The enzyme-free capture phase was measured by hand as the asterisked period between the open-pore level (A) and stall level (C), shown in more detail in Figure 16b, bottom panel. The capture phase is discernible via its distinct noise and median current level characteristics. The enzyme-mediated translocation time (E) was also measured. Figure 16b shows representative current-time traces for each of the three libraries described above, acquired on separate flow cells. For example, the 10 kb library had an enzyme-free capture duration of 1.6 sec and enzyme-mediated translocation time of 35.3 sec. Though long captures were obtained for the T4 library, no full-length examples were recorded, possibly owing to the increased likelihood of encountering nicks in the strand. Figure 16c shows a plot of the log of the capture duration (A to C) vs. the log of the enzyme-mediated translocation duration. From 31 examples, a linear correlation (*R*² = 0.74) was obtained, confirming that it is possible to estimate the size of a strand using this method before decoding its sequence.

### Example 9

This example demonstrates how the duration of the signal from the initial, enzyme-free portion of DNA translocation (3'-5') through a nanopore may be used to estimate the size of one strand of a double-stranded DNA molecule before it is fully characterised, based solely on the duration of the capture/entry phase.

A "sticky-overhang Y-adapter" was prepared by annealing DNA oligonucleotides having the polynucleotide sequence of SEQ ID NOs: 34, 35, 36 and 37. A DNA motor (Dda helicase) was loaded onto the adapter.

A transposase adapter was prepared by annealing DNA nucleotides having polynucleotide sequences of SEQ ID NOs: 38 and 39. The "transpososome" was prepared by incubating MuA transposase with this adapter, at a final concentration of 32.5 nM transpososome.

*Escherichia coli* K-12 genomic DNA was purified from cells using a Monarch HMW DNA Extraction Kit for Tissue (New England Biolabs, cat # T3060). The genomic DNA was treated sequentially with an Ultra II FFPE repair kit (New England Biolabs, cat # M6630) and end-repair/dA-tailing module (New England Biolabs, cat # E7546), generating an ultra-long dA-tailed library. 10 µg of this library was incubated with transpososome diluted 1:40 with FDB in a final volume of 500 µL (FDB from SQK-ULK001, Oxford Nanopore Technologies, Ltd.), vortexed, and incubated for 5 min at 30 °C using a heat block. The mixture was subsequently incubated with Thermolabile Proteinase K (New England Biolabs, cat # P8111S) for 15 min at 37 °C and 15 min at 65 °C using heat blocks. To this mixture was added 20x molar excess dT-overhang Y-adapter, T4 DNA ligase and LNB (SQK-LSK109, Oxford Nanopore Technologies, Ltd.); adapters were ligated to the genomic DNA by incubation for 10 min at room temperature. The mixture was purified by addition of 0.5x volume NAF-10 buffer and a Nanobind disc (Circulomics, Inc.), two washes with LFB (SQK-LSK109, Oxford Nanopore Technologies, Ltd.), and eluted in 75 µL EB (SQK-LSK109, *ibid*.) overnight, yielding the "sequencing library".

Electrical measurements were acquired on MinION flow cell with CsgG pores inserted and MinION Mk1b from Oxford Nanopore Technologies. To 1170 µL FB (from Oxford Nanopore Technologies sequencing kit (SQK-LSK109)), 30 µL of FLT was added, yielding tether mix. 800 µL of tether mix was flowed through the system, followed by a 5 minute wait, then a further 200 µL of tether mix flowed through the system with the SpotON port open. 37.5 µL SQB from Oxford Nanopore Technologies sequencing kit (SQK-LSK109) and 37.5 µL of the DNA library were mixed, yielding "sequencing mix". 75 µL of the sequencing mix were added to a MinION flowcell via the SpotON flow cell port.

Data were collected using a custom script similar to that described in Example 7, with a capture/sequencing voltage of 180 mV. Additional classifications for the initial capture signal were added to the sequencing script, which would accept or reject a strand based on the duration, current and noise characteristics of the capture signal.

The table below shows the effect of using the size-selection script on this library compared to a control which did not apply the rejection criteria.

| **Experiment** | **Median read length, kb** |
|---|---|
| No size-selection | 8.25 |
| With size-selection | 16.11 |

This example thus demonstrates advantages of the disclosed methods.

### Example 10

This example describes a method of measuring the time duration taken for a first part of a polynucleotide to freely translocate through a nanopore, and controlling the movement of the second part of the polynucleotide with a polynucleotide binding protein bound to a hairpin adapter. The time taken for the first part of the double-stranded polynucleotide to translocate through or across the pore is measured. The length of the polynucleotide is thereby measured. A correlation between time taken for the first part of the double-stranded polynucleotide to translocate through or across the pore and the length of the second part of the polynucleotide is observed. The instrument control software is used to analyse the length of the first part of the polynucleotide in real time and reject the strand if below a specified length threshold, thereby enriching the proportion of molecules sequenced longer than the set threshold.

### Method

A **"hairpin adapter"** was made by annealing oligonucleotides having the polynucleotide sequences of SEQ ID NOs: 40 and 41. A polynucleotide binding protein (a Dda helicase) was loaded onto the hairpin adapter. The protein-bound hairpin adapter was purified using SPRI beads. An adapter lacking enzyme, but carrying a leader and tether site was prepared by annealing polynucleotides having the sequences of SEQ ID NOs: 42, 43 and 44, generating a **"leader-adapter".**

*Escherichia coli* genomic DNA was extracted using a Monarch HMW DNA Extraction Kit for Tissue (New England Biolabs, cat # T3060). The genomic DNA was treated sequentially with an Ultra II FFPE repair kit (New England Biolabs, cat # M6630) and end-repair/dA-tailing module (New England Biolabs, cat # E7546), generating an ultra-long dA-tailed library. Transposome mix was generated by mixing 6 µL FRA with 244 µL FDB. 10 µg of the dA-tailed library was incubated with the transpososome mix for 5 min at 30°C and 5 min at 80°C using a heat block, in a final volume of 650 µL. To this mixture 2.5 µL of 800 nM leader-adapter was added, and the mixture incubated at room temperature for 15 min to attach the leader-adapter to the tagmented library. The hairpin adapter was ligated to this library by addition of the hairpin adapter, T4 DNA ligase and LNB (SQK-LSK109, Oxford Nanopore Technologies, Ltd.), and incubation for 10 min at room temperature. The mixture was purified by addition of 0.5x volume NAF-10 buffer and a Nanobind disc (Circulomics, Inc.), two washes with LFB (SQK-LSK109, Oxford Nanopore Technologies, Ltd.), and eluted in 75 µL EB (SQK-LSK109, *ibid*.) overnight, yielding the "sequencing library".

Electrical measurements were acquired on a FLO-MIN111 MinION flowcell and GridION Mk1 from Oxford Nanopore Technologies. To 1170 µL FB (from Oxford Nanopore Technologies sequencing kit (SQK-LSK109)), 30 µL of FLT was added, yielding tether mix. 800 µL of tether mix was flowed through the system, followed by a 5 minute wait, then a further 200 µL of tether mix flowed through the system with the SpotON port open. 37.5 µL SQB from Oxford Nanopore Technologies sequencing kit (SQK-LSK109) and 37.5 µL of the sequencing library were mixed, yielding "sequencing mix". The sequencing mix was added to the MinION flowcell via the SpotON flow cell port.

The time taken for the first part of the strand to translocate was determined by a post-run analysis method, by detecting a change in the rolling standard deviation of the current signal between the first part of the strand and the second part of the strand. The length of the second part of the strand was determined by base calling and alignment to the *E. coli* reference sequence. To perform the online size-selection, a custom script was developed, using classifications specific to the duration, current and noise of the first part of the strand. Events shorter than the threshold duration were set to be rejected by the MinKNOW instrument control software.

The table below shows the effect of using the size-selection script on this library compared to a control which did not apply the rejection criteria.

| **Experiment** | **Median read length, kb** |
|---|---|
| No size-selection | 6.50 |
| With size-selection | 10.77 |

This example thus demonstrates advantages of the disclosed methods.

### Example 11

This example describes a method of measuring the time duration taken for a first part of a polynucleotide to freely translocate through a nanopore, and controlling the movement of the second part of the polynucleotide with a polynucleotide binding protein bound to a hairpin adapter. The time taken for the first part of the double-stranded polynucleotide to translocate through or across the pore is measured. The length of the polynucleotide is thereby measured. A correlation between time taken for the first part of the double-stranded polynucleotide to translocate through or across the pore and the length of the second part of the polynucleotide is observed. A bias in the time taken for the first part of the double-stranded polynucleotide to translocate is observed depending on which strand of the polynucleotide is captured first.

### Method

A **"hairpin adapter"** was made by annealing oligonucleotides having the polynucleotide sequence of SEQ ID NOs: 40 and 41. A polynucleotide binding protein (a Dda helicase) was loaded onto the hairpin adapter. The protein-bound hairpin adapter was purified using SPRI beads. An adapter lacking enzyme, but carrying a leader and tether site was prepared by annealing polynucleotides of SEQ ID NOs: 42, 43 and 44, generating a **"leader-adapter".**

Bacteriophage lambda DNA was purchased from New England Biolabs (cat # N3011). The genomic DNA was treated with an end-repair/dA-tailing module (New England Biolabs, cat # E7546), generating a dA-tailed library. Transposome mix was generated by mixing 0.47 µL FRA with 11.3 µL FDB (Oxford Nanopore Technologies). 1 µg of the dA-tailed library was incubated with the transpososome mix for 2 min at 30 °C and 2 min at 80 °C using a heat block, in a final volume of 45 µL. To this mixture 1 µL of 800 nM leader-adapter was added, and the mixture incubated at room temperature for 15 min to attach the leader-adapter to the tagmented library. The hairpin adapter was ligated to this library by addition of 40 nM hairpin adapter, T4 DNA ligase and LNB (SQK-LSK109, Oxford Nanopore Technologies, Ltd.), and incubation for 10 min at room temperature. The mixture was purified by addition of 0.4x volume SPRI beads (Beckman Coulter), two washes with LFB (SQK-LSK109, Oxford Nanopore Technologies, Ltd.), and eluted in 75 µL EB (SQK-LSK109, *ibid*.) overnight, yielding the "sequencing library".

Electrical measurements were acquired on a FLO-MIN111 MinION flowcell and GridION Mk1 from Oxford Nanopore Technologies. To 1170 µL FB (from Oxford Nanopore Technologies sequencing kit (SQK-LSK109)), 30 µL of FLT was added, yielding tether mix. 800 µL of tether mix was flowed through the system, followed by a 5 minute wait, then a further 200 µL of tether mix flowed through the system with the SpotON port open. 37.5 µL SQB from Oxford Nanopore Technologies sequencing kit (SQK-LSK109) and 37.5 µL of the sequencing library were mixed, yielding "sequencing mix". The sequencing mix was added to the MinION flowcell via the SpotON flow cell port.

The time taken for the first part of the strand to translocate was determined by a post-run analysis method, by detecting a change in the rolling standard deviation of the current signal between the first part of the strand and the second part of the strand. The read orientation relative to the reference and the length of the second part of the strand was determined by base calling and alignment to the lambda reference sequence. The table below shows, for all read length ranges tested, that the median translocation speed of the first part of the strand is faster for reads which align in the reverse direction relative to the reference than for reads which align in the forwards direction relative to the reference.

| aligned read length (bases) | read orientation | number of strands | median translocation speed (bases per second) |
|---|---|---|---|
| 0 - 4999 | - | 538 | 195679.17 |
| 0 - 4999 | + | 260 | 190290.4 |
| 5000 - 9999 | - | 264 | 143347.05 |
| 5000 - 9999 | + | 168 | 123329.26 |
| 10000 - 14999 | - | 173 | 105325.8 |
| 10000 - 14999 | + | 83 | 84837.21 |
| 15000 - 19999 | - | 108 | 84458.23 |
| 15000 - 19999 | + | 41 | 69410.17 |

This example thus demonstrates advantages of the disclosed methods.

### Example 12

This example describes the training of a machine learning algorithm to predict the GC content of a DNA strand based solely on the signal generated by the enzyme-free translocation of the DNA strand through a nanopore. Labelling of training data is accomplished by loading a motor on a hairpin adapter attached to one end of the DNA strand - this enables the recording of the enzyme-free translocation of the first strand, followed immediately by the enzyme-controlled translocation of the second strand, which can be basecalled using Guppy Basecaller to yield GC content.

A **"hairpin adapter"** is prepared by annealing DNA oligonucleotides of SEQ ID NOs: 40 and 41. A DNA motor (Dda helicase) is loaded onto the adapter, and the disulfide is closed via reaction with TMAD.

A **"leader-adapter"** is prepared by annealing DNA oligonucleotides of SEQ ID NOs: 42, 43 and 44.

*Escherichia coli* genomic DNA is extracted using a Monarch HMW DNA Extraction Kit for Tissue (New England Biolabs, cat # T3060). The genomic DNA is treated sequentially with an Ultra II FFPE repair kit (New England Biolabs, cat # M6630) and end-repair/dA-tailing module (New England Biolabs, cat # E7546), generating an ultra-long dA-tailed library. 6 µL FRA are diluted to 250 µL using FDB, then added to 750 µL of a DNA sample (~40 µg) in a final volume of 1 mL (FRA and FDB both from SQK-ULK001, Oxford Nanopore Technologies, Ltd.). The mixture is vortexed, and incubated for 5 min at 30 °C and 5 min at 75 °C using a heat block, then allowed to cool to room temperature. 6 µL of 800 nM leader-adapter is added to this mixture, and the mixture is incubated at room temperature for 15 min to attach the leader-adapter to the tagmented library. The hairpin adapter is ligated to this library by addition of the hairpin adapter, T4 DNA ligase and LNB (SQK-LSK109, Oxford Nanopore Technologies, Ltd.), and incubation for 10 min at room temperature. The mixture is purified by addition of 0.5x volume NAF-10 buffer and a Nanobind disc (Circulomics, Inc.), two washes with LFB (SQK-LSK109, Oxford Nanopore Technologies, Ltd.), and eluted in 225 µL EB (SQK-LSK109, ibid.) overnight, yielding the **"DNA library".** The resulting DNA Library has a 5' single stranded leader on one end and a hairpin with motor loaded on the other end.

Electrical measurements are acquired on a FLO-MIN111 MinION flowcell and GridION Mk1 from Oxford Nanopore Technologies. To 1170 µL FB (from Oxford Nanopore Technologies sequencing kit (SQK-LSK109)), 30 µL of FLT is added, yielding tether mix. 800 µL of tether mix is flowed through the system, followed by a 5 minute wait, then a further 200 µL of tether mix is flowed through the system with the SpotON port open. 37.5 µL SQB from Oxford Nanopore Technologies sequencing kit (SQK-LSK109) and 37.5 µL of the DNA Library are mixed, yielding "sequencing mix". The sequencing mix is added to the MinION flowcell via the SpotON flow cell port.

When a DNA strand is captured by a nanopore, a two-phase event is recorded. The first part of the event consists of the enzyme-free translocation of the first strand ("template") through the nanopore. The second part of the event consists of the enzyme-controlled movement of the second strand ("complement") through the nanopore.

The complement sequence, and hence its GC content, is determined by basecalling with Guppy Basecaller (Oxford Nanopore Technologies, Ltd.). Training examples are generated by labelling enzyme-free template signals with their GC content as determined by basecalling. A machine learning algorithm is trained from a set of such training examples. The trained model is evaluated on a set of examples held out from the training set. The evaluation results show that the model is able to predict GC content using only the enzyme-free template signal.

### Example 13

This example describes the training of a classifier to identify amplicons from a panel based solely on the signal generated by the enzyme-free translocation of the DNA strand through a nanopore. Labelling of training data is accomplished by loading a motor on a hairpin adapter attached to one end of the DNA strand - this enables the recording of the enzyme-free translocation of the first strand, followed immediately by the enzyme-controlled translocation of the second strand, which can be basecalled using Guppy Basecaller and aligned to the amplicon references to determine the identity of the amplicon.

A **"hairpin adapter"** is prepared by annealing DNA oligonucleotides of SEQ ID NOs: 40 and 41. A DNA motor (Dda helicase) is loaded onto the adapter, and the disulfide is closed via reaction with TMAD.

A **"leader-adapter"** is prepared by annealing DNA oligonucleotides of SEQ ID NOs: 42, 43 and 45.

A panel of DNA amplicons is generated by PCR amplification of genomic DNA with a set of primers. The DNA panel is treated with UltraII end repair/dA-tailing module (NEB) to generate 3' A overhangs. The hairpin adapter and leader-adapter are attached to the ends of the DNA amplicons by T4 DNA ligase and LNB (SQB-LSK109, Oxford Nanopore Technologies, Ltd.). The ligated DNA panel is purified with Agencourt AMPure (Beckman Coulter) beads, washed twice with SFB (Oxford Nanopore Technologies Ltd.), then eluted in EB (Oxford Nanopore Technologies Ltd.), yielding the **"DNA library."**

Electrical measurements are acquired on a FLO-MIN111 MinION flowcell and GridION Mk1 from Oxford Nanopore Technologies. To 1170 µL FB (from Oxford Nanopore Technologies sequencing kit (SQK-LSK109)), 30 µL of FLT is added, yielding tether mix. 800 µL of tether mix is flowed through the system, followed by a 5 minute wait, then a further 200 µL of tether mix is flowed through the system with the SpotON port open. 37.5 µL SQB from Oxford Nanopore Technologies sequencing kit (SQK-LSK109) and 37.5 µL of the DNA Library are mixed, yielding "sequencing mix". The sequencing mix is added to the MinION flowcell via the SpotON flow cell port.

When a DNA strand is captured by a nanopore, a two-phase event is recorded. The first part of the event consists of the enzyme-free translocation of the first strand ("template") through the nanopore. The second part of the event consists of the enzyme-controlled movement of the second strand ("complement") through the nanopore.

The identity of the amplicon can be determined by basecalling the complement signal using Guppy Basecaller, then aligning the basecalls to the amplicon references. Training examples are generated by labelling enzyme-free template signals with the amplicon identity as determined by basecalling. A machine learning classifier is trained using a set of training examples. The trained model is evaluated on a set of examples held out from the training set. The evaluation results show that the model is able to correctly predict the identity of amplicons within the panel using only the enzyme-free template signal.

### Example 14

This example describes the training of a machine learning algorithm to predict the GC content of a DNA strand based solely on the signal generated by the enzyme-free translocation of the DNA strand through a nanopore. Labelling of training data is accomplished by recording the enzyme-free translocation (3'-5') of a DNA strand through a nanopore, followed by the enzyme-controlled translocation (5'-3') of the DNA strand through the nanopore, which can be basecalled using Guppy Basecaller to yield GC content.

A "dT-overhang Y-adapter" is prepared by annealing DNA oligonucleotides of SEQ ID NOs: 34, 46, 36 and 37. A DNA motor (Dda helicase) is loaded onto the adapter.

A "sticky-overhang Y-adapter" is prepared by annealing DNA oligonucleotides of SEQ ID NOs: 34, 35, 36 and 37. A DNA motor (Dda helicase) is loaded onto the adapter.

A transposase adapter is prepared by annealing DNA nucleotides of SEQ ID NOs: 47 and 39. The "transpososome" is prepared by incubating MuA transposase with this adapter.

*Escherichia coli* K-12 genomic DNA is purified from cells using a Monarch HMW DNA Extraction Kit for Tissue (New England Biolabs, cat # T3060). The genomic DNA is treated sequentially with an Ultra II FFPE repair kit (New England Biolabs, cat # M6630) and end-repair/dA-tailing module (New England Biolabs, cat # E7546), generating an ultra-long dA-tailed library. 6 µL of transpososome are diluted to 250 µL using FDB from SQK-ULK001 (Oxford Nanopore Technologies) and added to 40 µg of the dA-tailed library in a final volume of 1 mL (FDB from SQK-ULK001, Oxford Nanopore Technologies, Ltd.). The mixture is vortexed and incubated for 5 min at 30 °C and 5 min at 75 °C using a heat block. To this mixture is added 2 nM dT-overhang Y-adapter and 2 nM sticky-overhang Y-adapter, T4 DNA ligase and LNB (SQK-LSK109, Oxford Nanopore Technologies, Ltd.); adapters are ligated to the genomic DNA by incubation for 10 min at room temperature. The mixture is purified by addition of 0.5x volume NAF-10 buffer and a Nanobind disc (Circulomics, Inc.), two washes with LFB (SQK-LSK109, Oxford Nanopore Technologies, Ltd.), and eluted in 225 µL EB (SQK-LSK109, ibid.) overnight, yielding the "sequencing library".

Electrical measurements are acquired on MinION flow cell with CsgG pores inserted and MinION Mk1b from Oxford Nanopore Technologies. To 1170 µL FB (from Oxford Nanopore Technologies sequencing kit (SQK-LSK109)), 30 µL of FLT is added, yielding tether mix. 800 µL of tether mix is flowed through the system, followed by a 5 minute wait, then a further 200 µL of tether mix is flowed through the system with the SpotON port open. 37.5 µL SQB from Oxford Nanopore Technologies sequencing kit (SQK-LSK109) and 37.5 µL of the DNA Library are mixed, yielding "sequencing mix". The sequencing mix is added to the MinION flowcell via the SpotON flow cell port.

When a DNA strand is captured by a nanopore, the enzyme-free capture signal (3'-5') is recorded first, followed by the enzyme-driven translocation (5'-3'). The DNA sequence, and hence its GC content, is determined by basecalling with Guppy Basecaller (Oxford Nanopore Technologies, Ltd.). Training examples are generated by labelling enzyme-free capture signals with their GC content as determined by basecalling the enzyme-driven translocation signal. A machine learning algorithm is trained from a set of such training examples. The trained model is evaluated on a set of examples held out from the training set. The evaluation results show that the model is able to predict GC content using only the enzyme-free capture signal.

### Example 15

This example describes the training of a classifier to identify amplicons from a panel based solely on the signal generated by the enzyme-free translocation of the DNA strand through a nanopore. Labelling of training data is accomplished by recording the enzyme-free translocation (3'-5') of a DNA strand through a nanopore, followed by the enzyme-controlled translocation (5'-3') of the DNA strand through the nanopore, which can be basecalled using Guppy Basecaller and aligned to the amplicon references to determine the identity of the amplicon.

A "leader-adapter" is prepared by annealing DNA oligonucleotides of SEQ ID NOs: 34, 46, 36 and 37. A DNA motor (Dda helicase) is loaded onto the adapter.

A panel of DNA amplicons is generated by PCR amplification of genomic DNA with a set of primers. The DNA panel is treated with UltraII end repair/dA-tailing module (NEB) to generate 3' A overhangs. The leader-adapter is attached to the ends of the DNA amplicons by T4 DNA ligase and LNB (SQK-LSK109, Oxford Nanopore Technologies, Ltd.). The ligated DNA panel is purified with Agencourt AMPure (Beckman Coulter) beads, washed twice with SFB (Oxford Nanopore Technologies Ltd.), then eluted in EB (Oxford Nanopore Technologies Ltd.), yielding the **"DNA library."**

Electrical measurements are acquired on MinION flow cell with CsgG pores inserted and MinION Mk1b from Oxford Nanopore Technologies. To 1170 µL FB (from Oxford Nanopore Technologies sequencing kit (SQK-LSK109)), 30 µL of FLT is added, yielding tether mix. 800 µL of tether mix is flowed through the system, followed by a 5 minute wait, then a further 200 µL of tether mix is flowed through the system with the SpotON port open. 37.5 µL SQB from Oxford Nanopore Technologies sequencing kit (SQK-LSK109) and 37.5 µL of the DNA Library are mixed, yielding "sequencing mix". The sequencing mix is added to the MinION flowcell via the SpotON flow cell port.

When a DNA strand is captured by a nanopore, the enzyme-free capture signal (3'-5') is recorded first, followed by the enzyme-driven translocation (5'-3'). The identity of the amplicon can be determined by basecalling the enzyme-driven translocation signal using Guppy Basecaller, then aligning the basecalls to the amplicon references. Training examples are generated by labelling enzyme-free capture signals with the amplicon identity as determined by basecalling. A machine learning classifier is trained using a set of training examples. The trained model is evaluated on a set of examples held out from the training set. The evaluation results show that the model is able to correctly predict the identity of amplicons within the panel using only the enzyme-free capture signal.

### Description of the Sequence Listing

SEQ ID NO: 1 shows the amino acid sequence of (hexa-histidine tagged) exonuclease I (EcoExo I) from *E. coli.*
SEQ ID NO: 2 shows the amino acid sequence of the exonuclease III enzyme from *E. coli.*
SEQ ID NO: 3 shows the amino acid sequence of the RecJ enzyme from *T. thermophilus* (TthRecJ-cd).
SEQ ID NO: 4 shows the amino acid sequence of bacteriophage lambda exonuclease. The sequence is one of three identical subunits that assemble into a trimer. (http://www.neb.com/nebecomm/products/productM0262. asp).
SEQ ID NO: 5 shows the amino acid sequence of Phi29 DNA polymerase from *Bacillus subtilis* phage Phi29.
SEQ ID NO: 6 shows the amino acid sequence of Trwc Cba (*Citromicrobium bathyomarinum*) helicase.
SEQ ID NO: 7 shows the amino acid sequence of Hel308 Mbu (*Methanococcoides burtonii*) helicase.
SEQ ID NO: 8 shows the amino acid sequence of the Dda helicase 1993 from Enterobacteria phage T4.
SEQ ID NO: 9: shows the nucleotide sequence of a tag
SEQ ID NO: 11: shows the nucleotide sequence of a polynucleotide strand discussed in example 5.
SEQ ID NO: 12: shows the nucleotide sequence of a polynucleotide strand discussed in example 5.
SEQ ID NO: 13 shows the nucleotide sequence of a polynucleotide analyte discussed in example 5.
SEQ ID NO: 14 shows the nucleotide sequence of a polynucleotide analyte discussed in example 5.
SEQ ID NO: 15 shows the nucleotide sequence of a polynucleotide adapter discussed in example 5.
SEQ ID NO: 16 shows the nucleotide sequence of a polynucleotide adapter discussed in example 5.
SEQ ID NOs: 20-33 show the nucleotide sequences of polynucleotides discussed in examples 7 and 8.
SEQ ID NOs: 34-47 show the nucleotide sequences of polynucleotides discussed in examples 9 to 15. 3 = iSpC3, 8 = iSp18, mU = 2'OMe uracil, 9 = iSp9, N3 = 3' amino C7 labelled with azidohexanoic acid.
SEQ ID NO: 1 - exonuclease I from *E. coli* 
MMNDGKQQSTFLFHDYETFGTHPALDRPAQFAAIRTDSEFNVIGEPEVFYCKPADDYLPQ
PGAVLITGITPQEARAKGENEAAFAARIHSLFTVPKTCILGYNNVRFDDEVTRNIFYRNF
YDPYAWSWQHDNSRWDLLDVMRACYALRPEGINWPENDDGLPSFRLEHLTKANGIEHSNA
HDAMADVYATIAMAKLVKTRQPRLFDYLFTHRNKHKLMALIDVPQMKPLVHVSGMFGAWR
GNTSWVAPLAWHPENRNAVIMVDLAGDISPLLELDSDTLRERLYTAKTDLGDNAAVPVKL
VHINKCPVLAQANTLRPEDADRLGINRQHCLDNLKILRENPQVREKVVAIFAEAEPFTPS
DNVDAQLYNGFFSDADRAAMKIVLETEPRNLPALDITFVDKRIEKLLFNYRARNFPGTLD
YAEQQRWLEHRRQVFTPEFLQGYADELQMLVQQYADDKEKVALLKALWQYAEEIVSGSGH
HHHHH
SEQ ID NO: 2 - exonuclease III enzyme from *E. coli* 
MKFVSFNINGLRARPHQLEAIVEKHQPDVIGLQETKVHDDMFPLEEVAKLGYNVFYHGQK
GHYGVALLTKETPIAVRRGFPGDDEEAQRRIIMAEIPSLLGNVTVINGYFPQGESRDHPI
KFPAKAQFYQNLQNYLETELKRDNPVLIMGDMNISPTDLDIGIGEENRKRWLRTGKCSFL
PEEREWMDRLMSWGLVDTFRHANPQTADRFSWFDYRSKGFDDNRGLRIDLLLASQPLAEC
CVETGIDYEIRSMEKPSDHAPVWATFRR
SEQ ID NO: 3 - RecJ enzyme from T. thermophilus 
MFRRKEDLDPPLALLPLKGLREAAALLEEALRQGKRIRVHGDYDADGLTGTAILVRGLAA
LGADVHPFIPHRLEEGYGVLMERVPEHLEASDLFLTVDCGITNHAELRELLENGVEVIVT
DHHTPGKTPPPGLVVHPALTPDLKEKPTGAGVAFLLLWALHERLGLPPPLEYADLAAVGT
IADVAPLWGWNRALVKEGLARIPASSWVGLRLLAEAVGYTGKAVEVAFRIAPRINAASRL
GEAEKALRLLLTDDAAEAQALVGELHRLNARRQTLEEAMLRKLLPQADPEAKAIVLLDPE
GHPGVMGIVASRILEATLRPVFLVAQGKGTVRSLAPISAVEALRSAEDLLLRYGGHKEAA
GFAMDEALFPAFKARVEAYAARFPDPVREVALLDLLPEPGLLPQVFRELALLEPYGEGNP
EPLFL
SEQ ID NO: 4 - bacteriophage lambda exonuclease 
MTPDIILQRTGIDVRAVEQGDDAWHKLRLGVITASEVHNVIAKPRSGKKWPDMKMSYFHT
LLAEVCTGVAPEVNAKALAWGKQYENDARTLFEFTSGVNVTESPIIYRDESMRTACSPDG
LCSDGNGLELKCPFTSRDFMKFRLGGFEAIKSAYMAQVQYSMWVTRKNAWYFANYDPRMK
REGLHYVVIERDEKYMASFDEIVPEFIEKMDEALAEIGFVFGEQWR
SEQ ID NO: 5 - Phi29 DNA polymerase 
MKHMPRKMYSCAFETTTKVEDCRVWAYGYMNIEDHSEYKIGNSLDEFMAWVLKVQADLYF
HNLKFDGAFIINWLERNGFKWSADGLPNTYNTIISRMGQWYMIDICLGYKGKRKIHTVIY
DSLKKLPFPVKKIAKDFKLTVLKGDIDYHKERPVGYKITPEEYAYIKNDIQIIAEALLIQ
FKQGLDRMTAGSDSLKGFKDIITTKKFKKVFPTLSLGLDKEVRYAYRGGFTWLNDRFKEK
EIGEGMVFDVNSLYPAQMYSRLLPYGEPIVFEGKYVWDEDYPLHIQHIRCEFELKEGYIP
TIQIKRSRFYKGNEYLKSSGGEIADLWLSNVDLELMKEHYDLYNVEYISGLKFKATTGLF
KDFIDKWTYIKTTSEGAIKQLAKLMLNSLYGKFASNPDVTGKVPYLKENGALGFRLGEEE
TKDPVYTPMGVFITAWARYTTITAAQACYDRIIYCDTDSIHLTGTEIPDVIKDIVDPKKL
GYWAHESTFKRAKYLRQKTYIQDIYMKEVDGKLVEGSPDDYTDIKFSVKCAGMTDKIKKE
VTFENFKVGFSRKMKPKPVQVPGGVVLVDDTFTIKSGGSAWSHPQFEKGGGSGGGSGGSA
WSHPQFEK
SEQ ID NO: 6 - Trwc Cba helicase 
MLSVANVRSPSAAASYFASDNYYASADADRSGQWIGDGAKRLGLEGKVEARAFDALLRGE
LPDGSSVGNPGQAHRPGTDLTFSVPKSWSLLALVGKDERIIAAYREAVVEALHWAEKNAA
ETRVVEKGMVVTQATGNLAIGLFQHDTNRNQEPNLHFHAVIANVTQGKDGKWRTLKNDRL
WQLNTTLNSIAMARFRVAVEKLGYEPGPVLKHGNFEARGISREQVMAFSTRRKEVLEARR
GPGLDAGRIAALDTRASKEGIEDRATLSKQWSEAAQSIGLDLKPLVDRARTKALGQGMEA
TRIGSLVERGRAWLSRFAAHVRGDPADPLVPPSVLKQDRQTIAAAQAVASAVRHLSQREA
AFERTALYKAALDFGLPTTIADVEKRTRALVRSGDLIAGKGEHKGWLASRDAVVTEQRIL
SEVAAGKGDSSPAITPQKAAASVQAAALTGQGFRLNEGQLAAARLILISKDRTIAVQGIA
GAGKSSVLKPVAEVLRDEGHPVIGLAIQNTLVQMLERDTGIGSQTLARFLGGWNKLLDDP
GNVALRAEAQASLKDHVLVLDEASMVSNEDKEKLVRLANLAGVHRLVLIGDRKQLGAVDA
GKPFALLQRAGIARAEMATNLRARDPVVREAQAAAQAGDVRKALRHLKSHTVEARGDGAQ
VAAETWLALDKETRARTSIYASGRAIRSAVNAAVQQGLLASREIGPAKMKLEVLDRVNTT
REELRHLPAYRAGRVLEVSRKQQALGLFIGEYRVIGQDRKGKLVEVEDKRGKRFRFDPAR
IRAGKGDDNLTLLEPRKLEIHEGDRIRWTRNDHRRGLFNADQARVVEIANGKVTFETSKG
DLVELKKDDPMLKRIDLAYALNVHMAQGLTSDRGIAVMDSRERNLSNQKTFLVTVTRLRD
 
HLTLVVDSADKLGAAVARNKGEKASAIEVTGSVKPTATKGSGVDQPKSVEANKAEKELTR
SKSKTLDFGI
**SEQ ID NO: 7 - Hel308 Mbu helicase** 
MMIRELDIPRDIIGFYEDSGIKELYPPQAEAIEMGLLEKKNLLAAIPTASGKTLLAELAM
IKAIREGGKALYIVPLRALASEKFERFKELAPFGIKVGISTGDLDSRADWLGVNDIIVAT
SEKTDSLLRNGTSWMDEITTVVVDEIHLLDSKNRGPTLEVTITKLMRLNPDVQVVALSAT
VGNAREMADWLGAALVLSEWRPTDLHEGVLFGDAINFPGSQKKIDRLEKDDAVNLVLDTI
KAEGQCLVFESSRRNCAGFAKTASSKVAKILDNDIMIKLAGIAEEVESTGETDTAIVLAN
CIRKGVAFHHAGLNSNHRKLVENGFRQNLIKVISSTPTLAAGLNLPARRVIIRSYRRFDS
NFGMQPIPVLEYKQMAGRAGRPHLDPYGESVLLAKTYDEFAQLMENYVEADAEDIWSKLG
TENALRTHVLSTIVNGFASTRQELFDFFGATFFAYQQDKWMLEEVINDCLEFLIDKAMVS
ETEDIEDASKLFLRGTRLGSLVSMLYIDPLSGSKIVDGFKDIGKSTGGNMGSLEDDKGDD
ITVTDMTLLHLVCSTPDMRQLYLRNTDYTIVNEYIVAHSDEFHEIPDKLKETDYEWFMGE
VKTAMLLEEWVTEVSAEDITRHFNVGEGDIHALADTSEWLMHAAAKLAELLGVEYSSHAY
SLEKRIRYGSGLDLMELVGIRGVGRVRARKLYNAGFVSVAKLKGADISVLSKLVGPKVAY
NILSGIGVRVNDKHFNSAPISSNTLDTLLDKNQKTFNDFQ
**SEQ ID NO: 8 - Dda helicase** 
MTFDDLTEGQKNAFNIVMKAIKEKKHHVTINGPAGTGKTTLTKFIIEALISTGETGIILA
APTHAAKKILSKLSGKEASTIHSILKINPVTYEENVLFEQKEVPDLAKCRVLICDEVSMY
DRKLFKILLSTIPPWCTIIGIGDNKQIRPVDPGENTAYISPFFTHKDFYQCELTEVKRSN
APIIDVATDVRNGKWIYDKVVDGHGVRGFTGDTALRDFMVNYFSIVKSLDDLFENRVMAF
TNKSVDKLNSIIRKKIFETDKDFIVGEIIVMQEPLFKTYKIDGKPVSEIIFNNGQLVRII
EAEYTSTFVKARGVPGEYLIRHWDLTVETYGDDEYYREKIKIISSDEELYKFNLFLGKTA
ETYKNWNKGGKAPWSDFWDAKSQFSKVKALPASTFHKAQGMSVDRAFIYTPCIHYADVEL
AQQLLYVGVTRGRYDVFYV
**SEQ ID NO: 9**
   GGAACCTCTCTGACAA
**SEQ ID NO: 11** 
/5Phos/CGTCCTGTCGCTGTGTCTCGTTTTTTTTTT/iSp18//iSp18//iSp18//iSp18/AATGT
ACTTCGTTCAGTTACGTATTGCTAGAGCCGAGACACAGCGACAGGACGT
**SEQ ID NO: 12** 
/5Phos/GCAATACGTAACTGAACGAAGT/iBNA-A//iBNA-MeC//iBNA-A//iBNA-T//iBNA-T
/TTTGAGGCGAGCGGTCAA
**SEQ ID NO: 13** 
GCCATCAGATTGTGTTTGTTAGTCGCTGCCATCAGATTGTGTTTGTTAGTCGCTTTTTTTTTTTGGAATTTTT
TTTTTGGAATTTTTTTTTTGCGCTAACAACCTCCTGCCGTTTTGCCCGTGCATATCGGTCACGAACAAATCTG
ATTACTAAACACAGTAGCCTGGATTTGTTCTATCAGTAATCGACCTTATTCCTAATTAAATAGAGCAAATCCC
CTTATTGGGGGTAAGACATGAAGATGCCAGAAAAACATGACCTGTTGGCCGCCATTCTCGCGGCAAAGGAACA
AGGCATCGGGGCAATCCTTGCGTTTGCAATGGCGTACCTTCGCGGCAGATATAATGGCGGTGCGTTTACAAAA
ACAGTAATCGACGCAACGATGTGCGCCATTATCGCCTAGTTCATTCGTGACCTTCTCGACTTCGCCGGACTAA
GTAGCAATCTCGCTTATATAACGAGCGTGTTTATCGGCTACATCGGTACTGACTCGATTGGTTCGCTTATCAA
ACGCTTCGCTGCTAAAAAAGCCGGAGTAGAAGATGGTAGAAATCAATAATCAACGTAAGGCGTTCCTCGATAT
GCTGGCGTGGTCGGAGGGAACTGATAACGGACGTCAGAAAACCAGAAATCATGGTTATGACGTCATTGTAGGC
GGAGAGCTATTTACTGATTACTCCGATCACCCTCGCAAACTTGTCACGCTAAACCCAAAACTCAAATCAACAG
GCGCCGGACGCTACCAGCTTCTTTCCCGTTGGTGGGATGCCTACCGCAAGCAGCTTGGCCTGAAAGACTTCTC
TCCGAAAAGTCAGGACGCTGTGGCATTGCAGCAGATTAAGGAGCGTGGCGCTTTACCTATGATTGATCGTGGT
GATATCCGTCAGGCAATCGACCGTTGCAGCAATATCTGGGCTTCACTGCCGGGCGCTGGTTATGGTCAGTTCG
AGCATAAGGCTGACAGCCTGATTGCAAAATTCAAAGAAGCGGGCGGAACGGTCAGAGAGATTGATGTATGAGC
AGAGTCACCGCGATTATCTCCGCTCTGGTTATCTGCATCATCGTCTGCCTGTCATGGGCTGTTAATCATTACC
GTGATAACGCCATTACCTACAAAGCCCAGCGCGACAAAAATGCCAGAGAACTGAAGCTGGCGAACGCGGCAAT
TACTGACATGCAGATGCGTCAGCGTGATGTTGCTGCGCTCGATGCAAAATACACGAAGGAGTTAGCTGATGCT
AAAGCTGAAAATGATGCTCTGCGTGATGATGTTGCCGCTGGTCGTCGTCGGTTGCACATCAAAGCAGTCTGTC
AGTCAGTGCGTGAAGCCACCACCGCCTCCGGCGTGGATAATGCAGCCTCCCCCCGACTGGCAGACACCGCTGA
ACGGGATTATTTCACCCTCAGAGAGAGGCTGATCACTATGCAAAAACAACTGGAAGGAACCCAGAAGTATATT
AATGAGCAGTGCAGATAGAGTTGCCCATATCGATGGGCAACTCATGCAATTATTGTGAGCAATACACACGCGC
TTCCAGCGGAGTATAAATGCCTAAAGTAATAAAACCGAGCAATCCATTTACGAATGTTTGCTGGGTTTCTGTT
TTAACAACATTTTCTGCGCCGCCACAAATTTTGGCTGCATCGACAGTTTTCTTCTGCCCAATTCCAGAAACGA
 
AGAAATGATGGGTGATGGTTTCCTTTGGTGCTACTGCTGCCGGTTTGTTTTGAACAGTAAACGTCTGTTGAGC
ACATCCTGTAATAAGCAGGGCCAGCGCAGTAGCGAGTAGCATTTTTTTCATGGTGTTATTCCCGATGCTTTTT
GAAGTTCGCAGAATCGTATGTGTAGAAAATTAAACAAACCCTAAACAATGAGTTGAAATTTCATATTGTTAAT
ATTTATTAATGTATGTCAGGTGCGATGAATCGTCATTGTATTCCCGGATTAACTATGTCCACAGCCCTGACGG
GGAACTTCTCTGCGGGAGTGTCCGGGAATAATTAAAACGATGCACACAGGGTTTAGCGCGTACACGTATTGCA
TTATGCCAACGCCCCGGTGCTGACACGGAAGAAACCGGACGTTATGATTTAGCGTGGAAAGATTTGTGTAGTG
TTCTGAATGCTCTCAGTAAATAGTAATGAATTATCAAAGGTATAGTAATATCTTTTATGTTCATGGATATTTG
TAACCCATCGGAAAACTCCTGCTTTAGCAAGATTTTCCCTGTATTGCTGAAATGTGATTTCTCTTGATTTCAA
CCTATCATAGGACGTTTCTATAAGATGCGTGTTTCTTGAGAATTTAACATTTACAACCTTTTTAAGTCCTTTT
ATTAACACGGTGTTATCGTTTTCTAACACGATGTGAATATTATCTGTGGCTAGATAGTAAATATAATGTGAGA
CGTTGTGACGTTTTAGTTCAGAATAAAACAATTCACAGTCTAAATCTTTTCGCACTTGATCGAATATTTCTTT
AAAAATGGCAACCTGAGCCATTGGTAAAACCTTCCATGTGATACGAGGGCGCGTAGTTTGCATTATCGTTTTT
ATCGTTTCAATCTGGTCTGACCTCCTTGTGTTTTGTTGATGATTTATGTCAAATATTAGGAATGTTTTCACTT
AATAGTATTGGTTGCGTAACAAAGTGCGGTCCTGCTGGCATTCTGGAGGGAAATACAACCGACAGATGTATGT
AAGGCCAACGTGCTCAAATCTTCATACAGAAAGATTTGAAGTAATATTTTAACCGCTAGATGAAGAGCAAGCG
CATGGAGCGACAAAATGAATAAAGAACAATCTGCTGATGATCCCTCCGTGGATCTGATTCGTGTAAAAAATAT
GCTTAATAGCACCATTTCTATGAGTTACCCTGATGTTGTAATTGCATGTATAGAACATAAGGTGTCTCTGGAA
GCATTCAGAGCAATTGAGGCAGCGTTGGTGAAGCACGATAATAATATGAAGGATTATTCCCTGGTGGTTGACT
GATCACCATAACTGCTAATCATTCAAACTATTTAGTCTGTGACAGAGCCAACACGCAGTCTGTCACTGTCAGG
AAAGTGGTAAAACTGCAACTCAATTACTGCAATGCCCTCGTAATTAAGTGAATTTACAATATCGTCCTGTTCG
GAGGGAAGAACGCGGGATGTTCATTCTTCATCACTTTTAATTGATGTATATGCTCTCTTTTCTGACGTTAGTC
TCCGACGGCAGGCTTCAATGACCCAGGCTGAGAAATTCCCGGACCCTTTTTGCTCAAGAGCGATGTTAATTTG
TTCAATCATTTGGTTAGGAAAGCGGATGTTGCGGGTTGTTGTTCTGCGGGTTCTGTTCTTCGTTGACATGAGG
TTGCCCCGTATTCAGTGTCGCTGATTTGTATTGTCTGAAGTTGTTTTTACGTTAAGTTGATGCAGATCAATTA
ATACGATACCTGCGTCATAATTGATTATTTGACGTGGTTTGATGGCCTCCACGCACGTTGTGATATGTAGATG
ATAATCATTATCACTTTACGGGTCCTTTCCGGTGAAAAAAAAGGTACCAAAAAAAACATCGTCGTGAGTAGTG
AACCGTAAGCATGTAGGA
**SEQ ID NO: 14** 
GGTCAGGTTGTGGTGATTGGTCGCTGATGCAAAATGTTTTATGTGAAACCGCCTGCGGGCGGTTTTGTCATTT
ATGGAGCGTGAGGAATGGGTAAAGGAAGCAGTAAGGGGCATACCCCGCGCGAAGCGAAGGACAACCTAGGAGA
AGTCCACGCAGTTGCTGAGTGTGATCGATGCCATCAGCGAAGGGCCGATTGAAGGTCCGGTGGATGGCTTAAA
AAGCGTGCTGCTGAACAGTACGCCGGTGCTGGACACTGAGGGGAATACCAACATATCCGGTGTCACGGTGGTG
TTCCGGGCTGGTGAGCAGGAGCAGACTCCGCCGGAGGGATTTGAATCCTCCGGCTCCGAGACGGTGCTGGGTA
CGGAAGTGAAATATGACACGCCGATCACCCGCACCATTACGTCTGCAAACATCGACCGTCTGCGCTTTACCTT
CGGTGTACAGGCACTGGTGGAAACCACCTCAAAGGGTGACAGGAATCCGTCGGAAGTCCGCCTGCTGGTTCAG
ATACAACGTAACGGTGGCTGGGTGACGGAAAAAGACATCACCATTAAGGGCAAAACCACCTCGCAGTATCTGG
CCTCGGTGGTGATGGGTAACCTGCCGCCGCGCCCGTTTAATATCCGGATGCGCAGGATGACGCCGGACAGCAC
CACAGACCAGCTGCAGAACAAAACGCTCTGGTCGTCATACACTGAAATCATCGATGTGAAACAGTGCTACCCG
AACACGGCACTGGTCGGCGTGCAGGTGGACTCGGAGCAGTTCGGCAGCCAGCAGGTGAGCCGTAATTATCATC
TGCGCGGGCGTATTCTGCAGGTGCCGTCGAACTATAACCCGCAGACGCGGCAATACAGCGGTATCTGGGACGG
AACGTTTAAACCGGCATACAGCAACAACATGGCCTGGTGTCTGTGGGATATGCTGACCCATCCGCGCTACGGC
ATGGGGAAACGTCTTGGTGCGGCGGATGTGGATAAATGGGCGCTGTATGTCATCGGCCAGTACTGCGACCAGT
CAGTGCCGGACGGCTTTGGCGGCACGGAGCCGCGCATCACCTGTAATGCGTACCTGACCACACAGCGTAAGGC
GTGGGATGTGCTCAGCGATTTCTGCTCGGCGATGCGCTGTATGCCGGTATGGAACGGGCAGACGCTGACGTTC
GTGCAGGACCGACCGTCGGATAAGACGTGGACCTATAACCGCAGTAATGTGGTGATGCCGGATGATGGCGCGC
CGTTCCGCTACAGCTTCAGCGCCCTGAAGGACCGCCATAATGCCGTTGAGGTGAACTGGATTGACCCGAACAA
CGGCTGGGAGACGGCGACAGAGCTTGTTGAAGATACGCAGGCCATTGCCCGTTACGGTCGTAATGTTACGAAG
ATGGATGCCTTTGGCTGTACCAGCCGGGGGCAGGCACACCGCGCCGGGCTGTGGCTGATTAAAACAGAACTGC
TGGAAACGCAGACCGTGGATTTCAGCGTCGGCGCAGAAGGGCTTCGCCATGTACCGGGCGATGTTATTGAAAT
CTGCGATGATGACTATGCCGGTATCAGCACCGGTGGTCGTGTGCTGGCGGTGAACAGCCAGACCCGGACGCTG
ACGCTCGACCGTGAAATCACGCTGCCATCCTCCGGTACCGCGCTGATAAGCCTGGTTGACGGAAGTGGCAATC
CGGTCAGCGTGGAGGTTCAGTCCGTCACCGACGGCGTGAAGGTAAAAGTGAGCCGTGTTCCTGACGGTGTTGC
TGAATACAGCGTATGGGAGCTGAAGCTGCCGACGCTGCGCCAGCGACTGTTCCGCTGCGTGAGTATCCGTGAG
AACGACGACGGCACGTATGCCATCACCGCCGTGCAGCATGTGCCGGAAAAAGAGGCCATCGTGGATAACGGGG
CGCACTTTGACGGCGAACAGAGTGGCACGGTGAATGGTGTCACGCCGCCAGCGGTGCAGCACCTGACCGCAGA
AGTCACTGCAGACAGCGGGGAATATCAGGTGCTGGCGCGATGGGACACACCGAAGGTGGTGAAGGGCGTGAGT
TTCCTGCTCCGTCTGACCGTAACAGCGGACGACGGCAGTGAGCGGCTGGTCAGCACGGCCCGGACGACGGAAA
CCACATACCGCTTCACGCAACTGGCGCTGGGGAACTACAGGCTGACAGTCCGGGCGGTAAATGCGTGGGGGCA
GCAGGGCGATCCGGCGTCGGTATCGTTCCGGATTGCCGCACCGGCAGCACCGTCGAGGATTGAGCTGACGCCG
GGCTATTTTCAGATAACCGCCACGCCGCATCTTGCCGTTTATGACCCGACGGTACAGTTTGAGTTCTGGTTCT
CGGAAAAGCAGATTGCGGATATCAGACAGGTTGAAACCAGCACGCGTTATCTTGGTACGGCGCTGTACTGGAT
AGCCGCCAGTATCAATATCAAACCGGGCCATGATTATTACTTTTATATCCGCAGTGTGAACACCGTTGGCAAA
 
TCGGCATTCGTGGAGGCCGTCGGTCGGGCGAGCGATGATGCGGAAGGTTACCTGGATTTTTTCAAAGGCAAGA
TAACCGAATCCCATCTCGGCAAGGAGCTGCTGGAAAAAGTCGAGCTGACGGAGGATAACGCCAGCAGACTGGA
GGAGTTTTCGAAAGAGTGGAAGGATGCCAGTGATAAGTGGAATGCCATGTGGGCTGTCAAAATTGAGCAGACC
AAAGACGGCAAACATTATGTCGCGGGTATTGGCCTCAGCATGGAGGACACGGAGGAAGGCAAACTGAGCCAGT
TTCTGGTTGCCGCCAATCGTATCGCATTTATTGACCCGGCAAACGGGAATGAAACGCCGATGTTTGTGGCGCA
GGGCAACCAGATATTCATGAACGACGTGTTCCTGAAGCGCCTGACGGCCCCCACCATTACCAGCGGCGGCAAT
CCTCCGGCCTTTTCCCTGACACCGGACGGAAAGCTGACCGCTAAAAATGCGGATATCAGTGGCAGTGTGAATG
CGAACTCCGGGACGCTCAGTAATGTGACGATAGCTGAAAACTGTACGATAAACGGTACGCTGAGGGCGGAAAA
AATCGTCGGGGACATTGTAAAGGCGGCGAGCGCGGCTTTTCCGCGCCAGCGTGAAAGCAGTGTGGACTGGCCG
TCAGGTACCCGTACTGTCACCGTGACCGATGACCATCCTTTTGATCGCCAGATAGTGGTGCTTCCGCTGACGT
TTCGCGGAAGTAAGCGTACTGTCAGCGGCAGGACAACGTATTCGATGTGTTATCTGAAAGTACTGATGAACGG
TGCGGTGATTTATGATGGCGCGGCGAACGAGGCGGTACAGGTGTTCTCCCGTATTGTTGACATGCCAGCGGGT
CGGGGAAACGTGATCCTGACGTTCACGCTTACGTCCACACGGCATTCGGCAGATATTCCGCCGTATACGTTTG
CCAGCGATGTGCAGGTTATGGTGATTAAGAAACAGGCGCTGGGCATCAGCGTGGTCTGAGTGTGTTACAGAGG
TTCGTCCGGGAACGGGCGTTTTATTATAAAACAGTGAGAGGTGAACGATGCGTAATGTGTGTATTGCCGTTGC
TGTCTTTGCCGCACTTGCGGTGACAGTCACTCCGGCCCGTGCGGAAGGTGGACATGGTACGTTTACGGTGGGC
TATTTTCAAGTGAAACCGGGTACATTGCCGTCGTTGTCGGGCGGGGATACCGGTGTGAGTCATCTGAAAGGGA
TTAACGTGAAGTACCGTTATGAGCTGACGGACAGTGTGGGGGTGATGGCTTCCCTGGGGTTCGCCGCGTCGAA
AAAGAGCAGCACAGTGATGACCGGGGAGGATACGTTTCACTATGAGAGCCTGCGTGGACGTTATGTGAGCGTG
ATGGCCGGACCGGTTTTACAAATCAGTAAGCAGGTCAGTGCGTACGCCATGGCCGGAGTGGCTCACAGTCGGT
GGTCCGGCAGTACAATGGATTACCGTAAGACGGAAATCACTCCCGGGTATATGAAAGAGACGACCACTGCCAG
GGACGAAAGTGCAATGCGGCATACCTCAGTGGCGTGGAGTGCAGGTATACAGATTAATCCGGCAGCGTCCGTC
GTTGTTGATATTGCTTATGAAGGCTCCGGCAGTGGCGACTGGCGTACTGACGGATTCATCGTTGGGGTCGGTT
ATAAATTCTGATTAGCCAGGTAACACAGTGTTATGACAGCCCGCCGGAACCGGTGGGCTTTTTTGTGGGGTGA
ATATGGCAGTAAAGATTTCAGGAGTCCTGAAAGACGGCACAGGAAAACCGGTACAGAACTGCACCATTCAGCT
GAAAGCCAGACGTAACAGCACCACGGTGGTGGTGAACACGGTGGGCTCAGAGAATCCGGATGAAGCCGGGCGT
TACAGCATGGATGTGGAGTACGGTCAGTACAGTGTCATCCTGCAGGTTGACGGTTTTCCACCATCGCACGCCG
GGACCATCACCGTGTATGAAGATTCACAACCGGGGACGCTGAATGATTTTCTCTGTGCCATGACGGAGGATGA
TGCCCGGCCGGAGGTGCTGCGTCGTCTTGAACTGATGGTGGAAGAGGTGGCGCGTAACGCGTCCGTGGTGGCA
CAGAGTACGGCAGACGCGAAGAAATCAGCCGGCGATGCCAGTGCATCAGCTGCTCAGGTCGCGGCCCTTGTGA
CTGATGCAACTGACTCAGCACGCGCCGCCAGCACGTCCGCCGGACAGGCTGCATCGTCAGCTCAGGAAGCGTC
CTCCGGCGCAGAAGCGGCATCAGCAAAGGCCACTGAAGCGGAAAAAAGTGCCGCAGCCGCAGAGTCCTCAAAA
AACGCGGCGGCCACCAGTGCCGGTGCGGCGAAAACGTCAGAAACGAATGCTGCAGCGTCACAACAATCAGCCG
CCACGTCTGCCTCCACCGCGGCCACGAAAGCGTCAGAGGCCGCCACTTCAGCACGAGATGCGGTGGCCTCAAA
AGAGGCAGCAAAATCATCAGAAACGAACGCATCATCAAGTGCCGGTCGTGCAGCTTCCTCGGCAACGGCGGCA
GAAAATTCTGCCAGGGCGGCAAAAACGTCCGAGACGAATGCCAGGTCATCTGAAACAGCAGCGGAACGGAGCG
CCTCTGCCGCGGCAGACGCAAAAACAGCGGCGGCGGGGAGTGCGTCAACGGCATCCACGAAGGCGACAGAGGC
TGCGGGAAGTGCGGTATCAGCATCGCAGAGCAAAAGTGCGGCAGAAGCGGCGGCAATACGTGCAAAAAATTCG
GCAAAACGTGCAGAAGATATAGCTTCAGCTGTCGCGCTTGAGGATGCGGACACAACGAGAAAGGGGATAGTGC
AGCTCAGCAGTGCAACCAACAGCACGTCTGAAACGCTTGCTGCAACGCCAAAGGCGGTTAAGGTGGTAATGGA
TGAAACGAACAGAAAAGCCCACTGGACAGTCCGGCACTGACCGGAACGCCAACAGCACCAACCGCGCTCAGGG
GAACAAACAATACCCAGATTGCGAACACCGCTTTTGTACTGGCCGCGATTGCAGATGTTATCGACGCGTCACC
TGACGCACTGAATACGCTGAATGAACTGGCCGCAGCGCTCGGGAATGATCCAGATTTTGCTACCACCATGACT
AACGCGCTTGCGGGTAAACAACCGAAGAATGCGACACTGACGGCGCTGGCAGGGCTTTCCACGGCGAAAAATA
AATTACCGTATTTTGCGGAAAATGATGCCGCCAGCCTGACTGAACTGACTCAGGTTGGCAGGGATATTCTGGC
AAAAAATTCCGTTGCAGATGTTCTTGAATACCTTGGGGCCGGTGAGAATTCGGCCTTTCCGGCAGGTGCGCCG
ATCCCGTGGCCATCAGATATCGTTCCGTCTGGCTACGTCCTGATGCAGGGGCAGGCGTTTGACAAATCAGCCT
ACCCAAAACTTGCTGTCGCGTATCCATCGGGTGTGCTTCCTGATATGCGAGGCTGGACAATCAAGGGGAAACC
CGCCAGCGGTCGTGCTGTATTGTCTCAGGAACAGGATGGAATTAAGTCGCACACCCACAGTGCCAGTGCATCC
GGTACGGATTTGGGGACGAAAACCACATCGTCGTTTGATTACGGGACGAAAACAACAGGCAGTTTCGATTACG
GCACCAAATCGACGAATAACACGGGGGCTCATGCTCACAGTCTGAGCGGTTCAACAGGGGCCGCGGGTGCTCA
TGCCCACACAAGTGGTTTAAGGATGAACAGTTCTGGCTGGAGTCAGTATGGAACAGCAACCATTACAGGAAGT
TTATCCACAGTTAAAGGAACCAGCACACAGGGTATTGCTTATTTATCGAAAACGGACAGTCAGGGCAGCCACA
GTCACTCATTGTCCGGTACAGCCGTGAGTGCCGGTGCACATGCGCATACAGTTGGTATTGGTGCGCACCAGCA
TCCGGTTGTTATCGGTGCTCATGCCCATTCTTTCAGTATTGGTTCACACGGACACACCATCACCGTTAACGCT
GCGGGTAACGCGGAAAACACCGTCAAAAACATTGCATTTAACTATATTGTGAGGCTTGCATAATGGCATTCAG
AATGAGTGAACAACCACGGACCATAAAAATTTATAATCTGCTGGCCGGAACTAATGAATTTATTGGTGAAGGT
GACGCATATATTCCGCCTCATACCGGTCTGCCTGCAAACAGTACCGATATTGCACCGCCAGATATTCCGGCTG
GCTTTGTGGCTGTTTTCAACAGTGATGAGGCATCGTGGCATCTCGTTGAAGACCATCGGGGTAAAACCGTCTA
TGACGTGGCTTCCGGCGACGCGTTATTTATTTCTGAACTCGGTCCGTTACCGGAAAATTTTACCTGGTTATCG
CCGGGAGGGGAATATCAGAAGTGGAACGGCACAGCCTGGGTGAAGGATACGGAAGCAGAAAAACTGTTCCGGA
TCCGGGAGGCGGAAGAAACAAAAAAAAGCCTGATGCAGGTAGCCAGTGAGCATATTGCGCCGCTTCAGGATGC
TGCAGATCTGGAAATTGCAACGAAGGAAGAAACCTCGTTGCTGGAAGCCTGGAAGAAGTATCGGGTGTTGCTG
 
AACCGTGTTGATACATCAACTGCACCTGATATTGAGTGGCCTGCTGTCCCTGTTATGGAGTAATCGTTTTGTG
ATATGCCGCAGAAACGTTGTATGAAATAACGTTCTGCGGTTAGTTAGTATATTGTAAAGCTGAGTATTGGTTT
ATTTGGCGATTATTATCTTCAGGAGAATAATGGAAGTTCTATGACTCAATTGTTCATAGTGTTTACATCACCG
CCAATTGCTTTTAAGACTGAACGCATGAAATATGGTTTTTCGTCATGTTTTGAGTCTGCTGTTGATATTTCTA
AAGTCGGTTTTTTTTCTTCGTTTTCTCTAACTATTTTCCATGAAATACATTTTTGATTATTATTTGAATCAAT
TCCAATTACCTGAAGTCTTTCATCTATAATTGGCATTGTATGTATTGGTTTATTGGAGTAGATGCTTGCTTTT
CTGAGCCATAGCTCTGATATCCAAATGAAGCCATAGGCATTTGTTATTTTGGCTCTGTCAGCTGCATAACGCC
AAAAAATATATTTATCTGCTTGATCTTCAAATGTTGTATTGATTAAATCAATTGGATGGAATTGTTTATCATA
AAAAATTAATGTTTGAATGTGATAACCGTCCTTTAAAAAAGTCGTTTCTGCAAGCTTGGCTGTATAGTCAACT
AACTCTTCTGTCGAAGTGATATTTTTAGGCTTATCTACCAGTTTTAGACGCTCTTTAATATCTTCAGGAATTA
TTTTATTGTCATATTGTATCATGCTAAATGACAATTTGCTTATGGAGTAATCTTTTAATTTTAAATAAGTTAT
TCTCCTGGCTTCATCAAATAAAGAGTCGAATGATGTTGGCGAAATCACATCGTCACCCATTGGATTGTTTATT
TGTATGCCAAGAGAGTTACAGCAGTTATACATTCTGCCATAGATTATAGCTAAGGCATGTAATAATTCGTAAT
CTTTTAGCGTATTAGCGACCCATCGTCTTTCTGATTTAATAATAGATGATTCAGTTAAATATGAAGGTAATTT
CTTTTGTGCAAGTCTGACTAACTTTTTTATACCAATGTTTAACATACTTTCATTTGTAATAAACTCAATGTCA
TTTTCTTCAATGTAAGATGAAATAAGAGTAGCCTTTGCCTCGCTATACATTTCTAAATCGCCTTGTTTTTCTA
TCGTATTGCGAGAATTTTTAGCCCAAGCCATTAATGGATCATTTTTCCATTTTTCAATAACATTATTGTTATA
CCAAATGTCATATCCTATAATCTGGTTTTTGTTTTTTTGAATAATAAATGTTACTGTTCTTGCGGTTTGGAGG
AATTGATTCAAATTCAAGCGAAATAATTCAGGGTCAAAATATGTATCAATGCAGCATTTGAGCAAGTGCGATA
AATCTTTAAGTCTTCTTTCCCATGGTTTTTTAGTCATAAAACTCTCCATTTTGATAGGTTGCATGCTAGATGC
TGATATATTTTAGAGGTGATAAAATTAACTGCTTAACTGTCAATGTAATACAAGTTGTTTGATCTTTGCAATG
ATTCTTATCAGAAACCATATAGTAAATTAGTTACACAGGAAATTTTTAATATTATTATTATCATTCATTATGT
ATTAAAATTAGAGTTGTGGCTTGGCTCTGCTAACACGTTGCTCATAGGAGATATGGTAGAGCCGCAGACACGT
CGTATGCAGGAACGTGCTGCGGCTGGCTGGTGAACTTCCGATAGTGCGGGTGTTGAATGATTTCCAGTTGCTA
CCGATTTTACATATTTTTTGCATGAGAGAATTTGTACCACCTCCCACCGACCATCTATGACTGTACGCCACTG
TCCCTAGGACTGCTATGTGCCGGAGCGGACATTACAAACGTCCTTCTCGGTGCATGCCACTGTTGCCAATGAC
CTGCCTAGGAATTGGTTAGCAAGTTACTACCGGATTTTGTAAAAACAGCCCTCCTCATATAAAAAGTATTCGT
TCACTTCCGATAAGCGTCGTAATTTTCTATCTTTCATCATATTCTAGATCCCTCTGAAAAAATCTTCCGAGTT
TGCTAGGCACTGATACATAACTCTTTTCCAATAATTGGGGAAGTCATTCAAATCTATAATAGGTTTCAGATTT
GCTTCAATAAATTCTGACTGTAGCTGCTGAAACGTTGCGGTTGAACTATATTTCCTTATAACTTTTACGAAAG
AGTTTCTTTGAGTAATCACTTCACTCAAGTGCTTCCCTGCCTCCAAACGATACCTGTTAGCAATATTTAATAG
CTTGAAATGATGAAGAGCTCTGTGTTTGTCTTCCTGCCTCCAGTTCGCCGGGCATTCAACATAAAAACTGATA
GCACCCGGAGTTCCGGAAACGAAATTTGCATATACCCATTGCTCACGAAAAAAAATGTCCTTGTCGATATAGG
GATGAATCGCTTGGTGTACCTCATCTACTGCGAAAACTTGACCTTTCTCTCCCATATTGCAGTCGCGGCACGA
TGGAACTAAATTAATAGGCATCACCGAAAATTCAGGATAATGTGCAATAGGAAGAAAATGATCTATATTTTTT
GTCTGTCCTATATCACCACAAAATGGACATTTTTCACCTGATGAAACAAGCATGTCATCGTAATATGTTCTAG
CGGGTTTGTTTTTATCTCGGAGATTATTTTCATAAAGCTTTTCTAATTTAACCTTTGTCAGGTTACCAACTAC
TAAGGTTGTAGGCTCAAGAGGGTGTGTCCTGTCGTAGGTAAATAACTGACCTGTCGAGCTTAATATTCTATAT
TGTTGTTCTTTCTGCAAAAAAGTGGGGAAGTGAGTAATGAAATTATTTCTAACATTTATCTGCATCATACCTT
CCGAGCATTTATTAAGCATTTCGCTATAAGTTCTCGCTGGAAGAGGTAGTTTTTTCATTGTACTTTACCTTCA
TCTCTGTTCATTATCATCGCTTTTAAAACGGTTCGACCTTCTAATCCTATCTGACCATTATAATTTTTTAGAA
TGGTTTCATAAGAAAGCTCTGAATCAACGGACTGCGATAATAAGTGGTAGGA
**SEQ ID NO: 15**
   GGTTAAACACCCAAGCAGACGCCTTTGAGGCGAGCGGTCAA
**SEQ ID NO: 16** 
/5SpC3//iSpC3//iSpC3//iSpC3//iSpC3//iSpC3//iSpC3//iSpC3//iSpC3//iSpC3//iS
pC3//iSpC3//iSpC3//iSpC3//iSpC3//iSpC3//iSpC3//iSpC3//iSpC3//iSpC3/TTTTTT
TTTT/iSpC3//iSpC3//iSpC3//iSpC3//iSpC3//iSpC3//iSpC3//iSpC3//iSpC3//iSpC3
/TTTTTTTTTTGGCGTCTGCTTGGGTGTTTAACCT
**SEQ ID NO: 20** 
GCCATCAGATTGTGTTTGTTAGTCGCTGCCATCAGATTGTGTTTGTTAGTCGCTTTTTTTTTTTGGAATTTTT
TTTTTGGAATTTTTTTTTTGCGCTAACAACCTCCTGCCGTTTTGCCCGTGCATATCGGTCACGAACAAATCTG
ATTACTAAACACAGTAGCCTGGATTTGTTCTATCAGTAATCGACCTTATTCCTAATTAAATAGAGCAAATCCC
CTTATTGGGGGTAAGACATGAAGATGCCAGAAAAACATGACCTGTTGGCCGCCATTCTCGCGGCAAAGGAACA
AGGCATCGGGGCAATCCTTGCGTTTGCAATGGCGTACCTTCGCGGCAGATATAATGGCGGTGCGTTTACAAAA
ACAGTAATCGACGCAACGATGTGCGCCATTATCGCCTAGTTCATTCGTGACCTTCTCGACTTCGCCGGACTAA
GTAGCAATCTCGCTTATATAACGAGCGTGTTTATCGGCTACATCGGTACTGACTCGATTGGTTCGCTTATCAA
ACGCTTCGCTGCTAAAAAAGCCGGAGTAGAAGATGGTAGAAATCAATAATCAACGTAAGGCGTTCCTCGATAT
GCTGGCGTGGTCGGAGGGAACTGATAACGGACGTCAGAAAACCAGAAATCATGGTTATGACGTCATTGTAGGC
 
GGAGAGCTATTTACTGATTACTCCGATCACCCTCGCAAACTTGTCACGCTAAACCCAAAACTCAAATCAACAG
GCGCCGGACGCTACCAGCTTCTTTCCCGTTGGTGGGATGCCTACCGCAAGCAGCTTGGCCTGAAAGACTTCTC
TCCGAAAAGTCAGGACGCTGTGGCATTGCAGCAGATTAAGGAGCGTGGCGCTTTACCTATGATTGATCGTGGT
GATATCCGTCAGGCAATCGACCGTTGCAGCAATATCTGGGCTTCACTGCCGGGCGCTGGTTATGGTCAGTTCG
AGCATAAGGCTGACAGCCTGATTGCAAAATTCAAAGAAGCGGGCGGAACGGTCAGAGAGATTGATGTATGAGC
AGAGTCACCGCGATTATCTCCGCTCTGGTTATCTGCATCATCGTCTGCCTGTCATGGGCTGTTAATCATTACC
GTGATAACGCCATTACCTACAAAGCCCAGCGCGACAAAAATGCCAGAGAACTGAAGCTGGCGAACGCGGCAAT
TACTGACATGCAGATGCGTCAGCGTGATGTTGCTGCGCTCGATGCAAAATACACGAAGGAGTTAGCTGATGCT
AAAGCTGAAAATGATGCTCTGCGTGATGATGTTGCCGCTGGTCGTCGTCGGTTGCACATCAAAGCAGTCTGTC
AGTCAGTGCGTGAAGCCACCACCGCCTCCGGCGTGGATAATGCAGCCTCCCCCCGACTGGCAGACACCGCTGA
ACGGGATTATTTCACCCTCAGAGAGAGGCTGATCACTATGCAAAAACAACTGGAAGGAACCCAGAAGTATATT
AATGAGCAGTGCAGATAGAGTTGCCCATATCGATGGGCAACTCATGCAATTATTGTGAGCAATACACACGCGC
TTCCAGCGGAGTATAAATGCCTAAAGTAATAAAACCGAGCAATCCATTTACGAATGTTTGCTGGGTTTCTGTT
TTAACAACATTTTCTGCGCCGCCACAAATTTTGGCTGCATCGACAGTTTTCTTCTGCCCAATTCCAGAAACGA
AGAAATGATGGGTGATGGTTTCCTTTGGTGCTACTGCTGCCGGTTTGTTTTGAACAGTAAACGTCTGTTGAGC
ACATCCTGTAATAAGCAGGGCCAGCGCAGTAGCGAGTAGCATTTTTTTCATGGTGTTATTCCCGATGCTTTTT
GAAGTTCGCAGAATCGTATGTGTAGAAAATTAAACAAACCCTAAACAATGAGTTGAAATTTCATATTGTTAAT
ATTTATTAATGTATGTCAGGTGCGATGAATCGTCATTGTATTCCCGGATTAACTATGTCCACAGCCCTGACGG
GGAACTTCTCTGCGGGAGTGTCCGGGAATAATTAAAACGATGCACACAGGGTTTAGCGCGTACACGTATTGCA
TTATGCCAACGCCCCGGTGCTGACACGGAAGAAACCGGACGTTATGATTTAGCGTGGAAAGATTTGTGTAGTG
TTCTGAATGCTCTCAGTAAATAGTAATGAATTATCAAAGGTATAGTAATATCTTTTATGTTCATGGATATTTG
TAACCCATCGGAAAACTCCTGCTTTAGCAAGATTTTCCCTGTATTGCTGAAATGTGATTTCTCTTGATTTCAA
CCTATCATAGGACGTTTCTATAAGATGCGTGTTTCTTGAGAATTTAACATTTACAACCTTTTTAAGTCCTTTT
ATTAACACGGTGTTATCGTTTTCTAACACGATGTGAATATTATCTGTGGCTAGATAGTAAATATAATGTGAGA
CGTTGTGACGTTTTAGTTCAGAATAAAACAATTCACAGTCTAAATCTTTTCGCACTTGATCGAATATTTCTTT
AAAAATGGCAACCTGAGCCATTGGTAAAACCTTCCATGTGATACGAGGGCGCGTAGTTTGCATTATCGTTTTT
ATCGTTTCAATCTGGTCTGACCTCCTTGTGTTTTGTTGATGATTTATGTCAAATATTAGGAATGTTTTCACTT
AATAGTATTGGTTGCGTAACAAAGTGCGGTCCTGCTGGCATTCTGGAGGGAAATACAACCGACAGATGTATGT
AAGGCCAACGTGCTCAAATCTTCATACAGAAAGATTTGAAGTAATATTTTAACCGCTAGATGAAGAGCAAGCG
CATGGAGCGACAAAATGAATAAAGAACAATCTGCTGATGATCCCTCCGTGGATCTGATTCGTGTAAAAAATAT
GCTTAATAGCACCATTTCTATGAGTTACCCTGATGTTGTAATTGCATGTATAGAACATAAGGTGTCTCTGGAA
GCATTCAGAGCAATTGAGGCAGCGTTGGTGAAGCACGATAATAATATGAAGGATTATTCCCTGGTGGTTGACT
GATCACCATAACTGCTAATCATTCAAACTATTTAGTCTGTGACAGAGCCAACACGCAGTCTGTCACTGTCAGG
AAAGTGGTAAAACTGCAACTCAATTACTGCAATGCCCTCGTAATTAAGTGAATTTACAATATCGTCCTGTTCG
GAGGGAAGAACGCGGGATGTTCATTCTTCATCACTTTTAATTGATGTATATGCTCTCTTTTCTGACGTTAGTC
TCCGACGGCAGGCTTCAATGACCCAGGCTGAGAAATTCCCGGACCCTTTTTGCTCAAGAGCGATGTTAATTTG
TTCAATCATTTGGTTAGGAAAGCGGATGTTGCGGGTTGTTGTTCTGCGGGTTCTGTTCTTCGTTGACATGAGG
TTGCCCCGTATTCAGTGTCGCTGATTTGTATTGTCTGAAGTTGTTTTTACGTTAAGTTGATGCAGATCAATTA
ATACGATACCTGCGTCATAATTGATTATTTGACGTGGTTTGATGGCCTCCACGCACGTTGTGATATGTAGATG
ATAATCATTATCACTTTACGGGTCCTTTCCGGTGAAAAAAAAGGTACCAAAAAAAACATCGTCGTGAGTAGTG
AACCGTAAGCATGTAGGA
**SEQ ID NO: 21**
   /5BiotinTEG/TTTTTTTTTT/iSp18/AATGTACTTCGTTCAGTTACGTATTGCT
**SEQ ID NO: 22** 
/5Phos/GCAATACGTAACTGAACGAAGT/iBNA-A//iBNA-MeC//iBNA-A//iBNA-T//iBNA-
T/TTTGAGGCGAGCGGTCAATTTTTTTTTTTTTTTTTTTTTTTTT
**SEQ ID NO: 24** 
/5Phos/TGCAATACGTAACTGAACGAAGTACATTTTTTTGAAGATAGAGCGATTTTTTTTTTTTTTTTGTAC
TTCGTTCAGTTACGTATTGCATCCT
**SEQ ID NO: 25** 
/5Phos/TGCAATACGTAACTGAACGAAGTACATTTTTTTGAAGATAGAGCGATTTTTTTTTTTTTTTTGTAC
TTCGTTCAGTTACGTATTGCAT
**SEQ ID NO: 26** 
/5Phos/TGCAATACGTAACTGAACGAAGTACATTTTTTUGAAGATAGAGCGATTTTT/iFluorT//iFluo
rT//iFluorT/TTTTTTTTTGTACTTCGTTCAGTTACGTATTGCATCCT
**SEQ ID NO: 27**
   /5BNA-T//iBNA-MeC//iBNA-G//iBNA-MeC//iBNA-T/CTATCTTC
**SEQ ID NO: 28** 
GTTATTCAAGACTTCTTTAATACACTTTTTTTTTT/iSp18/AATGTACTTCGTTCAGTTACGTATTGCTTTG
GCGTCTGCTTGGGTGTTTAACCT
**SEQ ID NO: 30**
   GCAATACGTAACTGAACGAAGT/iBNA-A//iBNA-MeC//iBNA-A//iBNA-T//3BNA-T/
**SEQ ID NO: 32**
   GTGTATTAAAGAAGTCTTGAATAAC
**SEQ ID NO: 33** 
/5Phos/GGTTAAACACCCAAGCAGACGCCTTTGAGGCGAGCGGTCAA/iSpC3//iSpC3//iSpC3//iSp
C3//iSpC3//iSpC3//iSpC3//iSpC3//iSpC3//iSpC3//iSpC3//iSpC3//iSpC3//iSpC3/
/iSpC3//iSpC3//iSpC3//iSpC3//iSpC3//iSpC3//iSpC3//iSpC3//iSpC3//iSpC3//iS
pC3//iSpC3//iSpC3//iSpC3//iSpC3//3SpC3/
**SEQ ID NO: 34** 
GTTATTCAAGACTTCTTTAATACACTTTTTTTTTT/iSp9/AATGTACTTCGTTCAGTTACGTATTGCTTTGG
CGTCTGCTTGGGTGTTTAACCT
**SEQ ID NO: 35** 
/5Phos/AACACCCAAGCAGACGCCTTT/iSpC3//iSpC3//iSpC3//iSpC3//iSpC3//iSpC3//iS
pC3//iSpC3//iSpC3//iSpC3//iSpC3//iSpC3//iSpC3//iSpC3//iSpC3//iSpC3//iSpC3
//iSpC3//iSpC3//iSpC3//iSpC3//iSpC3//iSpC3//iSpC3//iSpC3//iSpC3//iSpC3//i
SpC3//iSpC3//3SpC3/
**SEQ ID NO: 36**
   TTTGCAATACGTAACTGAACGAAGT/iBNA-A//iBNA-MeC//iBNA-A//iBNA-T//3BNA-T/
**SEQ ID NO: 37**
   GTGTATTAAAGAAGTCTTGAATAAC/iSpC3//iSpC3//iSpC3/GAGGCGAGCGGT/3ddC/
**SEQ ID NO: 38**
   /5Phos/GTTTTCGCATTTATCGTGAAACGCTTTCGCGTTTTTCGTGCGCCGCTTCA
**SEQ ID NO: 39**
   /5Phos/IIIIITGAAGCGGCGCACGAAAAACGCGAAAGCGTTTCACGATAAATGCGAAAACAGGTTA
**SEQ ID NO: 40** 
/5Phos/CGTCCTGTCGCTGTGTCTCG/iSpC3//iSpC3//iSpC3//iSpC3//iSpC3//iSpC3//iSp
C3//iSpC3//iSpC3//iSpC3/TTTTTTTTTT/iSp18/AATGTACTTCGTTCAGTTACGTATTGCTAGAG
CCGAGACACAGCGACAGGACGT
**SEQ ID NO: 41**
   GCAATACGTAACTGAACGAAGT/iBNA-A//iBNA-MeC//iBNA-A//iBNA-T//3BNA-T/
**SEQ ID NO: 42** 
333333333333333333333333333333CTACATCTCCTTATTCGCTGCACTTTTTTTTTT8CCTGTACTT
CGTTCAGTTACGTATTGCT-N3
**SEQ ID NO: 43**
   GTGCAGCGAATAAGGAGATGTAGTTTGAGGCGAGCGGTCAA
**SEQ ID NO: 44**
   GGTTAAACACCCAAGCAAGCAATACGTAACTGAACmGmAmAmGmUmAmCmAmGmG
**SEQ ID NO: 45**
   GCAATACGTAACTGAACGAAGTACAGGTTT
**SEQ ID NO: 46** 
/5Phos/GGTTAAACACCCAAGCAGACGCCTTT/iSpC3//iSpC3//iSpC3//iSpC3//iSpC3//iSpC
3//iSpC3//iSpC3//iSpC3//iSpC3//iSpC3//iSpC3//iSpC3//iSpC3//iSpC3//iSpC3//
 
iSpC3//iSpC3//iSpC3//iSpC3//iSpC3//iSpC3//iSpC3//iSpC3//iSpC3//iSpC3//iSp
C3//iSpC3//iSpC3//3SpC3/
**SEQ ID NO: 47**
   /5Phos/GTTTTCGCATTTATCGTGAAACGCTTTCGCGTTTTTCGTGCGCCGCTTCA

## Claims

1. A method of characterising a polynucleotide in a sample, the method comprising:
(i) contacting a nanopore with a polynucleotide;
(ii a) taking measurements as a first part of the polynucleotide moves freely with respect to the nanopore under an applied force;
(ii b) assessing one or more properties of the first part of the polynucleotide;
(iii) (a) where the first part of the polynucleotide has one or more desired properties, controlling the movement of a second part of the polynucleotide with respect to the nanopore using a polynucleotide binding protein and taking measurements as the second part of the polynucleotide moves with respect to the nanopore to determine one or more characteristics of the polynucleotide, thereby characterising the polynucleotide; or (b) where the first part of the polynucleotide does not have one or more desired properties, rejecting the polynucleotide.

2. A method according to claim 1 wherein, when the first part of the polynucleotide does not have one or more desired properties, step (iii) comprises repeating steps (i), (ii a) and (ii b) with further polynucleotides from the sample until a polynucleotide having a first part having one or more desired properties is identified.

3. A method according to claim 1 or 2, wherein said one or more desired properties are selected from the approximate length of the first part of the polynucleotide, the structure of the first part of the polynucleotide, and the composition of the first part of the polynucleotide;
preferably wherein assessing said one or more properties of the polynucleotide comprises determining the approximate length of the first part of the polynucleotide;
preferably wherein assessing the approximate length of the first part of the polynucleotide comprises allowing the first part of the polynucleotide to translocate freely through or across the nanopore under an applied potential and determining the time taken for the first part of the polynucleotide to translocate through or across the nanopore.

4. A method according to any one of the preceding claims, wherein the polynucleotide is a double-stranded polynucleotide comprising a first strand connected to a second strand by a hairpin or hairpin adapter;
preferably wherein prior to step (i) a polynucleotide binding protein is bound to and/or stalled at the hairpin or hairpin adapter.

5. A method according to any one of the preceding claims, wherein determining one or more characteristics of the polynucleotide comprises determining the sequence of the polynucleotide.

6. A method according to any one of the preceding claims, comprising:
(i) contacting a nanopore with a polynucleotide having a polynucleotide binding protein capable of controlling the movement of the polynucleotide stalled thereon;
(ii a) determining the time taken for a first part of the polynucleotide to move freely with respect to the nanopore under an applied force; and
(ii b) determining the approximate length of the first part of the polynucleotide.

7. A method according to any one of the preceding claims, wherein an adapter is attached to one or both ends of the polynucleotide prior to step (i);
preferably wherein the polynucleotide comprises a single stranded leader sequence at one end and has a polynucleotide binding protein bound thereto at the other end on the same strand of the polynucleotide or to an adapter attached to the other end of the same strand of the polynucleotide.

8. A method according to any one of the preceding claims, wherein prior to step (i) a polynucleotide binding protein capable of controlling the movement of the polynucleotide is stalled on the polynucleotide or on an adapter attached to the polynucleotide.

9. A method according to any one of the preceding claims, wherein in step (ii a) the first part of the polynucleotide moves freely with respect to the nanopore in a first direction relative to the applied force, and in step (iii) the polynucleotide binding protein controls the movement of the second part of the polynucleotide with respect to the nanopore in a second direction relative to the applied force.

10. A method according to any one of the preceding claims, wherein step (i) comprises contacting the nanopore with a first end of the polynucleotide or an adapter attached to the first end of the polynucleotide and the polynucleotide binding protein is bound to a second end of the polynucleotide or to an adapter attached to the second end of the polynucleotide.

11. A method according to any one of the preceding claims, wherein:
a) step (i) comprises contacting the nanopore with a leader sequence at the first end of the polynucleotide and the polynucleotide binding protein is stalled at a second end of the polynucleotide or on an adapter attached to the second end of the polynucleotide; and
b) the first part of the polynucleotide is the part between the leader sequence and the polynucleotide binding protein and the second part of the polynucleotide is the same as the first part of the polynucleotide; and
c) the polynucleotide binding protein is orientated on the polynucleotide such that the polynucleotide binding protein controls the movement of the second part of the polynucleotide with respect to the nanopore against the applied force.

12. A method according to any one of claims 1 to 23, wherein the polynucleotide is single stranded or double stranded.;
preferably wherein:
i) the polynucleotide is single-stranded; the polynucleotide comprises a leader sequence, wherein the leader sequence is located at the first end of the polynucleotide or is comprised in an adapter attached to the first end of the polynucleotide; and the polynucleotide binding protein is stalled at a second end of the polynucleotide or is stalled on an adapter at the second end of the polynucleotide; or
ii) the polynucleotide is double stranded and comprises a first strand and a second strand; the polynucleotide comprises a leader sequence located at a first end of the first strand or comprised in an adapter attached to the first end of the first strand; the first strand and the second strand are attached together by a hairpin adapter attached to (i) the second end of the first strand and (ii) a first end of the second strand; and the polynucleotide binding protein is stalled at a second end of the second strand or is stalled on an adapter at the second end of the second strand of the polynucleotide;
and optionally
a) the first part of the polynucleotide comprises (i) the part of the first stand between the leader sequence and the hairpin adapter, (ii) the hairpin adapter, and (iii) the part of the second strand between the hairpin adapter and the polynucleotide binding protein; and the second part of the polynucleotide is the same as the first part of the polynucleotide; and
b) the polynucleotide binding protein is orientated on the polynucleotide such that the polynucleotide binding protein controls the movement of the second part of the polynucleotide back through or across the nanopore against the applied force.

13. A method according to any one of the preceding claims, wherein the polynucleotide comprises a portion which is complementary to a tag sequence, wherein preferably the tag sequence is attached to the nanopore.

14. A method according to any one of the preceding claims, wherein the polynucleotide is double stranded; the portion which is complementary to a tag sequence is a portion of the first strand of the polynucleotide and/or the portion having an oligonucleotide hybridised thereto is a portion of the first strand of the polynucleotide; and wherein the second strand hybridises to the tag sequence as the first strand moves with respect to the nanopore.

15. A method according to any one of the preceding claims, wherein in step (ii a) the free movement of the polynucleotide with respect to the nanopore is governed by an ultra-fast polynucleotide-handling enzyme.

## Patentansprüche

1. Verfahren zur Charakterisierung eines Polynukleotids in einer Probe, das Verfahren umfassend:
(i) Kontaktieren einer Nanopore mit einem Polynukleotid;
(ii a) Durchführen von Messungen, während sich ein erster Teil des Polynukleotids unter einer aufgebrachten Kraft frei in Bezug auf die Nanopore bewegt;
(ii b) Bewerten einer oder mehrerer Eigenschaften des ersten Teils des Polynukleotids;
(ii) (a) wenn der erste Teil des Polynukleotids eine oder mehrere gewünschte Eigenschaften aufweist, Steuern der Bewegung eines zweiten Teils des Polynukleotids in Bezug auf die Nanopore unter Verwendung eines Polynukleotidbindungsproteins und Durchführen von Messungen, während sich der zweite Teil des Polynukleotids in Bezug auf die Nanopore bewegt, um eine oder mehrere Eigenschaften des Polynukleotids zu bestimmen, wodurch das Polynukleotid charakterisiert wird; oder (b) wenn der erste Teil des Polynukleotids eine oder mehrere gewünschte Eigenschaften nicht aufweist, Verwerfen des Polynukleotids.

2. Verfahren nach Anspruch 1, wobei, wenn der erste Teil des Polynukleotids nicht eine oder mehrere gewünschte Eigenschaften aufweist, Schritt (iii) das Wiederholen der Schritte (i), (ii a) und (ii b) mit weiteren Polynukleotiden aus der Probe umfasst, bis ein Polynukleotid mit einem ersten Teil mit einer oder mehreren gewünschten Eigenschaften identifiziert wird.

3. Verfahren nach Anspruch 1 oder 2, wobei die eine oder die mehreren gewünschten Eigenschaften aus der ungefähren Länge des ersten Teils des Polynucleotids, der Struktur des ersten Teils des Polynucleotids und der Zusammensetzung des ersten Teils des Polynucleotids ausgewählt sind;
vorzugsweise wobei das Beurteilen der einen oder mehreren Eigenschaften des Polynukleotids das Bestimmen der ungefähren Länge des ersten Teils des Polynukleotids umfasst;
vorzugsweise wobei das Bestimmen der ungefähren Länge des ersten Teils des Polynukleotids das Ermöglichen der freien Translokation des ersten Teils des Polynukleotids durch oder über die Nanopore unter einem angelegten Potential und das Bestimmen der Zeit umfasst, die der erste Teil des Polynukleotids für die Translokation durch oder über die Nanopore benötigt.

4. Verfahren nach einem der vorstehenden Ansprüche, wobei das Polynukleotid ein doppelsträngiges Polynukleotid ist, umfassend einen ersten Strang, der mit einem zweiten Strang durch eine Haarnadel oder einen Haarnadeladapter verbunden ist;
vorzugsweise wobei vor Schritt (i) ein Polynukleotidbindungsprotein an die Haarnadel oder den Haarnadeladapter gebunden und/oder daran festgehalten wird.

5. Verfahren nach einem der vorstehenden Ansprüche, wobei das Bestimmen einer oder mehrerer Eigenschaften des Polynukleotids das Bestimmen der Sequenz des Polynukleotids umfasst.

6. Verfahren nach einem der vorstehenden Ansprüche, umfassend:
(i) Kontaktieren einer Nanopore mit einem Polynukleotid, das ein Polynukleotidbindungsprotein aufweist, das in der Lage ist, die Bewegung des darauf festgehaltenen Polynukleotids zu steuern;
(ii a) Bestimmen der Zeit, die ein erster Teil des Polynukleotids benötigt, um sich unter einer angelegten Kraft frei in Bezug auf die Nanopore zu bewegen; und
(ii b) Bestimmen der ungefähren Länge des ersten Teils des Polynukleotids.

7. Verfahren nach einem der vorstehenden Ansprüche, wobei vor dem Schritt (i) ein Adapter an einem oder beiden Enden des Polynukleotids angebracht wird;
vorzugsweise wobei das Polynukleotid eine einzelsträngige Leadersequenz an einem Ende aufweist und ein Polynukleotidbindungsprotein daran am anderen Ende auf demselben Strang des Polynukleotids oder an einen Adapter gebunden ist, der an das andere Ende desselben Strangs des Polynukleotids gebunden ist.

8. Verfahren nach einem der vorstehenden Ansprüche, wobei vor Schritt (i) ein Polynukleotidbindungsprotein, das in der Lage ist, die Bewegung des Polynukleotids zu steuern, an das Polynukleotid oder an einen an das Polynukleotid gebundenen Adapter gebunden wird.

9. Verfahren nach einem der vorstehenden Ansprüche, wobei in Schritt (ii a) der erste Teil des Polynukleotids sich frei in Bezug auf die Nanopore in einer ersten Richtung relativ zu der aufgebrachten Kraft bewegt, und in Schritt (iii) das Polynukleotidbindungsprotein die Bewegung des zweiten Teils des Polynukleotids in Bezug auf die Nanopore in einer zweiten Richtung relativ zu der aufgebrachten Kraft steuert.

10. Verfahren nach einem der vorstehenden Ansprüche, wobei Schritt (i) das Kontaktieren der Nanopore mit einem ersten Ende des Polynukleotids oder einem an das erste Ende des Polynukleotids gebundenen Adapter umfasst und das Polynukleotidbindungsprotein an ein zweites Ende des Polynukleotids oder an einen an das zweite Ende des Polynukleotids gebundenen Adapter gebunden ist.

11. Verfahren nach einem der vorstehenden Ansprüche, wobei
a) Schritt (i) das Kontaktieren der Nanopore mit einer Leadersequenz am ersten Ende des Polynukleotids umfasst und das Polynukleotidbindungsprotein an einem zweiten Ende des Polynukleotids oder an einem Adapter, der an das zweite Ende des Polynukleotids gebunden ist, festgehalten wird; und
b) der erste Teil des Polynukleotids der Teil zwischen der Leadersequenz und dem Polynukleotidbindungsprotein ist und der zweite Teil des Polynukleotids der gleiche ist wie der erste Teil des Polynukleotids; und
c) das Polynukleotidbindungsprotein auf dem Polynukleotid ausgerichtet ist, sodass das Polynukleotidbindungsprotein die Bewegung des zweiten Teils des Polynukleotids in Bezug auf die Nanopore gegen die aufgebrachte Kraft steuert.

12. Verfahren nach einem der Ansprüche 1 bis 23, wobei das Polynukleotid einzelsträngig oder doppelsträngig ist;
vorzugsweise wobei:
i) das Polynukleotid einzelsträngig ist; das Polynukleotid eine Leadersequenz umfasst, wobei sich die Leadersequenz am ersten Ende des Polynukleotids befindet oder in einem Adapter enthalten ist, der an das erste Ende des Polynukleotids gebunden ist; und das Polynukleotidbindungsprotein an einem zweiten Ende des Polynukleotids befestigt ist oder an einem Adapter am zweiten Ende des Polynukleotids befestigt ist; oder
ii) das Polynukleotid doppelsträngig ist und einen ersten Strang und einen zweiten Strang umfasst; das Polynukleotid eine Leadersequenz umfasst, die sich an einem ersten Ende des ersten Strangs befindet oder in einem an das erste Ende des ersten Strangs gebundenen Adapter enthalten ist; der erste Strang und der zweite Strang durch einen an (i) das zweite Ende des ersten Strangs und (ii) ein erstes Ende des zweiten Strangs gebundenen Haarnadeladapter miteinander verbunden sind; und das Polynukleotidbindungsprotein an einem zweiten Ende des zweiten Strangs oder an einem Adapter am zweiten Ende des zweiten Strangs des Polynukleotids befestigt ist;
und optional
a) der erste Teil des Polynukleotids (i) den Teil des ersten Strangs zwischen der Leadersequenz und dem Haarnadeladapter, (ii) den Haarnadeladapter und (iii) den Teil des zweiten Strangs zwischen dem Haarnadeladapter und dem Polynukleotidbindungsprotein umfasst; und der zweite Teil des Polynukleotids der gleiche ist wie der erste Teil des Polynukleotids; und
b) das Polynukleotidbindungsprotein auf dem Polynukleotid ausgerichtet ist, sodass das Polynukleotidbindungsprotein die Bewegung des zweiten Teils des Polynukleotids zurück durch oder quer durch die Nanopore gegen die aufgebrachte Kraft steuert.

13. Verfahren nach einem der vorstehenden Ansprüche, wobei das Polynukleotid einen Abschnitt umfasst, der komplementär zu einer Tag-Sequenz ist, wobei die Tag-Sequenz vorzugsweise an die Nanopore gebunden ist.

14. Verfahren nach einem der vorstehenden Ansprüche, wobei das Polynukleotid doppelsträngig ist; der Abschnitt, der zu einer Tag-Sequenz komplementär ist, ein Abschnitt des ersten Strangs des Polynukleotids ist und/oder der Abschnitt, an den ein Oligonukleotid hybridisiert ist, ein Abschnitt des ersten Strangs des Polynukleotids ist; und wobei der zweite Strang an die Tag-Sequenz hybridisiert, wenn sich der erste Strang in Bezug auf die Nanopore bewegt.

15. Verfahren nach einem der vorstehenden Ansprüche, wobei in Schritt (ii a) die freie Bewegung des Polynukleotids in Bezug auf die Nanopore durch ein ultraschnelles polynukleotidhandhabendes Enzym gesteuert wird.

## Revendications

1. Procédé de caractérisation d'un polynucléotide dans un échantillon, le procédé comprenant :
(i) la mise en contact d'un nanopore avec un polynucléotide ;
(ii a) la prise de mesures alors qu'une première partie du polynucléotide se déplace librement par rapport au nanopore sous l'effet d'une force appliquée ;
(ii b) l'évaluation d'une ou plusieurs propriétés de la première partie du polynucléotide ;
(iii) (a) lorsque la première partie du polynucléotide possède une ou plusieurs propriétés souhaitées, la commande du mouvement d'une seconde partie du polynucléotide par rapport au nanopore à l'aide d'une protéine de liaison de polynucléotide et la prise de mesures alors que la seconde partie du polynucléotide se déplace par rapport au nanopore pour déterminer une ou plusieurs caractéristiques du polynucléotide, caractérisant ainsi le polynucléotide ; ou (b) lorsque la première partie du polynucléotide ne possède pas une ou plusieurs propriétés souhaitées, le rejet du polynucléotide.

2. Procédé selon la revendication 1 dans lequel, lorsque la première partie du polynucléotide ne possède pas une ou plusieurs propriétés souhaitées, l'étape (iii) comprend la répétition des étapes (i), (ii a) et (ii b) avec des polynucléotides supplémentaires de l'échantillon jusqu'à ce qu'un polynucléotide possédant une première partie possédant une ou plusieurs propriétés souhaitées soit identifié.

3. Procédé selon la revendication 1 ou 2, dans lequel lesdites une ou plusieurs propriétés souhaitées sont sélectionnées parmi la longueur approximative de la première partie du polynucléotide, la structure de la première partie du polynucléotide, et la composition de la première partie du polynucléotide ;
de préférence dans lequel l'évaluation desdites une ou plusieurs propriétés du polynucléotide comprend la détermination de la longueur approximative de la première partie du polynucléotide ;
de préférence dans lequel l'évaluation de la longueur approximative de la première partie du polynucléotide comprend le fait de permettre à la première partie du polynucléotide de transloquer librement à travers ou le long du nanopore sous un potentiel appliqué et la détermination du temps nécessaire à la première partie du polynucléotide pour transloquer à travers ou le long du nanopore.

4. Procédé selon l'une quelconque des revendications précédentes, dans lequel le polynucléotide est un polynucléotide double brin comprenant un premier brin relié à un second brin par une épingle à cheveux ou un adaptateur en épingle à cheveux ;
de préférence dans lequel, avant l'étape (i), une protéine de liaison de polynucléotide est liée et/ou bloquée au niveau de l'épingle à cheveux ou de l'adaptateur en épingle à cheveux.

5. Procédé selon l'une quelconque des revendications précédentes, dans lequel la détermination d'une ou plusieurs caractéristiques du polynucléotide comprend la détermination de la séquence du polynucléotide.

6. Procédé selon l'une quelconque des revendications précédentes, comprenant :
(i) la mise en contact d'un nanopore avec un polynucléotide possédant une protéine de liaison de polynucléotide capable de commander le mouvement du polynucléotide bloqué sur celle-ci ;
(ii a) la détermination du temps nécessaire à une première partie du polynucléotide pour se déplacer librement par rapport au nanopore sous l'effet d'une force appliquée ; et
(ii b) la détermination de la longueur approximative de la première partie du polynucléotide.

7. Procédé selon l'une quelconque des revendications précédentes, dans lequel un adaptateur est fixé à une ou aux deux extrémités du polynucléotide avant l'étape (i) ; de préférence dans lequel le polynucléotide comprend une séquence leader simple brin à une extrémité et possède une protéine de liaison de polynucléotide liée à celle-ci à l'autre extrémité sur le même brin du polynucléotide ou à un adaptateur attaché à l'autre extrémité du même brin du polynucléotide.

8. Procédé selon l'une quelconque des revendications précédentes, dans lequel avant l'étape (i), une protéine de liaison de polynucléotide capable de commander le mouvement du polynucléotide est bloquée sur le polynucléotide ou sur un adaptateur attaché au polynucléotide.

9. Procédé selon l'une quelconque des revendications précédentes, dans lequel, dans l'étape (ii a), la première partie du polynucléotide se déplace librement par rapport au nanopore dans une première direction par rapport à la force appliquée, et dans l'étape (iii), la protéine de liaison de polynucléotide commande le mouvement de la seconde partie du polynucléotide par rapport au nanopore dans une seconde direction par rapport à la force appliquée.

10. Procédé selon l'une quelconque des revendications précédentes, dans lequel l'étape (i) comprend la mise en contact du nanopore avec une première extrémité du polynucléotide ou un adaptateur attaché à la première extrémité du polynucléotide et la protéine de liaison de polynucléotide est liée à une seconde extrémité du polynucléotide ou à un adaptateur attaché à la seconde extrémité du polynucléotide.

11. Procédé selon l'une quelconque des revendications précédentes, dans lequel :
a) l'étape (i) comprend la mise en contact du nanopore avec une séquence leader à la première extrémité du polynucléotide et la protéine de liaison de polynucléotide est bloquée à une seconde extrémité du polynucléotide ou sur un adaptateur attaché à la seconde extrémité du polynucléotide ; et
b) la première partie du polynucléotide est la partie entre la séquence leader et la protéine de liaison de polynucléotide et la seconde partie du polynucléotide est la même que la première partie du polynucléotide ; et
c) la protéine de liaison de polynucléotide est orientée sur le polynucléotide de sorte que la protéine de liaison de polynucléotide commande le mouvement de la seconde partie du polynucléotide par rapport au nanopore contre la force appliquée.

12. Procédé selon l'une quelconque des revendications 1 à 23, dans lequel le polynucléotide est simple brin ou double brin ;
de préférence dans lequel :
i) le polynucléotide est simple brin ; le polynucléotide comprend une séquence leader, dans lequel la séquence leader est située au niveau de la première extrémité du polynucléotide ou est comprise dans un adaptateur attaché à la première extrémité du polynucléotide ; et la protéine de liaison de polynucléotide est bloquée au niveau d'une seconde extrémité du polynucléotide ou est bloquée sur un adaptateur à la seconde extrémité du polynucléotide ; ou
ii) le polynucléotide est double brin et comprend un premier brin et un second brin ; le polynucléotide comprend une séquence leader située au niveau d'une première extrémité du premier brin ou comprise dans un adaptateur attaché à la première extrémité du premier brin ; le premier brin et le second brin sont attachés ensemble par un adaptateur en épingle à cheveux attaché à (i) la seconde extrémité du premier brin et (ii) une première extrémité du second brin ; et la protéine de liaison de polynucléotide est bloquée au niveau d'une seconde extrémité du second brin ou est bloquée sur un adaptateur au niveau de la seconde extrémité du second brin du polynucléotide ;
et éventuellement
a) la première partie du polynucléotide comprend (i) la partie du premier brin entre la séquence leader et l'adaptateur en épingle à cheveux, (ii) l'adaptateur en épingle à cheveux, et (iii) la partie du second brin entre l'adaptateur en épingle à cheveux et la protéine de liaison de polynucléotide ; et la seconde partie du polynucléotide est la même que la première partie du polynucléotide ; et
b) la protéine de liaison de polynucléotide est orientée sur le polynucléotide de sorte que la protéine de liaison de polynucléotide commande le mouvement de la seconde partie du polynucléotide à travers ou le long du nanopore contre la force appliquée.

13. Procédé selon l'une quelconque des revendications précédentes, dans lequel le polynucléotide comprend une partie qui est complémentaire d'une séquence d'étiquette, dans lequel, de préférence, la séquence d'étiquette est attachée au nanopore.

14. Procédé selon l'une quelconque des revendications précédentes, dans lequel le polynucléotide est double brin ; la partie qui est complémentaire d'une séquence d'étiquette est une partie du premier brin du polynucléotide et/ou la partie possédant un oligonucléotide hybridé à celle-ci est une partie du premier brin du polynucléotide ; et dans lequel le second brin s'hybride à la séquence d'étiquette alors que le premier brin se déplace par rapport au nanopore.

15. Procédé selon l'une quelconque des revendications précédentes, dans lequel dans l'étape (ii a), le mouvement libre du polynucléotide par rapport au nanopore est régi par une enzyme de manipulation de polynucléotide ultra-rapide.
